(19) **Europäisches Patentamt / European Patent Office / Office européen des brevets**

(11) **EP 3 982 369 A1**

(12) # EUROPEAN PATENT APPLICATION
published in accordance with Art. 153(4) EPC

(43) Date of publication:
**13.04.2022 Bulletin 2022/15**

(21) Application number: **20817642.0**

(22) Date of filing: **08.06.2020**

(51) International Patent Classification (IPC):
**G16B 40/20** (2019.01)   **G06N 3/04** (2006.01)
**G06N 20/00** (2019.01)

(52) Cooperative Patent Classification (CPC):
**G06N 3/04; G06N 20/00; G16B 40/20**

(86) International application number:
**PCT/JP2020/022576**

(87) International publication number:
**WO 2020/246617 (10.12.2020 Gazette 2020/50)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **07.06.2019   JP 2019106814**

(71) Applicant: **CHUGAI SEIYAKU KABUSHIKI KAISHA**
**Tokyo 115-8543 (JP)**

(72) Inventors:
• **TERAMOTO, Reiji**
**Kamakura-shi, Kanagawa 247-8530 (JP)**
• **METSUGI, Shouichi**
**Kamakura-shi, Kanagawa 247-8530 (JP)**
• **KAKUZAKI, Taro**
**Kamakura-shi, Kanagawa 247-8530 (JP)**
• **SAKA, Koichiro**
**Kamakura-shi, Kanagawa 247-8530 (JP)**
• **KOGA, Hikaru**
**Gotemba-shi, Shizuoka 412-8513 (JP)**
• **SAMPEI, Zenjiro**
**Gotemba-shi, Shizuoka 412-8513 (JP)**

(74) Representative: **Vossius & Partner**
**Patentanwälte Rechtsanwälte mbB**
**Siebertstraße 3**
**81675 München (DE)**

(54) ## INFORMATION PROCESSING SYSTEM, INFORMATION PROCESSING METHOD, PROGRAM, AND METHOD FOR PRODUCING ANTIGEN-BINDING MOLECULE OR PROTEIN

(57)   A sequence learner performs machine learning on the basis of sequence information representing sequences including some or all of the sequences of a plurality of antigen-binding molecules and proteins and thereby generates a trained model. A virtual sequence generator generates, on the basis of the trained model, virtual sequence information obtained by mutating at least one of constituent elements constituting a sequence represented by the sequence information inputted into the trained model.

EP 3 982 369 A1

# Fig. 6

**Description**

[Technical Field]

The present invention relates to an information processing system, an information processing method, a program, and a method for producing an antigen-binding molecule or protein.
The present application claims priority to Japanese Patent Application No. 2019-106814 filed in Japan on June 7, 2019, the contents of which are incorporated herein by reference.

[Background Art]

Information processing techniques of machine learning have been recently utilized in the field of pharmaceuticals.
In a technique described in Patent Literature 1, for example, a machine learning engine is trained with use of affinity information on various antibodies and affinities of antibodies against antigens.

[Citation List]

[Patent Literature]

Patent Literature 1: International Publication No. WO 2018/132752

[Summary of Invention]

[Technical Problem]

Machine learning in the field of pharmaceuticals is required to predict information on the sequence and so on of a desired antigen-binding molecule and provide the information by using a trained model after training.
The present invention was made to solve the problem, and an object of the present disclosure is to provide an information processing system capable of providing information on a desired antigen-binding molecule or protein, an information processing method, a program, and a method for producing an antigen-binding molecule or protein.

[Solution to Problem]

The present invention was made to solve the above problem, and an aspect of the present invention is an information processing system including: a sequence learner configured to perform machine learning on the basis of sequence information representing sequences including some or all of the sequences of a plurality of antigen-binding molecules and thereby generate a trained model that has learned a character of the sequences; and a sequence generator configured to generate virtual sequence information obtained by mutating at least one of constituent elements constituting a sequence represented by the sequence information on the basis of the trained model.
An aspect of the present invention is an information processing system including: a sequence learner configured to perform machine learning on the basis of sequence information representing sequences including some or all of the sequences of a plurality of proteins and thereby generate a trained model that has learned a character of the sequences; and a sequence generator configured to generate virtual sequence information obtained by mutating at least one of constituent elements constituting a sequence represented by the sequence information on the basis of the trained model.
An aspect of the present invention is an information processing system including: a learner configured to perform machine learning on the basis of sequence information representing sequences including some or all of the sequences of a plurality of antigen-binding molecules or proteins and results of characterization of antigen-binding molecules or proteins represented by the sequences and thereby generate a second trained model; and an estimator configured to input virtual sequence information generated on the basis of a first trained model being the above trained model into the second trained model, execute arithmetic processing of the second trained model, and thereby estimate predicted values for characterization of antigen-binding molecules or proteins with sequences represented by the inputted virtual sequence information.
An aspect of the present invention is an information processing method in an information processing system, the method including: a sequence learning step of performing machine learning on the basis of sequence information representing sequences including some or all of the sequences of a plurality of antigen-binding molecules or proteins and thereby generating a trained model that has learned a character of the sequence information; and a sequence generation step of generating virtual sequence information obtained by mutating at least one of constituent elements constituting a sequence represented by the sequence information on the basis of the trained model.

An aspect of the present invention is a program configured to allow a computer in an information processing system to execute: a sequence learning procedure of performing machine learning on the basis of sequence information representing sequences including some or all of the sequences of a plurality of antigen-binding molecules or proteins and thereby generating a trained model; and a sequence generation procedure of generating virtual sequence information obtained by mutating at least one of constituent elements constituting a sequence represented by the sequence information on the basis of the trained model.

An aspect of the present invention is a method for producing an antigen-binding molecule or protein with use of any of the above information processing systems, wherein the antigen-binding molecule or protein is represented by a virtual sequence, and a predicted value for characterization is estimated for the virtual sequence.

[Advantageous Effect of Invention]

The present invention enables providing information on a desired antigen-binding molecule or protein.

[Brief Description of Drawings]

[Figure 1] Figure 1 shows a schematic diagram illustrating an example of an information processing system according to a first embodiment.

[Figure 2] Figure 2 shows an illustration for describing an example of a sequence of panning according to the present embodiment.

[Figure 3] Figure 3 shows a block diagram illustrating an example of a user terminal according to the present embodiment.

[Figure 4] Figure 4 shows a diagram illustrating an example of screen flow according to the present embodiment.

[Figure 5] Figure 5 shows a block diagram illustrating an example of a next-generation sequencer according to the present embodiment.

[Figure 6] Figure 6 shows a block diagram illustrating an example of a server according to the present embodiment.

[Figure 7] Figure 7 shows a diagram illustrating an example of experiment information according to the present embodiment.

[Figure 8] Figure 8 shows a diagram illustrating an example of experiment attribute information according to the present embodiment.

[Figure 9] Figure 9 shows a diagram illustrating an example of a dataset according to the present embodiment.

[Figure 10] Figure 10 shows a diagram illustrating another example of a dataset according to the present embodiment.

[Figure 11] Figure 11 shows a diagram illustrating an example of learning datasets according to the present embodiment.

[Figure 12] Figure 12 shows a diagram illustrating an example of prediction target sequence information according to the present embodiment.

[Figure 13] Figure 13 shows a diagram illustrating an example of characterization information according to the present embodiment.

[Figure 14] Figure 14 shows an illustration for describing an example of a learning process according to the present embodiment.

[Figure 15] Figure 15 shows a conceptual diagram illustrating the structure of an LSTM according to the present embodiment.

[Figure 16] Figure 16 shows a flowchart illustrating an example of operations of a virtual sequence generator according to the present embodiment.

[Figure 17] Figure 17 shows a flowchart illustrating an example of operations of the server according to the present embodiment.

[Figure 18] Figure 18 shows a flowchart illustrating another example of operations of the server according to the present embodiment.

[Figure 19] Figure 19 shows a block diagram illustrating an example of a server according to a second embodiment.

[Figure 20] Figure 20 shows a diagram illustrating an example of a dataset according to the present embodiment.

[Figure 21] Figure 21 shows a diagram illustrating another example of a dataset according to the present embodiment.

[Figure 22] Figure 22 shows a block diagram illustrating an example of a server according to a third embodiment.

[Figure 23] Figure 23 shows a diagram illustrating the summary of a learning model according to the present embodiment.

[Figure 24] Figure 24 shows a block diagram illustrating an example of a user terminal according to a fourth embodiment.

[Figure 25] Figure 25 shows a block diagram illustrating an example of a server according to the present embodiment.

[Figure 26] Figure 26 shows a diagram illustrating an example of sequence information according to the present embodiment.

[Figure 27] Figure 27 shows a diagram illustrating an example of characteristic information according to the present embodiment.

[Figure 28] Figure 28 shows a graph representing an example of a sensorgram according to the present embodiment.

[Figure 29] Figure 29 shows a diagram illustrating an example of prediction target sequence information according to the present embodiment.

[Figure 30] Figure 30 shows a diagram illustrating an example of evaluation result information according to the present embodiment.

[Figure 31] Figure 31 shows an illustration for describing an example of a learning process according to the present embodiment.

[Figure 32] Figure 32 shows an illustration for describing an example of an evaluation process according to the present embodiment.

[Figure 33] Figure 33 shows a flowchart illustrating an example of operations of the server according to the present embodiment.

[Figure 34] Figure 34 shows a flowchart illustrating another example of operations of the server according to the present embodiment.

[Figure 35] Figure 35 shows a block diagram illustrating an example of a server according to a fifth embodiment.

[Figure 36] Figure 36 shows a flowchart illustrating an example of operations of an information processing system according to Examples.

[Figure 37] Figure 37 shows a block diagram illustrating an example of the hardware configuration of the server according to the embodiments.

[Figure 38] Figure 38 shows a diagram illustrating an example of structural analysis information according to the fourth and fifth embodiments.

[Figure 39] Figure 39 shows a graph representing the relationship between sequences and characteristics according to Example.

[Figure 40] Figure 40 shows a graph representing the prediction precision for characteristics of sequences according to Example.

[Figure 41] Figure 41 shows plots demonstrating the similarity between training sequences and virtual sequences according to Example.

[Figure 42] Figure 42 shows other plots demonstrating the similarity between training sequences and virtual sequences according to Example.

[Figure 43A] Figure 43A shows a plot demonstrating the correlation between predicted values and actual measurements for a characteristic of sequences according to Example.

[Figure 43B] Figure 43B shows a plot demonstrating the correlation between predicted values and actual measurements for another characteristic of sequences according to Example.

[Figure 43C] Figure 43C shows a plot demonstrating the correlation between predicted values and actual measurements for another characteristic of sequences according to Example.

[Figure 43D] Figure 43D shows a plot demonstrating the correlation between predicted values and actual measurements for another characteristic of sequences according to Example.

[Figure 43E] Figure 43E shows a plot demonstrating the correlation between predicted values and actual measurements for another characteristic of sequences according to Example.

[Figure 43F] Figure 43F shows a plot demonstrating the correlation between predicted values and actual measurements for another characteristic of sequences according to Example.

[Figure 43G] Figure 43G shows a plot demonstrating the correlation between predicted values and actual measurements for another characteristic of sequences according to Example.

[Figure 43H] Figure 43H shows a plot demonstrating the correlation between predicted values and actual measurements for another characteristic of sequences according to Example.

[Figure 43I] Figure 43I shows a plot demonstrating the correlation between predicted values and actual measurements for another characteristic of sequences according to Example.

[Figure 44A] Figure 44A shows a plot demonstrating the correlation between predicted values and actual measurements for a characteristic of sequences according to another Example.

[Figure 44B] Figure 44B shows a plot demonstrating the correlation between predicted values and actual measurements for another characteristic of sequences according to another Example.

[Figure 44C] Figure 44C shows a plot demonstrating the correlation between predicted values and actual measurements for another characteristic of sequences according to another Example.

[Figure 44D] Figure 44D shows a plot demonstrating the correlation between predicted values and actual measurements for another characteristic of sequences according to another Example.

[Figure 44E] Figure 44E shows a plot demonstrating the correlation between predicted values and actual measurements for another characteristic of sequences according to another Example.

[Figure 44F] Figure 44F shows a plot demonstrating the correlation between predicted values and actual measurements for another characteristic of sequences according to another Example.

[Figure 44G] Figure 44G shows a plot demonstrating the correlation between predicted values and actual measurements for another characteristic of sequences according to another Example.

[Figure 44H] Figure 44H shows a plot demonstrating the correlation between predicted values and actual measurements for another characteristic of sequences according to another Example.

[Figure 44I] Figure 44I shows a plot demonstrating the correlation between predicted values and actual measurements for another characteristic of sequences according to another Example.

[Figure 45] Figure 45 shows graphs for describing an improved characteristic of sequences according to Example.

[Description of Embodiments]

<Terms and Others>

The following definitions and detailed descriptions are provided to facilitate understanding of the present disclosure described herein.

- Amino Acids

Herein, amino acids are each specified with a one-letter code, a three-letter codes, or both of them, for example, as expressed as Ala/A, Leu/L, Arg/R, Lys/K, Asn/N, Met/M, Asp/D, Phe/F, Cys/C, Pro/P, Gln/Q, Ser/S, Glu/E, Thr/T, Gly/G, Trp/W, His/H, Tyr/Y, Ile/I, or Val/V.

- Modification of Amino Acids

A known method such as site-specific mutagenesis (Kunkel et al. (Proc. Natl. Acad. Sci. USA (1985) 82, 488-492)) and Overlap extension PCR can be appropriately employed for modification of an amino acid in the amino acid sequence of an antigen-binding molecule. As an amino acid modification method to substitute with an amino acid other than natural amino acids, on the other hand, a plurality of known methods can be employed (Annu. Rev. Biophys. Biomol. Struct. (2006) 35, 225-249, Proc. Natl. Acad. Sci. U.S.A. (2003) 100 (11), 6353-6357). For example, a cell-free translation system (Clover Direct (ProteinExpress Co., Ltd.)) including tRNA in which a non-natural amino acid is bonded to amber suppressor tRNA complementary to a UAG codon (amber codon), one of stop codons, is also preferably used.

- Antigen

Herein, the structure of an "antigen" is not limited to a particular structure as long as the structure contains an epitope to which an antigen-binding domain binds. In certain modes, the antigen is a peptide of four or more amino acids, or a polypeptide, or a protein.

Examples of the antigen include membrane-associated molecules expressed on cell membranes, and soluble molecules extracellularly secreted by cells.

- Antigen-Binding Domain

Herein, domains of any structure that bind to an antigen of interest can be used as an "antibody-binding domain". Preferred examples of such domains include variable regions of a heavy chain and a light chain of an antibody; a module called an A domain, which is contained in Avimer, a cell membrane protein present in the living body, and consists of about 35 amino acids (International Publication Nos. WO 2004/044011, WO 2005/040229); Adnectin, which contains a 10Fn3 domain, a domain that binds to the protein in fibronectin, which is a glycoprotein expressed on cell membranes (International Publication No. WO 2002/032925); Affibody, which uses, as a scaffold, an IgG-binding domain constituting three helical bundles each consisting of 58 amino acids of Protein A (International Publication No. WO 1995/001937); DARPins (Designed Ankyrin Repeat proteins), which is a region in which ankyrin repeats (ARs), each containing 33 amino acid residues, that have a structure including repeatedly stacked subunits of a turn, two antiparallel helices, and a loop are exposed to the molecular surface (International Publication No. WO 2002/020565); Anticalin, which is four loop regions supporting one side of a barrel structure in which eight highly conserved antiparallel strands twist to the central direction in a lipocalin molecule such as neutrophil gelatinase-associated lipocalin (NGAL) (International Publication No. WO2003/029462); and a concave region of a parallel sheet structure in the inside of the horseshoe-shaped structure including repeatedly stacked leucine-rich-repeat (LRR) modules of a variable lymphocyte receptor (VLR) not having the structure of immunoglobulin as an acquired immune system of agnathans such as lampreys and hagfish (International Publication No. WO 2008/016854).

Preferred examples of the antigen-binding domain in the present disclosure include antigen-binding domains containing variable regions of a heavy chain and light chain of an antibody. Preferred examples of such antigen-binding domains include "scFv (single chain Fv)", "single chain antibodies", "Fv", "scFv2 (single chain Fv 2)", "Fab", and "F(ab')$_2$".

- Antigen-Binding Molecule

In the present disclosure, the term "antigen-binding molecule", which contains an antigen-binding domain, is used in the broadest sense, and specifically includes various molecular types containing an antigen-binding domain. The antigen-binding molecule may be a molecule consisting only of an antigen-binding domain, or a molecule containing an antigen-binding domain and another domain. If the antigen-binding molecule is a molecule formed of an antigen-binding domain and an Fc region bound together, for example, examples of such molecules include complete antibodies and antibody fragments. Antibodies can include single monoclonal antibodies (including agonist and antagonist antibodies), human antibodies, humanized antibodies, and chimeric antibodies. The term "antigen-binding molecule" in the present disclosure includes scaffold molecules formed as a library for construction of antigen-binding domains by using only a partial structure of three-dimensional structure, as a scaffold, such as existing stable $\alpha\beta/$ barrel protein structure.

- Antibody

Herein, the term "antibody" refers to the whole sequence of immunoglobulin that is natural or produced through partial or complete synthesis, or an antigen-binding molecule containing a partial sequence of immunoglobulin. An antibody can be isolated from a natural resource such as plasma and serum in which the antibody is naturally present and a culture supernatant of hybridoma cells producing the antibody, and can be partially or completely synthesized by using a technique of gene recombination or the like. Preferred examples of the antibody include isotypes of immunoglobulin and subclasses of these isotypes. Known as human immunoglobulin are nine classes (isotypes): IgG1, IgG2, IgG3, IgG4, IgA1, IgA2, IgD, IgE, and IgM. Among these isotypes, the term "antibody" in the present disclosure includes IgG1, IgG2, IgG3, and IgG4. While a plurality of allotype sequences resulting from genetic polymorphism are described as human IgG1, human IgG2, human IgG3, and human IgG4 constant regions in Sequences of proteins of immunological interest, NIH Publication No. 91-3242, any of them is acceptable in the present disclosure. In particular, in the case of the sequence of human IgG1, the amino acid sequence ranging from position 356 to position 358 as specified by EU numbering may be DEL or EEM. While a plurality of allotype sequences resulting from genetic polymorphism are described as human Igκ (Kappa) constant regions and human Igλ (Lambda) constant regions in Sequences of proteins of immunological interest, NIH Publication No. 91-3242, any of them is acceptable in the present disclosure.

- EU Numbering and Kabat Numbering

According to the method used in the present disclosure, amino acid positions allocated to CDR and FR of an antibody are specified by Kabat (Sequences of Proteins of Immunological Interest (National Institute of Health, Bethesda, Md., 1987 and 1991). Herein, if the antigen-binding molecule is an antibody or antigen-biding fragment, amino acids in variable regions are expressed according to the Kabat numbering, and amino acids in constant regions are expressed according to EU numbering, which is based on the amino acid positioning by Kabat.

- Variable Region

The term "variable region" or "variable domain" refers to a domain of the heavy chain or light chain of an antibody, the domain involved in allowing the antibody to bind to the antigen. The variable domains (VH and VL) of the heavy chain and the light chain of a natural antibody normally have similar structure including a framework region (FR) including four conserved domains and three hypervariable regions (HVR) (e.g., see Kindt et al. Kuby Immunology, 6th ed., W.H. Freeman and Co., page 91 (2007)). One VH or VL domain would be enough to provide antigen-binding specificity. In addition, an antibody that binds to a certain antigen may be isolated with use of the VH or VL domain from an antibody that binds to the antigen through screening of a complementary library to the VL or VH domain. For example, see Portolano et al., J. Immunol. 150:880-887 (1993); Clarkson et al., Nature 352:624-628 (1991).

- Hypervariable Region

The term "hypervariable region" or "HVR" used herein refers a region in the variable domain of an antibody, the region being hypervariable in its sequence ("CDR" (complementarity determining region)), and/or forming a loop definite in structure ("hypervariable loop"), and/or containing antigen-contacting residues ("antigen contacts"). Normally, an antibody contains six HVRs: three in VH (HI, H2, H3) and three in VL (L1, L2, L3). Exemplary HVRs herein include the following:

(a) hypervariable loops formed of amino acid residues 26 to 32 (L1), 50 to 52 (L2), 91 to 96 (L3), 26 to 32 (HI), 53 to 55 (H2), and 96 to 101 (H3) (Chothia and Lesk, J. Mol. Biol. 196:901-917 (1987));
(b) CDRs formed of amino acid residues 24 to 34 (L1), 50 to 56 (L2), 89 to 97 (L3), 31 to 35b (HI), 50 to 65 (H2), and 95 to 102 (H3) (Kabat et al., Sequences of Proteins of Immunological Interest, 5th Ed. Public Health Service, National Institutes of Health, Bethesda, MD (1991));
(c) antigen contacts formed of amino acid residues 27c to 36 (L1), 46 to 55 (L2), 89 to 96 (L3), 30 to 35b (HI), 47 to 58 (H2), and 93 to 101 (H3) (MacCallum et al. J. Mol. Biol. 262: 732-745 (1996)); and
(d) a combination of (a), (b), and/or (c) including HVR amino acid residues 46 to 56 (L2), 47 to 56 (L2), 48 to 56 (L2), 49 to 56 (L2), 26 to 35 (HI), 26 to 35b (HI), 49 to 65 (H2), 93 to 102 (H3), and 94 to 102 (H3).

Unless otherwise specified, HVR residues and other residues in a variable domain (e.g., FR residues) are herein numbered according to Kabat et al. in the above.

- Framework

The term "framework" or "FR" refers to variable domain residues except hypervariable region (HVR) residues. FR in a variable domain normally consists of four FR domains: FR1, FR2, FR3, and FR4. According to this, HVR and FR sequences normally appear in VH (or VL) in the following order: FR1-H1(L1)-FR2-H2(L2)-FR3-H3(L3)-FR4.

- Fc Region

The Fc region contains an amino acid sequence derived from the constant region of an antibody heavy chain. The Fc region is a part of the heavy chain constant region of an antibody, the part starting from the N terminus of the hinge region at the papain cleavage site, which is an amino acid at around position 216 specified by EU numbering, and including the hinge and CH2 and CH3 domains. The Fc region can be obtained from human IgG1, but the origin is not limited to IgG of a particular subclass. Preferred examples of the Fc region include an Fc region having binding activity to FcRn in an acidic pH region, as described later. Additional preferred examples of the Fc region include an Fc region having binding activity to the Fcγ receptor, as described later. One non-limiting mode of such an Fc region is, for example, an Fc region represented by human IgG1 (SEQ ID NO: XX), IgG2 (SEQ ID NO: XX), IgG3 (SEQ ID NO: XX), or IgG4 (SEQ ID NO: XX).

- Small Molecule Antibody

The antibody to be used in the present disclosure is not limited to the full-length molecule of an antibody, and may be a small molecule antibody or a modified product thereof. The small molecule antibody is not limited in any way as long as the small molecule antibody contains an antibody fragment formed by deleting a part of a full-length antibody (e.g., a whole antibody such as whole IgG), and has binding activity to an antigen. The small molecule antibody in the present disclosure is not limited in any way as long as the small molecule antibody is a part of a full-length antibody; however, it is preferable for the small molecule antibody to contain a heavy chain variable region (VH) or/and a light chain variable region (VL). A substitution, deletion, addition, and/or insertion may be present in the amino acid sequence of VH or VL. Further, a part of VH or/and VL may be deleted as long as the resultant has binding activity to an antigen. The variable region may be chimerized or humanized. Specific examples of the antibody fragment include Fab, Fab', F(ab')2, and Fv. Specific examples of the small molecule antibody include Fab, Fab', F(ab')$_2$, Fv, scFv (single chain Fv), Diabody, and sc(Fv)2 (single chain (Fv)2). Multimers of these antibodies (e.g., dimers, trimers, tetramers, polymers) are also included in the "small molecule antibody" in the present disclosure.

Diabody is a bivalent small molecule antibody constructed through gene fusion (Holliger et al. (Proc. Natl. Acad. Sci. U.S.A. 90, 6444-6448 (1993), European Patent Publication No. EP 404097, and PCT International Publication No. WO 1993/011161, etc.). Diabody is a dimer composed of two polypeptide chains, and VL and VH in each polypeptide chain are normally linked via a short linker such that the linker does not allow VL and VH to form a bond together in the chain, for example, in a length of about five residues. VL and VH encoded in the same polypeptide chain cannot form a single variable region fragment, but instead form a dimer, because of the shortness of the linker between VL and VH, and hence Diabody comes to have two antigen-binding sites.

scFv is obtained by linking the H chain variable region and L chain variable region of an antibody. In this scFv, the H chain variable region and L chain variable region are linked via a linker, preferably via a peptide linker (Huston et al. (Proc. Natl. Acad. Sci. U.S.A. (1988) 85, 5879-5883). The H chain variable region and L chain variable region in scFv may be derived from any of the antibodies described herein as the antibody. The peptide linker to link the variable regions is not limited in any way, and, for example, any single chain peptide consisting of about 3 to 25 residues, or a peptide linker described later may be used.

sc(Fv)2 is a small molecule antibody obtained by binding two VHs and two VLs via linkers or the like to form a single chain (Hudson et al. (J. Immunol. Methods (1999) 231, 177-189). sc(Fv)2 may be produced, for example, by joining scFv via a linker.

Preferred is an antibody in which two VHs and two VLs are aligned in the order of VH, VL, VH, VL ([VH] linker [VL] linker [VH] linker [VL]) from the N-terminal side of the single chain polypeptide. The order of two VHs and two VLs is not limited to this disposition, and they may be aligned in any order. For example, the following dispositions are also acceptable.

- [VL] linker [VH] linker [VH] linker [VL]
- [VH] linker [VL] linker [VL] linker [VH]
- [VH] linker [VH] linker [VL] linker [VL]
- [VL] linker [VL] linker [VH] linker [VH]
- [VL] linker [VH] linker [VL] linker [VH]

Any of the linkers describe in the above section - Antigen-Binding Molecule - can be used as the linker to bind the variable regions of an antibody. Examples of particularly preferred modes of sc(Fv)2 in the present disclosure include the following sc(Fv)2.

- [VH] peptide linker (15 amino acids) [VL] peptide linker (15 amino acids) [VH] peptide linker (15 amino acids) [VL]

While three linkers are normally required to bind four antibody variable regions, the linkers to be used may the same linker, or different linkers.

To obtain such a small molecule antibody, it is suitable to treat an antibody with an enzyme such as papain and pepsin to generate an antibody fragment; or to construct DNA encoding such an antibody fragment or small molecule antibody, introduce the DNA into an expression vector, and then express in an appropriate host cell (e.g., see Co, M. S. et al., J. Immunol. (1994) 152, 2968-2976; Better, M. and Horwitz, A. H., Methods Enzymol. (1989) 178, 476-496; Pluckthun, A. and Skerra, A., Methods Enzymol. (1989) 178, 497-515; Lamoyi, E., Methods Enzymol. (1986) 121, 652-663; Rousseaux, J. et al., Methods Enzymol. (1986) 121, 663-669; Bird, R. E. and Walker, B. W., Trends Biotechnol. (1991) 9, 132-137).

- Single Domain Antibody

A preferred example of the antigen-binding domain in the present invention is a single domain antibody (sdAb).

For the term "single domain antibody" herein, the structure is not limited as long as the domain alone can exert antigen-binding activity. Normal antibodies such as an IgG antibody exhibit antigen-binding activity in a state in which a variable region is formed through pairing of VH and VL, whereas single domain antibodies are known to be capable of exerting antigen-binding activity only by their own domain structure without pairing with another domain. Single domain antibodies typically have relatively low molecular weight, and exist in the form of monomer.

Examples of the single domain antibody include, but are not limited to, VHH of an animal belonging to Camelidae, antigen-binding molecules congenitally lacking the light chain such as shark VNAR, and antibody fragments containing the whole or part of the VH domain of an antibody or the whole or part of the VL domain of an antibody. Examples of the single domain antibody as an antibody fragment containing the whole or part of the VH/VL domain of an antibody include, but are not limited to, a single domain antibody artificially produced from human antibody VH or human antibody VL as described in U.S. Patent No. 6,248,516 B1 and so on. In some modes of implementation of the present invention, one single domain antibody has three CDRs (CDR1, CDR2, and CDR3).

The single domain antibody can be obtained from an animal capable of producing a single domain antibody, or by immunizing an animal capable of producing a single domain antibody. Examples of the animals capable of producing the single domain antibody include, but are not limited to, camelids, and transgenic animals to which a gene capable of producing the single domain antibody has been introduced. Camelids include camels, llamas, alpacas, dromedaries, and guanacos. Examples of transgenic animals to which a gene capable of producing the single domain antibody has been introduced include, but are not limited to, transgenic animals described in International Publication No. WO 2015/143414 and U.S. Patent Publication No. US2011/0123527 A1. Alternatively, a humanized single domain antibody can be obtained by converting the framework sequence of the single domain antibody obtained from an animal into a human germline sequence or a sequence similar thereto. The humanized single domain antibody (e.g., humanized VHH) is also a mode of implementation of the single domain antibody in the present invention. The "humanized single domain antibody" refers to a chimeric single domain antibody containing an amino acid residue from non-human CDR and an amino acid residue from human FR. In the humanized single domain antibody in a certain mode, the whole or substantially whole CDR corresponds to that of a non-human antibody, and the whole or substantially whole FR corresponds to that of a human antibody. A case that some of the residues in FR in the humanized antibody do not correspond to those of a human antibody is also intended to be an example of the case in which substantially whole FR corresponds to that of a human antibody. If VHH, a mode of the single domain antibody, is humanized, for example, it is needed that some of the residues in FR be residues not corresponding to those of a human antibody (C Vincke et al., The Journal of Biological Chemistry 284, 3273-3284.).

The single domain antibody can be obtained from a polypeptide library containing the single domain antibody through ELISA, panning, or the like. Examples of the polypeptide library containing the single domain antibody include, but are not limited to, naive antibody libraries obtained from various animals or a human (examples: Methods in Molecular Biology 2012 911 (65-78), Biochimica et Biophysica Acta - Proteins and Proteomics 2006 1764:8 (1307-1319)), antibody libraries obtained by immunizing various animals (example: Journal of Applied Microbiology 2014 117:2 (528-536)), and synthetic antibody libraries produced from antibody genes of various animals or a human (examples: Journal of Biomolecular Screening 2016 21: 1 (35-43), Journal of Biological Chemistry 2016 291:24 (12641-12657), AIDS 2016 30:11 (1691-1701)).

- Library

Herein, "library" refers to a plurality of antigen-binding molecules each containing an antigen-binding domain with different sequences or/and nucleic acids or polynucleotides encoding a plurality of antigen-binding molecules each containing an antigen-binding domain with different sequences. The antigen-binding molecules each containing an antigen-binding domain or/and nucleic acids encoding antigen-binding molecules each containing an antigen-binding domain, as the contents of the library, are not in a single sequence, but are a plurality of antigen-binding molecules with different sequences or/and nucleic acids encoding a plurality of antigen-binding molecules with different sequences.

In an embodiment in the present disclosure, a fusion polypeptide of the antigen-binding molecule in the present disclosure and a heterologous polypeptide can be produced. In a certain embodiment, the fusion polypeptide is obtained through fusion with a viral coat protein, for example, at least a part of a viral coat protein selected from the group consisting of pIII, pVIII, pVII, pIX, Soc, Hoc, gpD, pVI, and mutants thereof.

The antigen-binding molecule in the present disclosure can be scFv, a Fab fragment, $F(ab)_2$, or $F(ab')_2$ in a certain embodiment, and thus, in another embodiment, a library primarily including a plurality of fusion polypeptides of any of those antigen-binding molecules and a heterologous polypeptide with different sequences is provided. Specifically, a library primarily including a plurality of fusion polypeptides, with different sequences, in which any of those antigen-binding molecules and a viral coat protein, for example, at least a part of a viral coat protein selected from the group consisting of pIII, pVIII, pVII, pIX, Soc, Hoc, gpD, pVI, and mutants thereof are fused together is provided. The antigen-binding molecule in the present disclosure can further contain a dimerized domain. In a certain embodiment, the dimerized domain can be present between the variable region of the heavy chain or light chain of an antibody and at least a part of a viral coat protein. In the dimerized domain, at least one of the dimerization sequences and/or a sequence containing one or more cysteine residues can be contained. Preferably, the dimerized domain can be linked to the C terminus of the heavy chain variable region or constant region. The dimerized domain can employ various structures depending on whether the antibody variable region has been produced as a fusion polypeptide component with a viral coat protein component (no amber stop codon is possessed behind the dimerized domain) or whether the antibody variable region has been produced without primarily containing a viral coat protein component (e.g., an amber stop codon is possessed behind the dimerized domain). If the antibody variable region is produced as a fusion polypeptide component with a viral coat protein component, bivalent presentation is provided by one or more disulfide bonds and/or single dimerization sequences. Examples of one non-limiting mode of the library in the present disclosure include a library with a diversity of $1.2 \times 10^8$ or higher, that is, a library including a plurality of antigen-binding molecules each containing an antigen-binding domain with different sequences or nucleic acids encoding a plurality of antigen-binding molecules each containing an antigen-binding domain with different sequences, where the number of the plurality of antigen-binding molecules is $1.2 \times 10^8$ or more.

Herein, the term "with different sequences" in the expression "a plurality of antigen-binding molecules each containing an antigen-binding domain with different sequences" means that the sequences of the individual antigen-binding molecules in the library are different from each other. Thus, to the number of different sequences in the library, the number of independent clones differing in their sequences in the library is reflected, which is occasionally referred to as "library size". The library size of normal phage display libraries is $10^6$ to $10^{12}$, and the library size can be extended to $10^{14}$ by applying a known technique such as a ribosome display method. However, the actual number of phage particles used in panning selection for a phage library is typically 10 to 10,000 times larger than the library size. This excessive multiple is also called "library equivalent", and indicates the presence of 10 to 10,000 individual clones with the same amino acid sequence. Therefore, the term "with different sequences" in the present disclosure means that the sequences of individual antigen-binding molecules in the library reduced in the number of antigen-binding molecules by the library equivalent are excluded, are different, more specifically, there exist $10^6$ to $10^{14}$ molecules, preferably $10^7$ to $10^{12}$ molecules, more preferably $10^8$ to $10^{11}$, particularly preferably $10^8$ to $10^{10}$ of antigen-binding molecules with different sequences.

The term "a plurality of" in the expression "the library primarily including a plurality of antigen-binding molecules each containing an antigen-binding domain with different sequences, or/and nucleic acids encoding a plurality of antigen-binding molecules each containing an antigen-binding domain with different sequences" in the present disclosure normally refers to a collection of two or more types of a substance such as the antigen-binding molecule, fusion polypeptide, polynucleotide molecule, vector, or virus in the present disclosure. If two or more molecules of a certain substance are different with respect to a particular character, for example, this indicates that there are two or more types for the substance. An example is mutants for which amino acid mutation is observed at a particular amino acid site in the amino acid sequence. For example, if there are two or more antigen-binding molecules with sequences in which amino acids other than that at a particular amino acid site are substantially identical, preferably identical, there is a plurality of antigen-binding molecules. For another example, if there are two or more polynucleotide molecules with sequences in which nucleotides other than that encoding an amino acid at a particular amino acid site are substantially identical, preferably identical, there is a plurality of polynucleotide molecules.

(Definition of Experimental Techniques)

[Characterization]

As an aspect of the present disclosure, a trained model is generated by performing machine learning on the basis of sequence information on antigen-binding molecules and evaluation result information on characterization of the antigen-binding molecules. Examples of a non-limiting mode of characterization of antigen-binding molecules include evaluation of affinity, evaluation of pharmacological activity, evaluation of physical properties, evaluation of kinetics, and evaluation of safety of antigen-binding molecules, but are not limited to these evaluations.

- Evaluation of Affinity

The technique of evaluation of affinity for antigen-binding molecules is not limited in any way, and evaluation can be made by measuring binding activity of each antigen-binding molecule and an antigen. "Binding activity" refers to the total intensity of non-covalent interactions between one or more binding sites of a molecule (e.g., an antibody) and a binding partner for the molecule (e.g., an antigen). Here, "binding activity" is not strictly limited to 1:1 interaction between members of a certain binding pair (e.g., an antibody and an antigen). When members of a binding pair reflect monovalent 1:1 interaction, for example, the meaning of binding activity is intrinsic binding affinity ("affinity"). If members of a binding pair are capable of both monovalent binding and multivalent binding, the binding activity is the sum total of avidities of them. The binding activity of a molecule, X, to its partner, Y, can be generally represented by a dissociation constant (KD) or the "amount of analyte bound per unit amount of ligand". Binding activity can be measured with any of common methods known in the art including those described herein. Those skilled in the art can appropriately determine conditions except the concentration of a compound specific to target tissue. In a particular mode, the antigen-binding molecule provided herein is an antibody, and the binding activity of the antibody is a dissociation constant (KD) of $\leq 1$ $\mu M$, $\leq 100 nM$, $\leq 10$ nM, $\leq 1nM$, $\leq 0.1nM$, $\leq 0.01$ nM, or $\leq 0.001$ nM (e.g., $10^{-8}$ M or lower, such as $10^{-8}$ M to $10^{-13}$ M, such as $10^{-9}$ M to $10^{-13}$ M).

In a mode, for the binding activity of an antibody, a ligand capture method, for example, with a BIACORE (R) T200 or BIACORE (R) 4000 (GE Healthcare, Uppsala, Sweden), which uses surface plasmon resonance analysis as the measurement principle, is used. For the machine operations, BIACORE (R) Control Software is used. In a mode, an amine coupling kit (GE Healthcare, Uppsala, Sweden) is used in accordance with instruction from the supplier to immobilize molecules for ligand capture such as an anti-tag antibody, an anti-IgG antibody, and protein A on a sensor chip coated with carboxymethyldextran (GE Healthcare, Uppsala, Sweden). The ligand capture molecules are diluted with 10 mM sodium acetate solution at an appropriate pH, and injected at an appropriate flow rate in an appropriate injection time. In measurement of binding activity, a buffer containing 0.05% polysorbate 20 (another name: Tween (R)-20) is used as a buffer for measurement, and measurement is performed at a flow rate of 10 to 30 $\mu L$/min preferably at

a measurement temperature of 25°C or 37°C. When measurement is carried out by allowing the molecules for ligand capture to capture an antibody as a ligand, the antibody is injected to allow an intended amount of the antibody to be captured, and a serially diluted product (analyte) of an antigen and/or Fc receptor prepared with the buffer for measurement is then injected. When measurement is carried out by allowing the molecules for ligand capture to capture an antigen and/or Fc receptor as a ligand, the antigen and/or Fc receptor are/is injected to allow an intended amount of the antigen and/or Fc receptor to be captured, and a serially diluted product (analyte) of an antibody prepared with the buffer for measurement is then injected.

In a mode, measurement results are analyzed by using BIACORE (R) Evaluation Software. In carrying out calculation of kinetics parameters, sensorgrams for association and dissociation are simultaneously fit with use of a 1:1 Binding model, and association rates (kon or ka), dissociation rates (koff or kd), and equilibrium dissociation constants (KD) can be calculated. If the binding activity is weak, in particular, if calculation of kinetics parameters is difficult because of fast dissociation, the equilibrium dissociation constant (KD) may be calculated with use of a Steady state model. As another parameter for binding activity, the "amount of analyte bound per unit amount of ligand" can also be calculated by dividing the amount of a bound analyte at a specific concentration (RU) by the amount of the captured ligand (RU).

If the antigen is a soluble molecule, KD (dissociation rate constant) can be used as a value for antigen-binding activity; if the antigen is a membrane-associated molecule, apparent kd (apparent dissociation rate constant) can be used. kd (dissociation rate constant) and apparent KD (apparent dissociation rate constant) can be measured with a method known to those skilled in the art, and, for example, Biacore (GE healthcare), a flow cytometer, and so on can be used.

Another mode of characterization is, for example, a selection technique for antigen-binding molecules with use of a display library. In a mode, an example is panning using phage display. In evaluation of affinity, for example, a phage presenting an antigen-binding molecule that interacts with a target antigen can be concentrated through a process that a phage library presenting different antigen-binding molecules is prepared, a target antigen is brought into contact with the phages prepared, and an operation to wash out unbound phages is then carried out. A sequence having affinity with the target antigen can be identified by analyzing the nucleic acid sequence encoding the antigen-binding molecule contained in the concentrated phage.

In another mode, an example is panning using mammalian cell display. In evaluation of pharmacological activity using the display system, for example, cells having an antigen-binding molecule gene with desired pharmacological activity can be isolated by using a flow cytometer or the like through a process that a library containing different antigen-binding molecules is expressed in targeted mammalian cells and reporter activity and so on are changed according to the action that it exhibits on the cells. In evaluation of physical properties using the display system, for example, cells having an antigen-binding molecule gene capable of stably expressing at high level can be isolated by using a flow cytometer or the like through a process that a library containing different antigen-binding molecules is expressed in targeted mammalian cells and the expression levels are examined by staining with antibodies specific to the antigen-binding molecules.

Characterization of antigen-binding molecules by panning is not limited to the techniques using phages or mammalian cells, and various techniques can be used as long as they allow presentation of antigen-binding molecules, and examples thereof include, but are not limited to, a technique to allow ribosomes to present, a technique to allow mRNA to present, a technique to allow viruses other than phages to present, and a technique to allow bacteria such as Escherichia coli to present.

Another mode of characterization is, for example, a method of obtaining an antibody gene sequence from immunocytes derived from an individual or a method of obtaining an antibody protein sequence from serum. In evaluation of affinity involving extraction of an antibody gene sequence from immunocytes, for example, a sequence having affinity with a target antigen can be identified through a process that a target antigen protein is administered to an individual to induce immune sensitization, and an antibody gene for an antibody that binds to the target antigen is extracted from immunocytes having the gene.

The antigen to cause immune sensitization is not limited to a protein as in the technique and a gene encoding the protein or cells expressing the protein can be used.

Examples of the individual as a subject include humans, mice, rats, hamsters, rabbits, monkeys, chickens, camels, llamas, and alpacas, but are not limited thereto.

Examples of techniques for the analysis of nucleic acid sequences or appearance frequency include a technique in which gene recombinant organisms having nucleic acid sequences for different antigen-binding molecules are cloned and analysis is performed through the Sanger method using capillary electrophoresis, and a technique to analyze with use of a next-generation sequencer, but are not limited thereto.

In the analysis of nucleic acid sequences, strength of a characteristic can be additionally determined on the basis of appearance frequency. For example, the characteristic of an antigen-binding molecule encoded by a sequence with high appearance frequency in analysis of nucleic acid sequences after concentration can be estimated to be high, and the characteristic of an antigen-binding molecule encoded by a sequence with low appearance frequency after concentration can be estimated to be lower than that of an antigen-binding molecule encoded by a sequence with high appearance frequency.

The techniques to acquire information on antigen-binding molecules derived from the display library or an individual are applicable to various types of characterization, and not limited to the above.

- Evaluation of Pharmacological Activity

The technique of evaluation of pharmacological activity for antigen-binding molecules is not limited in any way, and evaluation can be made, for example, by measuring neutralization activity, agonist activity, or cytotoxic activity exhibited by antigen-binding molecules. In evaluation of cytotoxic activity as an example of pharmacological activity, examples of cytotoxic activity to be evaluated include antibody-dependent cell-mediated cytotoxicity (ADCC) activity, complement-dependent cytotoxicity (CDC) activity, T-cell-dependent cytotoxicity (TDCC) activity, and antibody-dependent cellular phagocytosis (ADCP) activity. CDC activity is cytotoxic activity due to the complement system. ADCC activity is such activity that an immunocyte or the like binds to the Fc region of an antigen-binding molecule containing an antigen-binding domain that binds to a membrane-associated molecule expressed on the cell membrane of a target cell via the Fcγ receptor expressed on the immunocyte, and the immunocyte causes damage to the target cell. TDCC activity is such activity that a T cell causes disorder to a target cell through bringing the target cell and the T cell into close proximity with use of a bi-specific antibody containing an antigen-binding domain that binds to a membrane-associated molecule expressed on the cell membrane of the target cell and an antigen-binding domain against any of the constituent subunits of the T cell receptor (TCR) complex on the T cell, in particular, an antigen-binding domain that binds to the CD3 epsilon chain. Whether an antigen-binding molecule of interest has ADCC activity, CDC activity, TDCC activity, or ADCP activity can be determined by a known method.

Neutralization activity is activity that is against ligands having biological activity against cells, such as viruses and toxins, and inhibits the biological activity. That is, a substance having neutralization activity is a substance that binds to such a ligand or a receptor to which the ligand binds to inhibit binding between the ligand and the receptor. A receptor that has been prevented from binding to the ligand by neutralization activity comes to be disabled from exerting the biological activity mediated by the receptor. When the antigen-binding molecule is an antibody, an antibody having such neutralization activity is generally called a neutralizing antibody, and the neutralization activity can be determined by measuring the inhibitory activity to binding between the ligand and a receptor. The ligand having biological activity to cells is not limited to viruses, toxins, and so on, and inhibitory activity to physiological action evoked by binding of an endogenous ligand such as cytokine and chemokine to a receptor is also understood as neutralization activity. Neutralization activity is not limited to cases with inhibition of binding between a ligand and a receptor, and activity to inhibit the function of a protein having biological activity is also understood as neutralization activity, and examples of the function of a protein include enzymatic activity.

- Evaluation of Physical Properties

The technique of evaluation of physical properties for antigen-binding molecules is not limited in any way, and examples of physical properties include thermal stability, chemical stability, solubility, viscosity, photostability, long-term storage stability, and non-specific adsorptivity. In evaluation of the various physical properties exemplified, they can be measured with methods known to those skilled in the art. The evaluation methods are not limited in any way, and, in evaluation of stability such as thermal stability, chemical stability, photostability, stability to mechanical stimulation, and long-term storage stability, for example, evaluation can be made by measuring the decomposition, chemical modification, and association of an antigen-binding molecule of interest before and after treatment intended the evaluation of stability such as heat treatment, exposure to a low-pH environment, exposure to light, stirring with a machine, and long-term storage. Examples of one non-limiting mode of the measurement method involving such evaluation of stability include techniques using chromatography such as ion-exchange chromatography chromatography and size exclusion chromatography, mass spectrometry, and electrophoresis, but are not limited thereto, and measurement can be performed with various techniques known to those skilled in the art.

Examples of evaluation of physical properties other than the above-described evaluations include evaluation of solubility of protein by a polyethylene glycol precipitation method, evaluation of viscosity by a small-angle X-ray scattering method, and evaluation of non-specific binding based on evaluation of binding to the Extracellular Matrix (ECM), but are not limited thereto.

Even for evaluation of protein expression levels, evaluation of binding to a resin for purification or ligand for purification, and evaluation of surface electric charge, evaluation can be made as evaluation of physical properties as long as measurement can be performed with a technique known to those skilled in the art.

- Evaluation of Kinetics

The technique of evaluation of kinetics for antigen-binding molecules is not limited in any way, and evaluation can be made by administering an antigen-binding molecule to an animal such as a mouse, a rat, a monkey, and a dog and measuring the amount of the antigen-binding molecule in the blood after administration over time, and evaluation can be made with a technique widely known to those skilled in the art as pharmacokinetics (PK) evaluation. In addition to the technique to directly evaluate PK, the behavior of an antigen-binding molecule in kinetics can be predicted from the amino acid sequence of an antigen-binding molecule by calculating the surface electric charge, isoelectric point, and so on of the antigen-binding molecule with software.

- Evaluation of Safety

The technique of evaluation of safety for antigen-binding molecules is not limited in any way, and examples thereof include an immunogenicity prediction tool such as ISPRI Web-Based Immunogenicity Screening (EpiVax, Inc.), evaluation of HLA binding of fragment peptides of an antigen-binding molecule, detection of a T-cell epitope by using MAPPs (MHC-Associated Peptide Proteomics) or evaluation of T-cell growth, and evaluation of immunogenicity. Evaluation can be made as long as measurement can be performed with a technique known to those skilled in the art such as evaluation of immunoreaction using binding to the rheumatoid factor (RF), PBMC, or whole blood and evaluation of platelet aggregation.

(Definitions of Terms and Techniques (LSTM, RF) Used in Machine Learning)

An RNN (Recurrent Neural Network) is a neural net formed by connecting a plurality of neural nets. An example of application thereof to peptide sequences is disclosed in Muller AT et al. (J Chem Inf Model. 2018 Feb 26;58(2):472-479.).

An LSTM (Long Short-Term Memory), a special form of the RNN, is an RNN configured to have superior long-term memory.

A GRU (Gated Recurrent Unit), a special form of the RNN, is an RNN in which neurons corresponding to long-term memory are present. A GAN is an adversarial network, being machine learning aiming for more accurate classification by using both a model that attempts accurate classification and a model that generates deceiving samples.

A VAE (Variational AutoEncoder) is what is trained with supervised learning using the same data for an input layer and an output layer in a neural net on the basis of the variational method.

A flow-based deep generative model is a model that learns the distribution of data by reversible change of variables on the basis of log likelihood.

A gaussian process is machine learning that outputs not only predicted values but also the distribution of predicted values for certain inputs. The distribution of predicted values is considered as the reliability of predicted values. Bayesian optimization is a technique to predict with higher precision through sampling (actually measuring) points that improve prediction precision on the basis of prediction results with a Gaussian process and updating a prediction model including the new actual measurements. Update of a prediction model can be repeatedly performed.

- Probability Model (HMM, MM)

An MM (Markov Model) is a model provided with a plurality of states and probabilities of transition between states. Information only on the previous state determines the probability of transition to the next state.

An HMM (Hidden Markov Model) is a model that is provided with a plurality of states and probabilities of transition between states, and outputs different quantities in different states with probabilities defined for respective states. Information only on the previous state determines the probability of transition to the next state.

- Techniques to Recognize Amino Acids (Character String, Numeric Vector, Sequence Information Represented by Physical Property Values)

What is first thought of as a machine learning technique for antibody sequences is, a method of inputting a character string with regarding a sequence as a character string. Also considered is a method of converting a character at each position in a sequence into a numeric value by using the physical property values (the molecular weight, electric charge, degree of hydrophobicity, volume of the side chain, etc.) and inputting the numeric values. Further considered is a method of converting the character string of a full-length sequence into numeric values by using statistics on which amino acid is likely to appear around each amino acid (Doc2Vec method).

If the Doc2Vec method is used, for example, a computer regards the amino acid sequence of a subject as a text. The computer seeks the amino acid sequence in order from the beginning of the sequence to the end, dividing the amino acid sequence into groups of a predetermined number (for example, three but may be any number other than three) of amino acids. For each group after division, the computer generates, as a word, a character string being an array of aligned characters each representing an amino acid. The computer maps the amino acid sequence, which is an array of the groups of amino acids aligned, as a text being an array of sequentially aligned words in a vector space by using the Doc2Vec method. Thus, a computer may analyze sequences by using a technique for document analysis with regarding a sequence as a text and regarding groups of amino acids as words.

(First Embodiment)

In the following, the first embodiment of the present invention will be described in detail with reference to drawings.

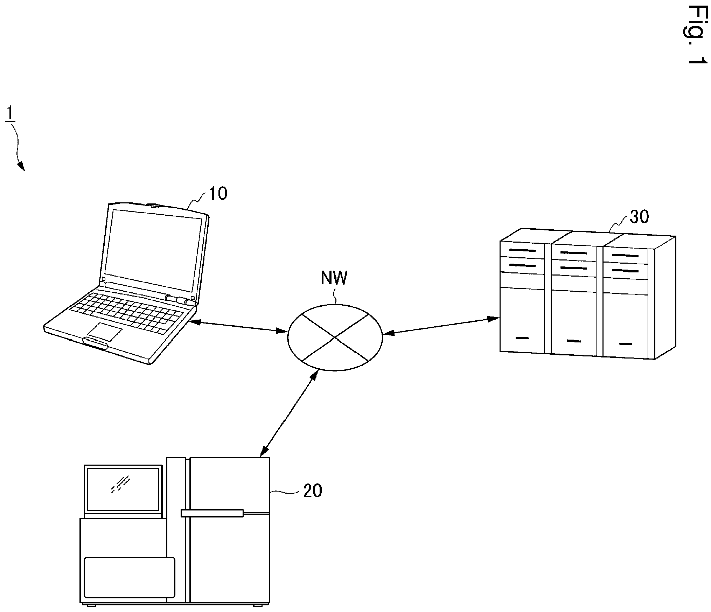

In the present embodiment, a server 30 in an information processing system 1 in Figure 1 performs machine learning on the basis of sequence information representing sequences of antigen-binding molecules and thereby generates a model trained with sequence learning (an example of the "first trained model"). The model trained with sequence learning is a trained model that has learned the character of the sequences represented by inputted sequence information and, as a result of learning, outputs prediction target sequence information (an example of the "virtual sequence information"). The prediction target sequences represented by the prediction target sequence information are virtual sequences obtained by mutating at least one of constituent amino acids in at least one of the sequences of antigen-binding molecules used for learning.

Thereby, the information processing system 1 can generate, for an antigen-binding molecule, virtual sequences obtained by mutating some of the amino acids. For example, the model trained with sequence learning is trained with use of sequence information on antigen-binding molecules having a desired property. In this case, the server 30 can generate virtual sequences that are likely to have the desired property, for example, as a group of many virtual sequences. In the present embodiment, an example with characterization using frequency of binding to an antigen through panning using an antigen will be described. Panning will be described later.

<Information Processing System>

Figure 1 shows a schematic diagram illustrating an example of the information processing system 1 according to the first embodiment.

The information processing system 1 includes a user terminal 10, a next-generation sequencer 20, and a server 30. The user terminal 10, the next-generation sequencer 20, and the server 30 are connected together via a network NW The network NW is, for example, a LAN (Local Area Network) or an information and communication network such as the Internet. The information and communication network may be a wired or wireless network, or a network formed by combining various networks. The user terminal 10, the next-generation sequencer 20, and the server 30 may communicate data thereamong via a storage medium or the like such as an HDD (hard disk drive) and a USB memory.

The user terminal 10 is a personal computer with which a user performs input/output. The user terminal 10 may be a portable terminal such as a tablet terminal and a smartphone.

The next-generation sequencer 20 is a device that analyzes nucleotide sequences of DNA (deoxyribonucleic acid).

The server 30 is an information processer such as a server. The server 30 performs learning with use of analysis result information representing analysis results from the next-generation sequencer 20. On the basis of input information from the user terminal 10 and learning results, the server 30 sends output information to the user terminal 10.

For example, in panning of a plurality of antibodies and a target antigen (an example of the "evaluation of affinity"), the next-generation sequencer 20 measures and analyzes each of the antibodies contained in a sample, and outputs analysis result information including sequence information representing the nucleotide sequences of the antibodies and the appearance frequencies (an example of "evaluation result information in evaluation of affinity"; also called number of reads). Appearance frequency is the number proportion of each sequence of the sequences of antibodies that have bound to the target antigen to the total number (total number of reads) of sequences analyzed by the next-generation sequencer 20. However, the present invention is not limited to this, and appearance frequency may be the number of sequences analyzed by the next-generation sequencer 20.

The server 30 receives analysis result information via the network NW or a storage medium, and generates, as a learning dataset, a group of sequences having a desired property according to sequence information and appearance frequencies included in the analysis result information (an example of acquisition). The server 30 learns on the basis of the learning dataset, and stores a model trained with sequence learning, which has learned the characters of sequences having a desired property. The server 30 generates, as prediction target sequences, a group of new virtual sequences having the character of the sequences with a desired property on the basis of the stored model trained with sequence learning.

Thereafter, for the prediction target sequence information representing the prediction target sequences generated, the server 30 gives prediction scores indicating affinity information on affinity with the target antigen (an example of "affinity information representing affinity with a target antigen") with use of a model trained with learning of characteristic prediction (an example of the "second trained model") described later. The affinity information is information indicating whether each antibody binds to the target antigen (binding antibody) or not (non-binding antibody).

According to the prediction scores given, the server 30 sends candidate antibody information representing candidate antibodies expected to bind to the target antigen to the user terminal 10. The user terminal 10 displays candidate antibody information according to the prediction scores received.

Thereby, the information processing system 1 can generate much sequence information narrowed down to sequences estimated to have a higher characteristic than in the case in which sequence information to estimate a characteristic against the target antigen is randomly generated. Accordingly, the information processing system 1 can provide information on desired antibodies with more reduced processing time or processing loads.

<Evaluation of Affinity (Panning)>

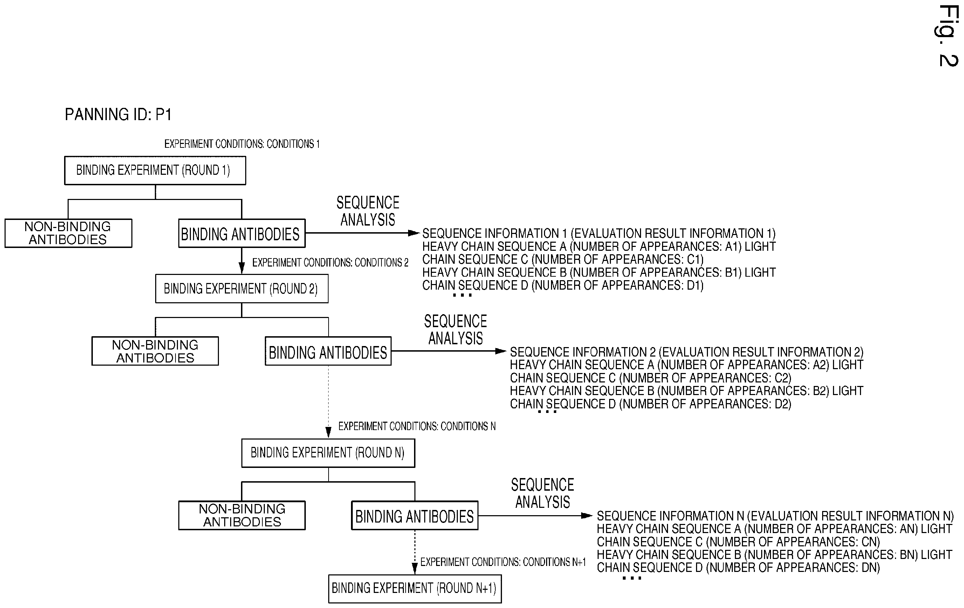

Figure 2 shows an illustration for describing an example of a sequence of panning according to the present embodiment.

An operation of panning ("BINDING EXPERIMENT" in the illustration) repeated in a sequence of panning will be described. In the sequence of panning, the mth operation of panning (m = 1 to M: m is a natural number) is also referred to as panning in the mth round or panning in round m. Each operation of panning is carried out through the following four steps of (P1) to (P4):

(P1) Reaction between the target antigen and antibodies
(P2) Washing-out of antibodies that have not bound to the target antigen ("NON-BINDING ANTIBODIES" in the illustration)
(P3) Elution of antibodies that have bound to the target antigen ("BINDING ANTIBODIES" in the illustration)
(P4) Amplification of DNAs to serve as templates in production of eluted antibodies

Here, antibodies are associated with the DNAs in one-to-one relationship with any coexisting antibody display technique.

In the first operation of panning (round 1), a collection of a plurality of antibodies (hereinafter, also referred to as an "antibody library") is subjected to panning. This collection in round 1 has been prepared in advance. In the second or later round, a collection of binding antibodies determined to have bound to the target antigen (an example of "antibodies having affinity with the target antigen") in the previous round is subjected to panning. In other words, in the second or later round, a collection of non-binding antibodies determined not to have bound to the target antigen (an example of "antibodies having low affinity") in the previous round is not subjected to panning. More specifically, antibodies to be subjected to the second or later round (binding antibodies in the immediately preceding round) are produced with use of DNAs amplified in the immediately preceding round. The sequence of panning ends, for example, when the operation of panning has been repeated for a predetermined number of rounds. However, the present invention is not limited to this, and the sequence of panning may end when the amounts of binding antibodies have become insufficient or when the experimenter has made such decision.

In each operation of panning, experiment conditions (an example of "evaluation conditions") are set. Experiment conditions are changeable conditions in reaction between the target antigen and antibodies. Experiment conditions include target antigen conditions, antibody conditions, conditions for a solution present in a place where reaction is performed, and reaction time and reaction temperature for the target antigen and antibodies.

The target antigen conditions include, for example, the concentration of the target antigen and the molecular information on the target antigen. The concentration of the target antigen indicates the concentration of the target antigen in a place where the target antigen and antibodies react (in a reaction solution). The molecular information on the target antigen includes, for example, the sample name and the amino acid sequence.

The antibody conditions include, for example, the antibody display technique, the origin of antibodies, the domain type, and the germline. The antibody display technique indicates an antibody display technique for antibodies to be subjected to panning. The origin of antibodies indicates the origin of antibodies to be subjected to panning, and is, for example, a human, a mouse, a rat, a hamster, a rabbit, a monkey, a chicken, a camel, a llama, an alpaca, or artificial synthesis. The domain type is, for example, a heavy chain or a light chain.

The conditions for a solution include, for example, the composition of a buffer (solution). "Buffer composition" refers to conditions for a solution such as the solution composition of a reaction solution and hydrogen-ion exponent (pH).

The reaction time indicates time for the target antigen and antibodies to coexist in a solution. The reaction temperature indicates a preset temperature when the target antigen and antibodies coexist in a solution.

In the example in Figure 2, the experiment conditions in round 1 correspond to CONDITIONS 1, and the experiment conditions in round 2 correspond to CONDITIONS 2. Experiment attribute information representing such experiment conditions can be managed for each operation of panning in the server 30. However, identical experiment conditions may be used in a sequence of rounds, and, in this case, CONDITIONS 1, 2, ..., N, N+1 in Figure 2 are identical experiment conditions.

In each operation of panning, DNAs for antibodies are amplified after step (P3), and thereafter the nucleotide sequences are analyzed by the next-generation sequencer 20. The next-generation sequencer 20 outputs, as antibody-by-antibody analysis results for a plurality of antibodies, sequence information representing the nucleotide sequences for the antibodies and evaluation result information on the antibodies. The evaluation result information includes, for example, the appearance frequency in each round and the round-to-round change rates of appearance frequency for each antibody. The nucleotide sequences are converted into amino acid sequences in the server 30.

Here, one antibody is composed of a heavy chain (H chain) part and a light chain (L chain) part combined together. In sequence information on one antibody in the analysis result information, the amino acid sequence of the heavy chain (H chain) part (also referred to as the "heavy chain sequence"), or the amino acid sequence of the light chain (L chain) part (also referred to as the "light chain sequence") are separately measured and analyzed. In other words, even if the next-generation sequencer 20 fails to identify a combination of the heavy chain sequence and the light chain sequence of an antibody, the next-generation sequencer 20 succeeds in identifying the heavy chain sequence and the light chain sequence. The next-generation sequencer 20 separately outputs sequence information representing heavy chain sequences and evaluation result information thereon and sequence information representing light chain sequences and evaluation result information thereon.

However, the present invention is not limited to this, and the next-generation sequencer 20 may measure a heavy chain and a light chain at once and output sequence information representing heavy chain sequences and light chain sequences and evaluation result information thereon.

Figure 2 shows that the next-generation sequencer 20 outputs, as analysis result information in the operation of panning in round 1, heavy chain sequence A, heavy chain sequence B, light chain sequence C, and light chain sequence D as sequence information on binding antibodies. In addition, Figure 2 shows that the next-generation sequencer 20 outputs, as result information in the operation of panning in round 1, appearance frequency A1 of heavy chain sequence A, appearance frequency B1 of heavy chain sequence B, appearance frequency C1 of light chain sequence C, and appearance frequency D1 of light chain sequence D.

In the above, the next-generation sequencer 20 determines nucleotide sequences for binding antibodies on the basis of sequence information and evaluation result information acquired from a sequence of panning.

As the sequence of panning in Figure 2 is regarded as one set, a plurality of sets of the sequence of panning may be carried out as panning. For example, the same target antigen is used in all the sets of the sequence of panning. At least one set of the sequence of panning is different from the other sets of the sequence of panning in at least one of the antibody library and experiment conditions.

Through the sequence of panning, the server 30 acquires a learning dataset for each operation of panning in rounds 1 to M, and learns on the basis of these learning datasets. Here, the learning datasets include a learning dataset for at least one round, that is, a learning dataset on sequences after an operation of panning in round I.

In the sequence of panning, an operation of panning in a certain round (e.g., round N+1) is carried out with use of antibodies (an example of antibodies having affinity) that have appeared in an operation of panning in the previous round (e.g., round N) and the target antigen. Here, the antibodies that have appeared may be each an antibody with an appearance frequency higher than a predetermined threshold, or an antibody with a rank of appearance frequency higher than a predetermined rank.

Thus, in the sequence of panning, a subsequent operation of panning is carried out with the target antigen for antibodies that have appeared in an operation of panning in the previous round. The information processing system 1 acquires learning datasets from such operations of panning to learn.

Thereby, more learning datasets on antibodies with high appearance frequency can be generated, in contrast to the case without learning datasets from the sequence of panning. Accordingly, the information processing system 1 can clarify the character of antibodies with high appearance frequency to a higher degree. Or, the information processing system 1 can progressively narrow down the types of antibodies as compared with learning of learning datasets from panning with numerous types of antibodies in every operation of panning. Thereby, the information processing system 1 can achieve reduced processing time or processing loads.

<User Terminal>

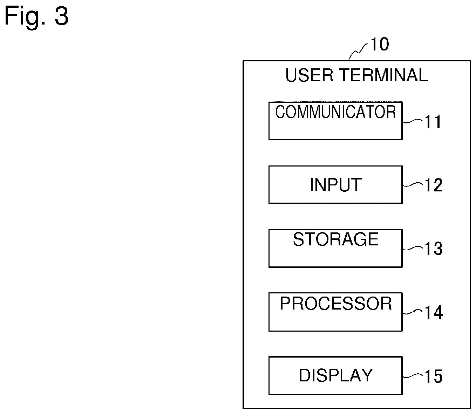

Figure 3 shows a block diagram illustrating an example of the user terminal 10 according to the present embodiment.

The user terminal 10 includes a communicator 11, an input 12, a storage 13, a processor 14, and a display 15.

The communicator 11 is a communication module that performs various types of communication via the network NW The communicator 11 performs various types of communication, for example, between itself and the server 30.

The input 12 is, for example, an input device such as a keyboard and a touch panel. The input 12 receives input information based on a user operation. The input 12 outputs the input information received to the processor 14.

The storage 13 is, for example, a storage device such as a hard disk drive and a memory. The storage 13 stores various programs such as firmware and application program for the processor 14 to execute, results of processing executed by the processor 14, and so on.

The processor 14 is, for example, a processor such as a central processor (CPU). For example, the processor 14 sends various types of information such as input information inputted from the input 12 to the server 30 via the communicator 11. The server 30 stores relationship information between input information and output information (e.g., a trained model, a table) in advance, and generates output information for input information. The processor 14 receives the output information generated by the server 30 via the communicator 11. The processor 14 orders the display 15 to display (an example of the output) the output information received.

If the storage 13 stores relationship information, the processor 14 may read out the relationship information for input information to generate output information, and order the display 15 to display the output information.

The display 15 is, for example, a display such as an organic electroluminescence display and a liquid crystal display. The display 15 displays in accordance with display information generated by the processor 14.

<Screen Flow in User Terminal>

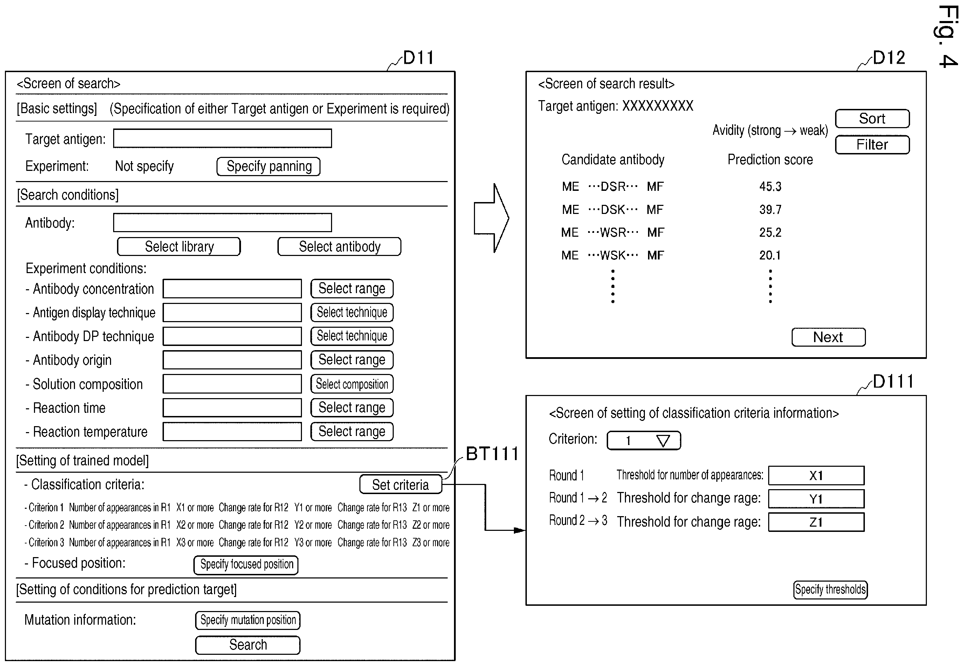

Figure 4 shows a diagram illustrating an example of screen flow according to the present embodiment.

This diagram shows an example of screen flow displayed by the display 15. A screen D11 is a screen for the input 12 to receive input information. A screen D12 is a screen to set classification criteria, and displayed when a button BT111 is pressed. The screen D12 is a screen on which the processor 14 orders to display output information after items on the screen D11 are filled and a search button is then pressed.

In the screen D11, the input 12 receives as input information, for example, at least one of target antigen information ("Target antigen" in the diagram), experiment information ("Experiment" in the diagram), experimental antibody information ("Antibody" in the diagram), experiment attribute information ("Experiment conditions" in the diagram), classification criteria information ("Classification criteria" in the diagram), focused position information ("Focused position" in the diagram), and mutation information. Here, the target antigen information is information that enables specification of the target antigen. The target antigen information is, for example, the name of the target antigen, and may be the sequence of an antigen or an identifier for an antigen. The experiment information is information that enables specification of the experiment such as information to identify a sequence of panning (also referred to as a "panning group") or a round (one operation of panning) and information representing the contents of the experiment. The experimental antibody information is information that enables specification of a collection of antibodies to be subjected to panning. The experimental antibody information is, for example, a name that identifies an antibody library to be subjected to an operation of panning in round 1. However, the present invention is not limited to this, and the experimental antibody information may be the names or amino acid sequences of one or more antibodies.

The experiment attribute information is information representing conditions changeable for each evaluation in panning. The experiment attribute information is, for example, information representing the above-described experiment conditions and infection titers (cfu) of eluted phages obtained from each experiment.

The classification criteria information (an example of criteria for affinity in evaluation of affinity) representing classification criteria for classifying antibodies into binding antibodies and non-binding antibodies in the stage of learning. In the stage of experiment, a group of sequences isolated in characterization experiment such as panning may include a sequence lacking a desired property in some cases. With setting classification criteria information inputted by a user, the server 30 can again classify antibodies analyzed by the next-generation sequencer 20 into binding antibodies and non-binding antibodies in the stage of learning. Thereby, the server 30 can determine, in some cases, antibodies determined as binding antibodies by mistake in the stage of experiment as non-binding antibodies in the stage of learning. In this case, the information processing system 1 can achieve increased precision for binding antibodies and thus enhanced classification precision.

The classification criteria information is thresholds for appearance frequencies or round-to-round change rates of appearance frequency. These thresholds may be set for each round, or set for each sequence of panning.

The classification criteria information includes information on a plurality of candidates (Criterion 1, Criterion 2, Criterion 3 in the diagram) (also referred to as classification criteria candidate information). For Criterion 1, 2, and 3, three thresholds have been inputted. Thresholds can be set for appearance frequency in each round or round-to-round change rates of appearance frequency. In the stage of learning, antibodies are classified according to criteria indicated by a plurality of pieces of classification criteria candidate information, and first and second trained models are generated. Of these first and second trained models, the server 30 selects the trained model with higher precision (the reproducibility of analysis result information being higher). In this way, the server 30 validates a plurality of candidates even for classification criteria to classify into binding antibodies and non-binding antibodies. Thereby, the information processing system 1 can achieve enhanced classification precision as compared with the case with fixed classification criteria.

The focused position information is information representing positions of amino acids in antibodies. The focused position information is used for narrowing sequence information to be learned down to sequence information only on particular positions in antibodies. For example, the focused position information is information representing positions of amino acids supposed to be important for binding to the target antigen in the variable region of antibodies.

With setting focused position information inputted by a user, the server 30 narrows the sequence information down to sequence information on a part represented by the focused position information to learn (with first and second learning models). Thereby, the information processing system 1 can reduce sequence information into that on a focused part, and hence can achieve reduction of processing time and processing loads required for learning as compared with the case with learning of full sequences. In addition, availability of setting focused position information allows learning with parts of good classification precision.

The focused position information may be automatically set by input from a computer.

The mutation information is information representing positions of amino acids in antibodies. The mutation information is used for sequences of antibodies for which prediction scores are calculated (referred to as "prediction target sequences") to narrow down parts intended to change in sequence information. The mutation information is information representing positions of amino acids supposed to be important for binding to the target antigen, for example, on the basis of positions that have provided an improved dissociation constant in other evaluation of affinity, the structure information on the target antigen, and so on.

The mutation information may be positions inputted by a user or positions inputted by a computer.

In the screen D12, the processor 14 has ordered to display, as output information, target antigen information ("Target antigen" in the diagram), candidate antibody information ("Candidate antibody" in the diagram) representing candidate antibodies that bind to the target antigen, and prediction scores indicative of degree of binding to the target antigen. For candidate antibody information, the processor 14 orders the display 15 to display candidates with high degree of binding (e.g., top 20 candidates) in descending order in degree of binding. Thus, the display 15 outputs candidate antibody information with considering degree of binding to the target antigen.

Each prediction score may be probability to bind, or a value of appearance frequency itself, or a value normalized by the maximum value of appearance frequency. Alternatively, each prediction score may be a value obtained by performing a certain operation for appearance frequency.

Now, the use case of the screen in Figure 4 will be described.

In the screen D11, a user sets at least either one of target antigen information and experiment information as a basic setup. Setting of at least either one of target antigen information and experiment information is necessary, but setting of the other items is arbitrary. A user can specify one or more combinations from panning groups or rounds as experiment information.

In the screen D11, a user can set antibody information or experimental antibody information as search conditions in the screen D11. If search conditions are set in the screen D11, candidate antibody information on candidates satisfying the search conditions set is outputted on the screen D12.

In the screen D11, a user can edit or add each of a plurality of classification criteria as setting of classification criteria information of the screen D11. A user can specify each of criteria to set category (appearance frequency or change rate), round number, and a threshold for the category.

In the screen D11, a user can set focused position information as setting of focused positions.

In the screen D11, a user can set mutation information as setting of search conditions for prediction targets. If mutation information is set in the screen D11, candidate antibody information on candidates with amino acid sequences being different at positions indicated by the mutation information is outputted on the screen D12. In other words, in this case, candidate antibody information on candidates with amino acid sequences being identical at positions other than the positions indicated by the mutation information is outputted on the screen D12.

<Next-Generation Sequencer>

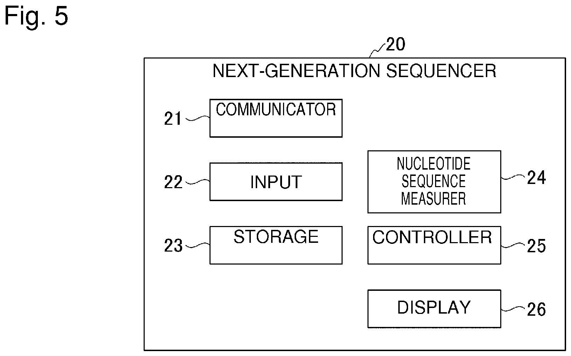

Figure 5 shows a block diagram illustrating an example of the next-generation sequencer 20 according to the present embodiment.

The next-generation sequencer 20 includes a communicator 21, an input 22, a storage 23, a nucleotide sequence measurer 24, a controller 25, and a display 26.

The communicator 21 is a communication module that performs various types of communication via the network NW The communicator 21 performs various types of communication, for example, between itself and the server 30. However, the present invention is not limited to this, and the next-generation sequencer 20 may include an output port that outputs data to a storage medium in place of or in addition to the communicator 21.

The input 22 is, for example, an input device such as a keyboard and a touch panel. The input 22 receives input information based on a user operation. The input 22 outputs the input information received to the controller 25.

The storage 23 is, for example, a storage device such as a hard disk drive and a memory. The storage 23 stores various programs such as firmware and application program for the controller 25 to execute, results of processing executed by the controller 25, and so on.

The nucleotide sequence measurer 24 is a sequencer that measures nucleotide sequences. In the nucleotide sequence measurer 24, a sample obtained as a result of panning is placed. The nucleotide sequence measurer 24, as commanded by the controller 25, measures nucleotide sequences contained in the sample placed. The nucleotide sequence measurer 24 outputs measurement results to the controller 25.

The controller 25 is a processor such as a central processor (CPU). The controller 25 controls next-generation sequencing by controlling the nucleotide sequence measurer 24, for example, on the basis of input from the input 22. The controller 25 analyzes the measurement results provided by the nucleotide sequence measurer 24 to calculate sequence information for each of antibodies contained in the sample. This sequence information is sequence information on heavy chain sequences or light chain sequences of antibodies.

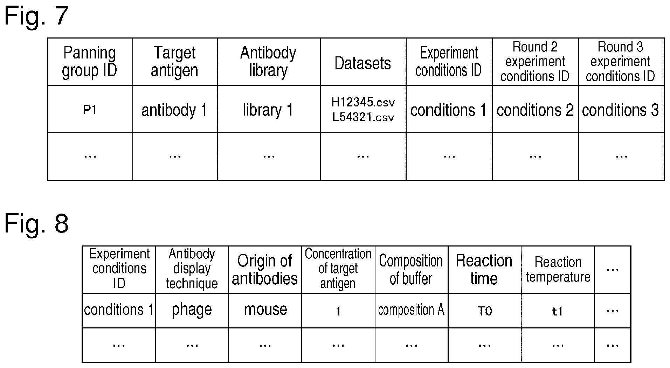

The controller 25 generates the input information inputted from the input 22, analysis result information associated with the calculated sequence information and appearance frequency (see Figure 7, Figure 8), and analysis result information associated with the calculated sequence information. Here, the input information includes, for example, a panning group ID to identify the sequence of panning, target antigen information on the target antigen subjected to panning, round number indicating the number of rounds, measured antibody information on antibodies measured in each round, and experiment condition information in the sequence of panning.

The controller 25 sends analysis result information on one or more operations of panning to the server 30 via the communicator 21. The controller 25 orders the display 26 to display various operation screens and an information input screen, various types of information on the progress of next-generation sequencing, and so on.

The input information may include setting information to be used for controlling the nucleotide sequence measurer 24. The appearance frequency of each piece of sequence information is calculated by the server 30 with use of the analysis result information. However, the present invention is not limited to this, and the next-generation sequencer 20 or another computer may calculate the appearance frequency of each piece of sequence information with use of the analysis result information.

The display 26 is, for example, a display such as an organic electroluminescence display and a liquid crystal display. The display 26 displays in accordance with display information generated by the controller 25.

<Servers>

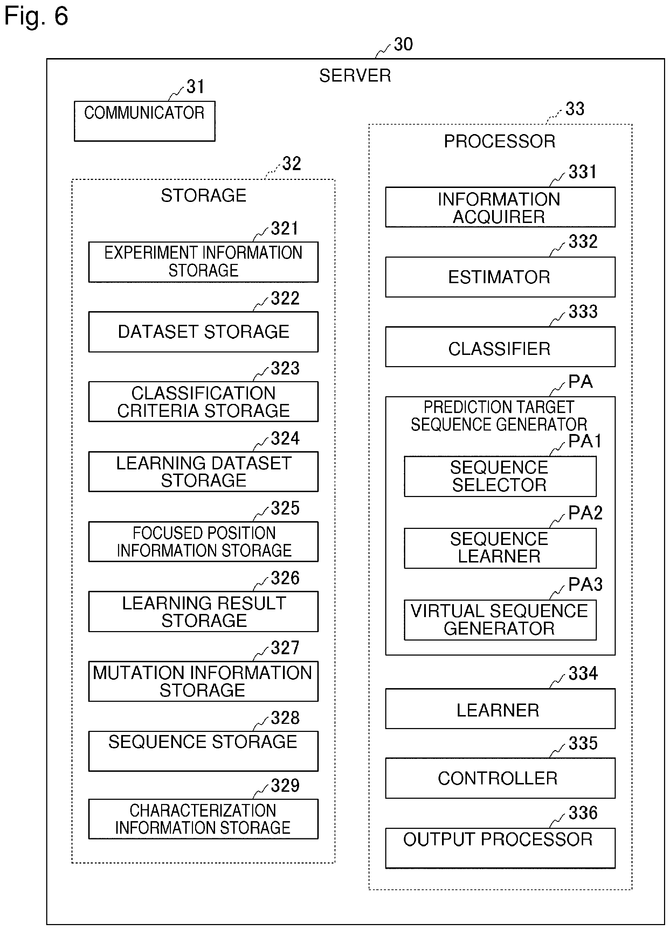

Figure 6 shows a block diagram illustrating an example of the server 30 according to the present embodiment.

The server 30 includes a communicator 31, a storage 32, and a processor 33.

The communicator 31 is a communication module that performs various types of communication via the network NW The communicator 31 performs various types of communication, for example, between itself and the user terminal 10 or the next-generation sequencer 20.

The storage 32 is, for example, a storage device such as a hard disk drive and a memory. The storage 32 stores various programs such as firmware and application program for the processor 33 to execute, results of processing executed by the processor 33, and so on.

The processor 33 is, for example, a processor such as a central processor (CPU). The processor 33 generates output information for input information inputted from the communicator 31, for example, on the basis of the input information and information stored in the storage 32. The communicator 31 sends the output information generated to the user terminal 10 via the communicator 31.

Specifically, the processor 33 acquires analysis result information from the next-generation sequencer 20 via the communicator 31, and stores it as datasets in the storage 32. At this time, the processor 33 converts nucleotide sequences contained in the information acquired into the corresponding amino acid sequences. The processor 33 generates learning datasets on the basis of the datasets stored, and learns on the basis of the learning datasets generated.

For example, the processor 33 first selects pieces of sequence information with desired appearance frequency (e.g., equal to or higher than a threshold). The processor 33 learns the character of the sequences in the pieces of sequence information selected to generate a model trained with sequence learning. Subsequently, the processor 33 learns, as a learning dataset, binding determination information according to the sequence information and the appearance frequency or round-to-round change rates of appearance frequency for each panning group ID or target antigen information. The processor 33 generates a model trained with learning of characteristic prediction as a learning result.

The processor 33 stores, as learning results, the model trained with sequence learning that has learned the character of sequences and the model trained with learning of characteristic prediction to give prediction scores in the storage 32.

The processor 33 acquires input information (e.g., target antigen information, experiment information, experimental antibody information, experiment attribute information, classification criteria information, focused position information, and mutation information) from the user terminal 10 via the communicator 31. The processor 33 generates prediction target sequence information by using the model trained with sequence learning. The processor 33 inputs the prediction target sequence information into the model trained with learning of characteristic prediction and outputs prediction scores. According to the prediction scores, the processor 33 generates candidate antibody information representing candidate antibodies that bind to the target antigen. The processor 33 sends the candidate antibody information generated to the user terminal 10 via the communicator 31.

<Storage of Server>

Details of the storage 32 will be described. The storage 32 includes an experiment information storage 321, a dataset storage 322, a classification criteria storage 323, a learning dataset storage 324, a focused position information storage 325, a learning result storage 326, a mutation information storage 327, a sequence storage 328, and a characterization information storage 329.

The experiment information storage 321 stores experiment information (see Figure 7) and experiment attribute information (see Figure 8). These sets of information are included in the analysis result information from the next-generation sequencer 20, and outputted by the processor 33.

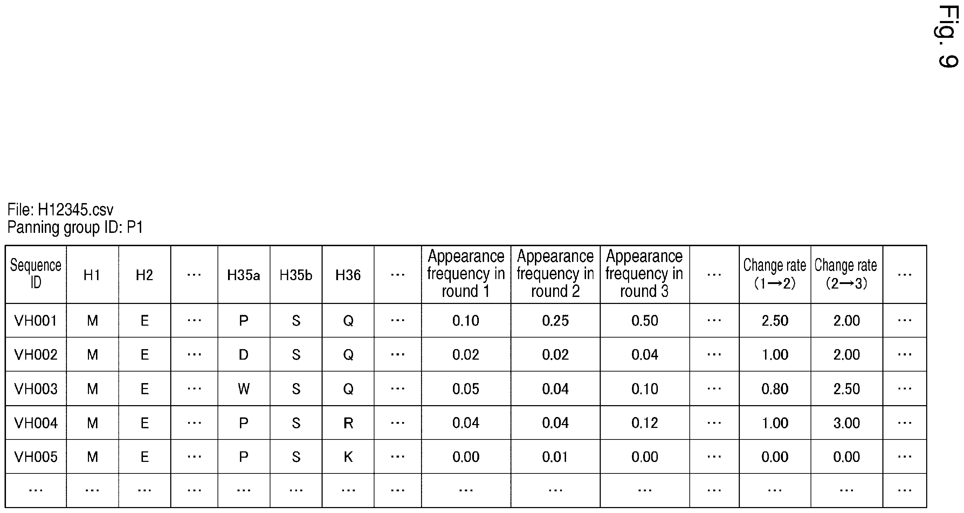

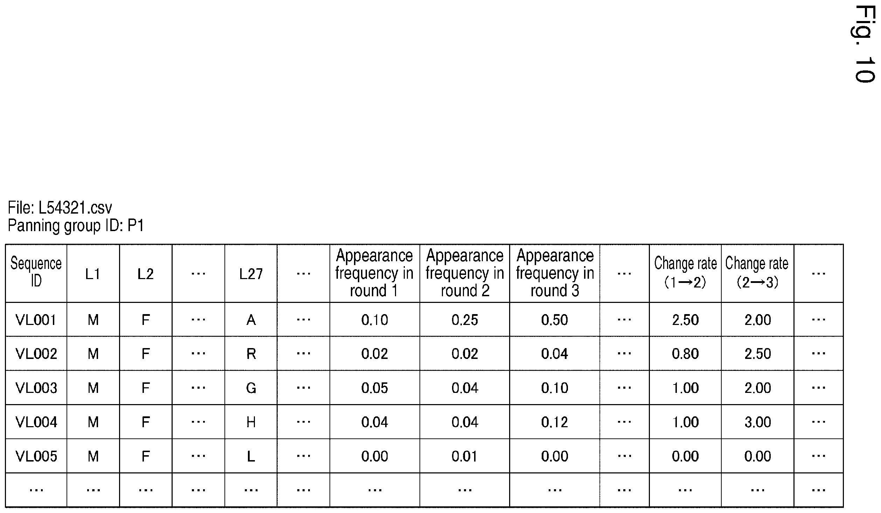

The dataset storage 322 stores, as a dataset, sequence information and evaluation result information (the appearance frequency in each round and round-to-round change rates of appearance frequency) for each antibody measured in a sequence of panning. Here, the dataset storage 322 stores a dataset on heavy chain sequences (see Figure 9) and a dataset on light chain sequences (see Figure 10) with distinguishing them from each other. These datasets are included in the analysis result information from the next-generation sequencer 20, and inputted by the processor 33.

It is not needed to input datasets both on heavy chain sequences and light chain sequences, and a dataset only on heavy chain sequences or only on light chain sequences may be inputted. In addition, it is also acceptable to input one, joined binding sequence that may be a dataset obtained by reading heavy chain sequences and light chain sequences at once.

The classification criteria storage 323 (an example of a criteria storage) stores classification criteria information. As described above, classification criteria information includes a plurality of pieces of classification criteria candidate information. The information is included in the input information from the user terminal 10, and set by the processor 33. However, the present invention is not limited to this, and the classification criteria information (a plurality of pieces of classification criteria candidate information) may be set in advance in the classification criteria storage 323.

The learning dataset storage 324 stores, as a learning dataset, binding determination information according to sequence information and evaluation result information (appearance frequency in each round and round-to-round change rates of appearance frequency) for each antibody containing a combination of a heavy chain sequence and a light chain sequence. However, the learning dataset storage 324 may store, as a learning dataset, binding determination information according to sequence information and evaluation result information separately for heavy chain sequences and light chain sequences.

The focused position information storage 325 stores focused position information. This information is included in the input information from the user terminal 10, and set by the processor 33.

The learning result storage 326 stores a model trained with sequence learning generated by a prediction target sequence generator PA and a model trained with learning of characteristic prediction generated by a learner 334.

The mutation information storage 327 stores mutation information. This information is included in the input information from the user terminal 10, and set by the processor 33.

The sequence storage 328 stores prediction target sequence information representing amino acid sequences of prediction target sequences. This prediction target sequence information is generated and set by the processor 33 with use of a model trained with sequence learning.

The characterization information storage 329 stores prediction scores given by the processor 33 with use of a model trained with learning of characteristic prediction with association with the respective prediction target sequences.

Now, an example of experiment information, experiment attribute information, datasets, learning datasets, and prediction target sequence information stored by the storage 32 will be described with reference to Figures 7 to 12.

Figure 7 shows a diagram illustrating an example of experiment information according to the present embodiment.

In the example illustrated in the diagram, the experiment information is a relational database in which items on target antigen information, an antibody library, datasets, an experiment conditions ID, a round 2 experiment conditions ID, and a round 3 experiment conditions ID are associated with each panning group ID to identify panning groups. Here, the item antibody library is one piece of experimental antibody information, and indicates an antibody library subjected to round 1. The item datasets indicate files of datasets based on analysis result information for the panning identified with a panning group ID. The item experiment conditions ID is identification information to identify experiment attribute information, and indicates experiment conditions in round 1. The items round 2 experiment conditions ID and round 3 experiment conditions ID respectively indicate an experiment conditions ID indicating experiment conditions in round 2 and an experiment conditions ID indicating experiment conditions in round 3.

The example illustrated in the diagram represents that, in the sequence of panning with "Panning group ID" being "P1", "Target antigen" is "antigen 1" and "antibody library" is "library 1". The example also represents that, in the sequence of panning of "P1", among the files of datasets, the file on heavy chain sequences is "H12345.csv" and the file on light chain sequences is "L54321.csv". In addition, the example represents that, in the sequence of panning of "P1", the experiment conditions in round 1 are "conditions 1 ", the experiment conditions in round 2 are "conditions 2", and the experiment conditions in round 3 are "conditions 3".

Figure 8 shows a diagram illustrating an example of experiment attribute information according to the present embodiment.

In the example illustrated in the diagram, the experiment attribute information is a database in which items on an antibody display technique, the origin of antibodies, the concentration of the target antigen, the composition of buffer, reaction time, and reaction temperature are associated with each experiment conditions ID. The database represents an example of relational databases, but the present invention is not limited to this, and the database may be a text file such as a CSV file or a NoSQL (the same is applied hereinafter).

The example illustrated in the diagram represents that the experiment conditions with "Experiment conditions ID" being "PI" are such that "Antibody display technique" is "phage", "Origin of antibodies" is "mouse", "Concentration of target antigen" is "1 (nM)", "Composition of buffer" is "composition A", "Reaction time" is "T0", and "Reaction temperature" is "t1".

As described above, the buffer composition may indicate hydrogen-ion exponent.

Figure 9 shows a diagram illustrating an example of a dataset according to the present embodiment.

In the dataset in the diagram, panning group ID is associated with "P1", and the file name is "H12345.csv". Specifically, it is represented that the dataset in the diagram is generated from analysis result information in the sequence of panning of "P1", and is a dataset for antibodies of heavy chain sequences.

In the example illustrated in the diagram, the dataset is a database in which items on sequence information on an antibody (HI, H2, ..., H35a, H35b, H36, ...), appearance frequency in round 1, appearance frequency in round 2, appearance frequency in round 3, a change rate (1 → 2), and a change rate (2 → 3) are associated with each sequence ID. Here, "Sequence ID" indicates an identifier to identify sequences of antibodies.

"H1", "H2", "H35a", "H35b", and "H36" each indicate the position of an amino acid in the variable region of an antibody heavy chain in accordance with Kabat numbering, and "H" indicates being a heavy chain.

The change rate (N → N+1) indicates the rate of change in appearance frequency between round N and round N+1, being a value obtained by dividing the appearance frequency in round N+1 by the appearance frequency in round N in the example in the diagram. Change rates may be calculated by the server 30.

The example illustrated in the diagram represents that the antibody identified by "Sequence ID" of "VH001" has an amino acid sequence in which the amino acid at position "H1" is "M (methionine)", the amino acid at position "H2" is "E (glutamic acid)", the amino acid at position "H35a" is "P (proline)", the amino acid at position "H35b" is "S (serine)", and the amino acid at position "H36" is "Q (glutamine)". The example also represents, as evaluation result information, that, for the antibody identified by "VH001", "Frequency of appearance in round 1" is "10", "Frequency of appearance in round 2" is "25", "Frequency of appearance in round 3" is "50", "Change rate (1 → 2)" is "2.50", and "Change rate (2 → 3)" is "2.00".

Figure 10 shows a diagram illustrating another example of a dataset according to the present embodiment.

In the dataset in the diagram, panning group ID is associated with "P1", and the file name is "L54321.csv". Specifically, it is represented that the dataset in the diagram is generated from analysis result information in the sequence of panning of "P1", and is a dataset for antibodies of light chain sequences.

In the example illustrated in the diagram, the dataset is a database in which items on sequence information on an antibody (L1, L2, ..., L27, ...), appearance frequency in round 1, appearance frequency in round 2, appearance frequency in round 3, a change rate (1 → 2), and a change rate (2 → 3) are associated with each sequence ID.

Each of "L1", "L2", and "L27" has been associated in advance with the position of an amino acid in antibodies. Each of these items indicates a position in the variable region of an antibody light chain, and a value thereof (an alphabet in the diagram) indicates an amino acid positioned at the position. The dataset in Figure 10 and that in Figure 9 are different in whether sequence information on antibodies represents positions in the variable region of antibody heavy chains or positions in the variable region of antibody light chains.

The example illustrated in the diagram represents that the antibody identified by "Sequence ID" of "VL001" has an amino acid sequence in which the amino acid at position "L1" is "M", the amino acid at position "L2" is "F (phenylalanine)", and the amino acid at position "L27" is "A (alanine)". The example also represents, as evaluation result information, that, for the antibody identified by "VL001", "Frequency of appearance in round 1" is "8", "Frequency of appearance in round 2" is "20", "Frequency of appearance in round 3" is "40", "Change rate (1 → 2)" is "2.50", and "Change rate (2 → 3)" is "2.00".

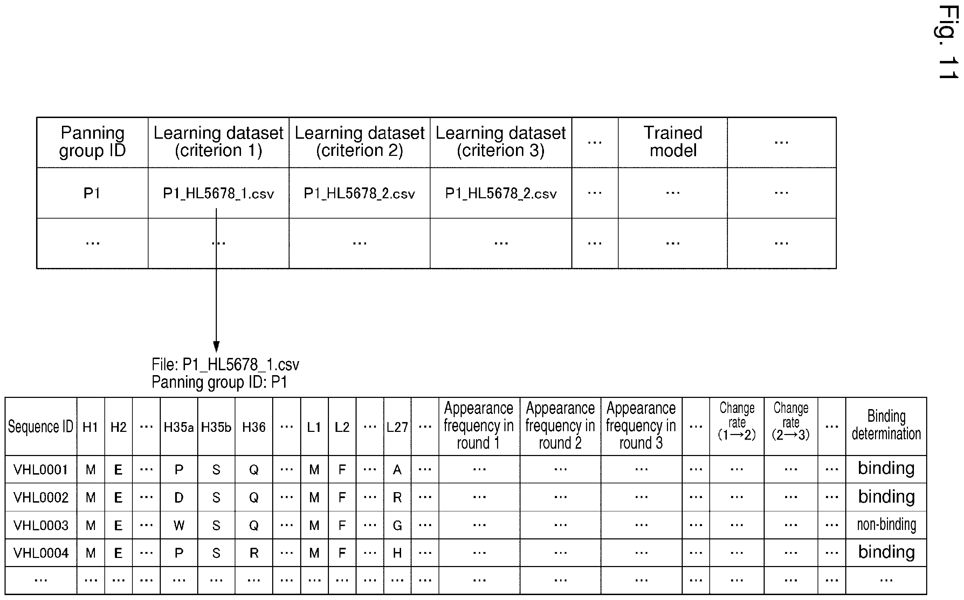

Figure 11 shows a diagram illustrating an example of learning datasets according to the present embodiment.

A learning dataset is stored for each panning group ID and classification criteria candidate information. The learning dataset in the diagram is a learning dataset collection with the panning group ID being "PI" and the classification criteria candidate information being "criterion 1".

In the example illustrated in the diagram, the dataset is a database in which items on sequence information on an antibody (HI, H2, ..., H35a, H35b, H36, ..., L1, L2, ..., L27, ...), appearance frequency in round 1, appearance frequency in round 2, appearance frequency in round 3, a change rate (1 → 2), a change rate (2 → 3), and binding determination information are associated with each sequence ID. The binding determination information represents whether an antibody is a binding antibody or a non-binding antibody under "criterion 1".

The example illustrated in the diagram represents that the antibody identified by "Sequence ID" of "VHL0001" has an amino acid sequence in which the amino acid at position "H1" is "M", the amino acid at position "H2" is "E", the amino acid at position "H35a" is "P", the amino acid at position "H35b" is "S", the amino acid at position "H36" is "Q", the amino acid at position "L1" is "M", the amino acid at position "L2" is "F", and the amino acid at position "L27" is "A", and "Binding determination" is "binding (binding antibody)".

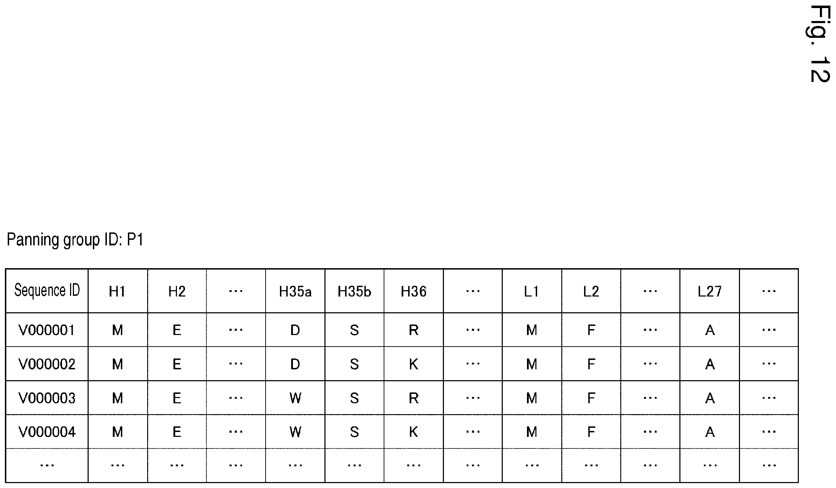

The server 30 uses the model trained with sequence learning, which has learned the character of binding sequences, to generate a group of virtual sequences having the character as in Figure 12.

Through the above manner, a group of binding sequences is defined. The model trained with sequence learning is a trained model that has learned which amino acid is likely to appear at a given position and which amino acid is likely to appear at a given position depending on what group of amino acids is present before the position. The server 30 generates many sequences expected to have a desired property on the basis of the model trained with sequence learning.

Figure 12 shows a diagram illustrating an example of prediction target sequence information according to the present embodiment.

The prediction target sequence information is information representing prediction target sequences.

In the example illustrated in the diagram, the prediction target sequence information is a database in which sequence information on an antibody (HI, H2, ..., H35a, H35b, H36, ..., L1, L2, ..., L27, ...) is associated with each sequence ID.

The example illustrated in the diagram represents that the antibody identified by "Sequence ID" of "V000001" is an antibody for which a prediction score is to be calculated, and has an amino acid sequence in which the amino acid at position "H1" is "M", the amino acid at position "H2" is "E", the amino acid at position "H35a" is "D (aspartic acid)", the amino acid at position "H35b" is "S", the amino acid at position "H36" is "R (arginine)", the amino acid at position "L1" is "M", the amino acid at position "L2" is "F", and the amino acid at position "L27" is "A".

In the example illustrated in the diagram, for example, the prediction target sequence information is one in the case in which H35a and H36 have been inputted as mutation information. That is, in sequence information, amino acids at positions designated by H35a and H36 are different but amino acids at the other positions are identical among a plurality of predicted antibody sequences.

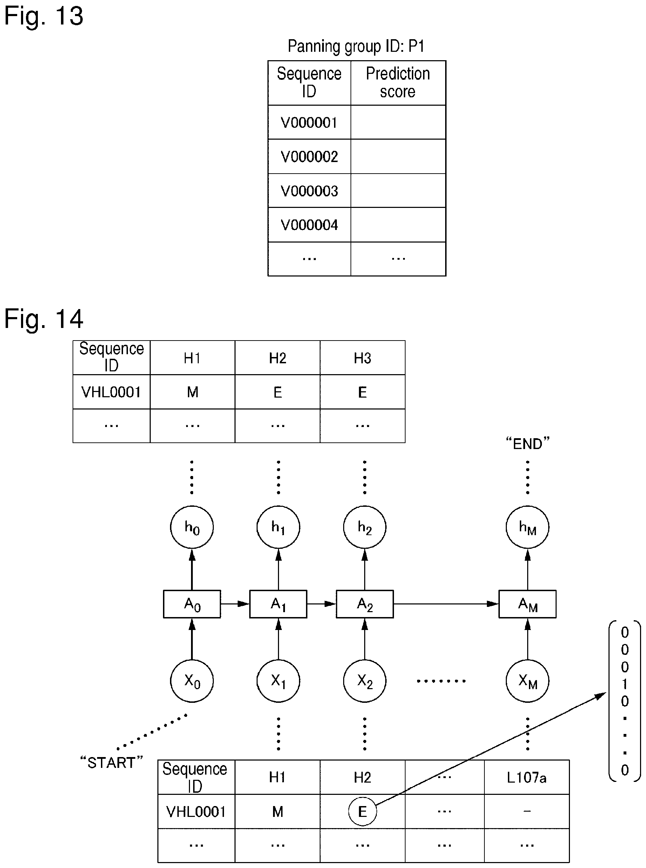

Figure 13 shows a diagram illustrating an example of characterization information according to the present embodiment.

The characterization information is information representing prediction results for prediction of characteristics of prediction target sequences with use of learning results and prediction target sequences.

In the example illustrated in the diagram, the characterization information is a database in which a prediction score is associated with each sequence ID. A prediction score is information representing the probability and strength to bind to the target antigen.

In the example illustrated in the diagram, "Prediction score" of the antibody identified by "Prediction target sequence ID" of "V000001" is blank because prediction has not been performed yet.

<Processor of Server>

Again, with reference to Figure 6, details of the processor 33 will be described.

The processor 33 includes an information acquirer 331, an estimator 332, a classifier 333, a prediction target sequence generator PA, a learner 334, a controller 335, and an output processor 336.

The information acquirer 331 acquires experiment information (see Figure 7) and experiment attribute information (see Figure 8) from analysis result information from the next-generation sequencer 20, and orders the experiment information storage 321 to store these sets of information. The information acquirer 331 acquires sequence information from the analysis result information from the next-generation sequencer 20. The information acquirer 331 calculates appearance frequency for each piece of sequence information in the analysis result information, and generates sequence information and appearance frequency as datasets. Here, the information acquirer 331 distinguishes datasets between heavy chain sequences and light chain sequences. Specifically, the information acquirer 331 assumes sequence information representing heavy chain sequences and evaluation result information thereon as a dataset for antibodies of heavy chain sequences (see Figure 9), and associates the file with experiment information (e.g., panning group ID) and orders the dataset storage 322 to store the resultant. The information acquirer 331 assumes sequence information representing light chain sequences and evaluation result information thereon as a dataset for antibodies of light chain sequences (see Figure 10), and associates the file with experiment information and orders the dataset storage 322 to store the resultant.

When the information acquirer 331 has acquired classification criteria information from the user terminal 10, the information acquirer 331 orders the classification criteria storage 323 to store the classification criteria information. When the information acquirer 331 has acquired focused position information from the user terminal 10, the information acquirer 331 orders the focused position information storage 325 to store the focused position information. When the information acquirer 331 has acquired mutation information from the user terminal 10, the information acquirer 331 orders the mutation information storage 327 to store the mutation information.

The estimator 332 estimates combinations of a heavy chain sequence and a light chain sequence on the basis of round number and appearance frequency in a sequence of panning. The estimator 332 makes an estimate that an antibody with an estimated combination of a heavy chain sequence and a light chain sequence exists.

Specifically, the estimator 332 calculates, for example, the correlation coefficient of appearance frequencies in multiple round numbers for each combination of a heavy chain sequence and a light chain sequence. For a combination with the highest correlation coefficient, the estimator 332 makes an estimate that an antibody with the combination of a heavy chain sequence and a light chain sequence was present. The estimator 332 excludes the heavy chain sequence and light chain sequence of the combination with the highest correlation coefficient, and calculates the correlation coefficient of appearance frequencies in multiple round numbers for each of the other combinations of a heavy chain sequence and a light chain sequence; thus, the above process by the estimator 332 is repeated.

In this way, the estimator 332 estimates combinations of a heavy chain sequence and a light chain sequence by correlation of appearance frequency in a plurality of rounds in a sequence of panning. Thereby, the information processing system 1 can estimate antibodies (combinations of a heavy chain sequence and a light chain sequence) with higher precision than in the case without performing panning.

The estimator 332 orders the dataset storage 322 to store combinations of a heavy chain sequence and a light chain sequence contained in antibodies estimated to be present (also referred to as an "existing antibody sequences").

In place of correlation functions of appearance frequencies in multiple round numbers, the estimator 332 may calculate, for example, the correlation coefficient of round-to-round change rates of appearance rates or differences of appearance rates. For a heavy chain sequence and a light chain sequence for which any one of the correlation coefficients of appearance frequencies in multiple round numbers, round-to-round change rates of appearance rates, and differences of appearance rates is identical (or generally identical), the estimator 332 may make an estimate that an antibody containing the heavy chain sequence and the light chain sequence was present.

However, the present invention is not limited to this, and the processor 33 may estimate combinations of a heavy chain sequence and a light chain sequence by using another technique.

The processor 33 may avoid estimation of combinations, and in this case is not required to include the estimator 332. For example, the processor 33 may perform analysis only of heavy chain sequences or analysis only of light chain sequences. In this case, the processor 33 may order the dataset storage 322 to store heavy chain sequences (or light chain sequences) as existing antibody sequences.

When sequence information on whole antibodies is included in the analysis result information, the processor 33 orders the dataset storage 322 to store the sequence information on whole antibodies included in the analysis result information as existing antibody sequences. When the next-generation sequencer 20 can read a heavy chain sequence and a light chain sequence at once, for example, the information acquirer 331 acquires, as analysis result information, sequence information on sequences of combinations of a heavy chain sequence and a light chain sequence, and orders the dataset storage 322 to store the sequence information.

The classifier 333 reads out a plurality of pieces of classification criteria candidate information from the classification criteria information in the classification criteria storage 323. The classifier 333 classifies antibodies represented by existing antibody sequences into binding antibodies and non-binding antibodies according to the classification criteria represented by the pieces of classification criteria candidate information.

Specifically, the classifier 333 determines whether the appearance frequency in each round (or the round-to-round change rate of appearance frequency) of an existing antibody sequence is equal to or higher than the threshold for appearance frequency (or change rates) in the classification criteria information. When the classifier 333 determined that the appearance frequency (or the change rate) of an existing antibody sequence is equal to or higher than the threshold, the classifier 333 determines that the antibody represented by the existing antibody sequence is a binding antibody. Otherwise (when the appearance frequency (or the change rate) of an existing antibody sequence is lower than the threshold), on the other hand, the classifier 333 determines that the antibody represented by the existing antibody sequence is a non-binding antibody.

Here, when a threshold has been set for each of a plurality of items in given classification criteria information (see Figure 4), the classifier 333 determines that an antibody represented by an existing antibody sequence is a binding antibody if each of the items is equal to or higher than the corresponding threshold. In the example in Figure 4, the classifier 333 determines that an antibody represented by an existing antibody sequence is a binding antibody if, in criteria 1, the appearance frequency in round 1 is equal to or higher than X1, the change rate of appearance frequency between round 1 and round 2 is equal to or higher than Y1, and the change rate of appearance frequency between round 1 and round 3 is equal to or higher than Z 1. Otherwise, the classifier 333 determines that an antibody represented by an existing antibody sequence is a non-binding antibody.

While the appearance frequency (or the change rate) of an existing antibody sequence in each round is either one of the appearance frequency of a heavy chain sequence and that of a light chain sequence (e.g., the appearance frequency of a heavy chain sequence, the appearance frequency of a light chain sequence, the minimum value of appearance frequency, or the maximum value of appearance frequency), the appearance frequency (or the change rate) of an existing antibody sequence in each round may be the average value or the like for a heavy chain sequence and a light chain sequence. The classifier 333 may add classification criteria candidate information. For example, the classifier 333 adds, as thresholds, new classification criteria candidate information as values generated by varying thresholds included in other classification criteria candidate information by a predetermined value.

The classifier 333 orders the learning dataset storage 324 to store, as a learning dataset, existing antibody sequences determined as those of binding antibodies, evaluation result information thereon (appearance frequency and change rates thereof), and binding determination information representing classification results for each piece of classification criteria candidate information. The binding determination information represents whether a binding antibody or a non-binding antibody. That is, the binding determination information is information representing whether an antibody binds to the target antigen or not (being non-binding).

The binding determination information may be values obtained by subtracting a threshold for appearance frequency (or change rates) in classification criteria information from the appearance frequency (or the round-to-round change rate of appearance frequency) of an existing antibody sequence in each round, or values based on variance or standard deviation.

<Generation Process for Prediction Target Sequences>

In the following, a generation process for prediction target sequences that is performed by the prediction target sequence generator PA will be described.

The prediction target sequence generator PA includes a sequence selector PA1, a sequence learner PA2, and a virtual sequence generator PA3.

The sequence selector PA1 reads out a learning dataset for each piece of classification criteria candidate information from the learning dataset storage 324. When focus information is stored in the focused position information storage 325, the sequence selector PA1 reads out the focus information. From the sequence information represented by the learning datasets, the sequence selector PA1 extracts sequence information on positions represented by the focus information. The sequence learner PA2 uses learning datasets including the sequence information extracted by the sequence selector PA1 (hereinafter, also referred to as "subject sequence information") and the binding determination information for a learning process. When no focus information is stored in the focused position information storage 325, the sequence learner PA2 regards the sequence information for all positions as subject sequence information, and uses learning datasets including the subject sequence information and the binding determination information for a learning process.

The sequence selector PA1 determines highly precise classification criteria on the basis of results of a learning process for each piece of classification criteria candidate information. The sequence selector PA1 selects learning datasets with highly precise classification criteria.

Now, details of a learning process using an LSTM (Long Short-Term Memory), as a learning model used by the sequence selector PA1, will be described. However, the present invention is not limited to this, and another learning model may be used for the learning process.

<Learning Process>

Figure 14 shows an illustration for describing an example of the learning process performed by the sequence learner PA2 according to the present embodiment.

An LSTM to be used for the learning process is composed of three layers: an input layer, an intermediate layer, and an output layer. In the example illustrated in Figure 14, the input layer is represented with $X_1, X_2, ...X_M$, the intermediate

layer with $A_1$, $A_2$, ...$A_M$, and the output layer with $h_1$, $h_2$, ...$h_M$. Each input in the input layer is an amino acid at a position represented by the focused position information among the amino acids at all positions in the learning datasets. The focused position information is, for example, one on a site of antibodies as consecutive positions in a sequence. However, the present invention is not limited to this, and the focused position information may include non-consecutive positions. When no focused position <Information is present, the inputs in the input layer are the amino acids at all positions in the learning datasets.

Into the intermediate layer $A_t$ at the tth (t = 0, 1, 2, ..., M), input information from the input layer $X_t$ and output information from the intermediate layer $A_{t-1}$ at the (t-1)th are inputted.

For the intermediate layer $A_t$ for each t, a plurality of parameters is stored. The parameters are, for example, parameters associated with processes of an input gate, an input determination gate, an oblivion gate, and an output gate present in the intermediate layer. The parameters are stored in advance in the storage 32.

When input information from the input layer $X_t$ and output information from the intermediate layer $A_{t-1}$ are inputted, the intermediate layer $A_t$ calculates a value of the output layer $h_t$ on the basis of the parameters stored in the storage 32, and outputs the value.

In the case of the learning process by the LSTM, amino acid information on the tth sequence in the sequence information is inputted into the input layer $X_t$. The amino acid information is in the form of a vector having 20 components, and each component corresponds to one of 20 types of amino acid. For example, the fourth component of the vector corresponds to the amino acid type "E". The situation that the amino acid at position H2 is "E" as in the illustration corresponds to such a vector that only the fourth component is "1" and the other components are "0", in the sequence information. Into the input layer Xo, a command to order the intermediate layer $A_0$ to output the value of the output layer ho ("START" in the illustration) is inputted. In this case, this command is also information indicating the start of output of an amino acid sequence.

The vector value $h_t$ of the output layer $h_t$ outputted from the LSTM is compared with (t+1)th amino acid information in the sequence information. As a result of comparison, the parameters in the intermediate layer $A_t$ are updated so as to reduce the difference. The value of the output layer $h_M$ is regarded as information indicating the end of the amino acid sequence (represented as "END" in Figure 14).

The example illustrated in Figure 14 shows data to be inputted when the LSTM is allowed to learn the sequence identified by the sequence ID "VHL0001" in Figure 11. The amino acid "M" at position H1 is inputted into the input layer "$X_1$". The amino acid "E" at position H2 is inputted into the input layer "$X_2$". Into the input layer "$X_M$", information indicating that there is no amino acid at position L107a ("-") is inputted. In learning, in turn, the value outputted from the output layer "ho" is compared with the amino acid "M" at position H1, and the value outputted from the output layer "$h_1$" is compared with the amino acid "E" at position H2.

Through the above learning process, amino acid information at the (t+1)th in the sequence is outputted from the intermediate layer $A_t$ to the output layer $h_t$ in the LSTM after learning.

An LSTM is prepared for each criterion, and the learning process is performed for the LSTM for each criterion with use of learning datasets under the criterion.

<Execution Process: Generation of Virtual Sequences>

An execution process to output virtual sequences with use of an LSTM after the learning process will be described.

When this execution process is performed, the vector value $h_{t-1}$ of the output layer $h_{t-1}$ in the LSTM is inputted into the input layer $X_t$. The LSTM can sequentially output the next amino acid in the sequence from the output layer $h_t$. Here, when the vector value $h_{t-1}$ of the output layer $h_{t-1}$ is inputted into the input layer $X_t$, for the values of all components of the vector, one amino acid is selected according to the probabilities of appearance of 20 types of amino acid in $h_{t-1}$, and such a vector that the vector component corresponding to the amino acid is "1" and the other components are "0" is inputted. In generating a group of numerous virtual sequences, for example, "selecting an amino acid at each position according to the probabilities set in the model to generate one sequence" is repeated in very many cycles (millions to tens of millions of cycles). Thus, a vector for which one corresponding amino acid has been determined to the vector value $h_{t-1}$ of the output layer $h_{t-1}$ is also referred to as the fixed vector value $h_{t-1}$, hereinafter.

The amino acid represented by the fixed vector value $h_{t-1}$ serves as tth amino acid information (constituent element) in the sequence information. In short, a sequence in which amino acids represented by the fixed vector value $h_{t-1}$ are aligned in order from t = 1 to t is outputted as a virtual sequence. Here, the virtual sequence is a sequence having the character of the sequence information in the learning datasets. In this way, a trained model that learns the character of sequence information in learning datasets and outputs a virtual sequence is called a model trained with sequence learning.

<Execution Process: Giving Prediction Score>

An execution process to output prediction scores with use of an LSTM after the learning process will be described.

The LSTM is trained only with sequence information with binding determination being "binding" in the learning datasets. When the execution process is performed, the vector value $X_t$ is inputted, as tth amino acid information in the inputted sequence information, into the input layer $X_t$ ($t \geq 1$). Into the input layer Xo, information indicating the start of output of an amino acid sequence ("START") is inputted. From the output layer $h_t$, the vector value $h_t$ is outputted.

When tth amino acid information is inputted into the input layer $X_t$, the vector value $h_t$ represents a predicted value for (t+1)th amino acid information. Because the LSTM has learned only with sequence information with binding determination being "binding", this predicted value is a predicted value with high possibility to be given binding determination of "binding". Accordingly, the inner product of the vector value $h_{t-1}$ and the vector value xt is information to calculate the likelihood P to give binding determination of "binding" for the whole sequence in tth amino acid information. To the inputted sequence information, the value obtained by multiplying the probability Pt from t = 1 to t = M, that is, the likelihood $P = P_1 \times P_2 \times P_3 \times ... \times P_M$ serves as a prediction score indicating affinity with the target antigen.

In this way, a trained model that outputs a prediction score for sequence information inputted is called a model trained with learning of characteristic prediction.

In the present embodiment, the information processing system 1 uses the same LSTM for the model trained with sequence learning and the model trained with learning of characteristic prediction, and hence can achieve reduced loads for the learning process as compared with the case in which a learning process is performed individually for each model. However, the present invention is not limited to this, and the model trained with sequence learning and model trained with learning of characteristic prediction may use different learning datasets for learning, and different learning models may be used. The model trained with learning of characteristic prediction may be a Gaussian process, and prediction scores obtained by the Gaussian process may be reliabilities for prediction.

<Intermediate Layer of LSTM>

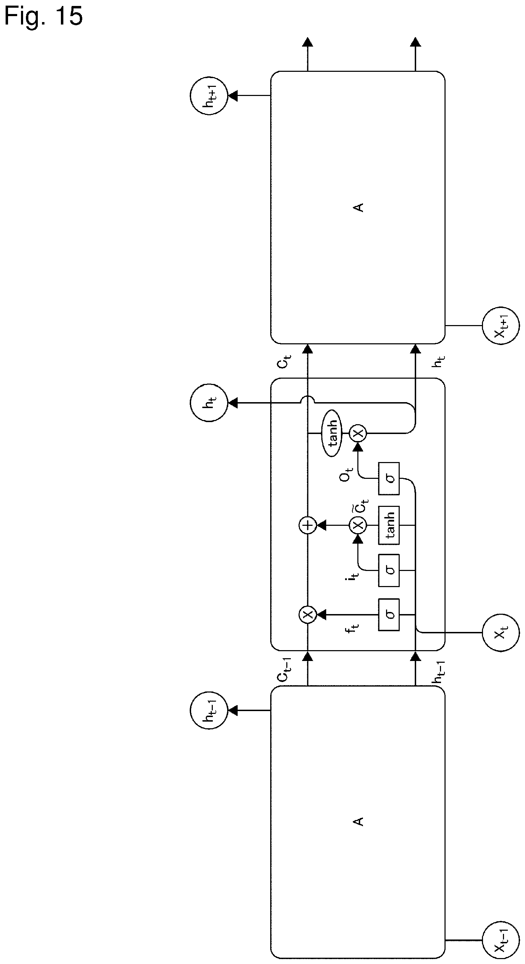

Figure 15 shows a conceptual diagram illustrating the structure of an LSTM according to the above embodiment.

This diagram is a part of the LSTM in Figure 14, and illustrates an example of the internal structure of the tth intermediate layer $A_t$. In the diagram, the (t-1)th cell state $C_{t-1}$, as input information from the intermediate layer $A_{t-1}$, and the vector value $h_{t-1}$ outputted from the output layer $h_{t-1}$ are inputted into the intermediate layer $A_t$. Into the intermediate layer $A_t$, the vector value $x_t$ is inputted from the input layer $X_t$.

$W_f$, $b_f$, $W_i$, $b_i$, $W_c$, $b_c$, $W_o$, and $b_o$ are stored as parameters for the LSTM in the storage 32. In the intermediate layer $A_t$, $f_t$ in expression (1), $i_t$ in expression (2), C~(tilde)$_t$ in expression (3), and ot in expression (5) are calculated for the inputted $C_{t-1}$, value $h_{t-1}$, and value xt by using the parameters stored in the storage 32: $W_f$, $b_f$, $W_i$, $b_i$, $W_c$, $b_c$, $W_o$, and $b_o$. Ct in expression (4) is calculated by using calculated $f_t$, $i_t$, and C~(tilde)$_t$, and the vector value $h_t$ is calculated by using Ct and ot. This vector value $h_t$ is outputted from the output layer $h_t$.

[Expression 1]

$$f_t = \sigma\left(W_f \cdot [h_{t-1}, x_t] + b_f\right) \quad \text{...} \quad \text{expression (1)}$$

$$i_t = \sigma\left(W_i \cdot [h_{t-1}, x_t] + b_i\right) \quad \text{...} \quad \text{expression (2)}$$

$$\tilde{C}_t = \tanh(W_C \cdot [h_{t-1}, x_t] + b_C) \quad \text{...} \quad \text{expression (3)}$$

$$C_t = f_t * C_{t-1} + i_t * \tilde{C}_t \quad \text{...} \quad \text{expression (4)}$$

$$o_t = \sigma\left(W_o [h_{t-1}, x_t] + b_o\right) \quad \text{...} \quad \text{expression (5)}$$

$$h_t = o_t * \tanh\left(C_t\right) \quad \cdots \quad \text{expression (6)}$$

Here, $\sigma$ denotes a sigmoid function.

In the learning process, the vector value $h_{t-1}$ outputted from the (t-1)th output layer $h_{t-1}$ and the tth vector value $x_t$ in the sequence information are compared. As a result of the comparison, the parameters $W_f$, $b_f$, $W_i$, $b_i$, $W_c$, $b_c$, $W_o$, and $b_o$ are updated to new values so as to reduce the error between the vector value $h_{t-1}$ and the vector value $x_t$.

The parameters after updating are stored in the storage 32. The parameters $W_f$ and $b_f$ are parameters associated with processing in the input gate. The parameters $W_i$ and $b_i$ are parameters associated with processing in the input determination gate. The parameters $W_c$ and $b_c$ are parameters associated with processing in the oblivion gate. The parameters $W_o$ and $b_o$ are parameters associated with processing in the output gate.

<Selection of Trained Model>

Again, with reference to Figure 6, in performing the learning process, the sequence selector PA1 first reads out learning datasets with binding determination being "binding" from the learning datasets. The sequence selector PA1 divides the learning datasets read out into learning datasets for the learning process and evaluation datasets for an evaluation process to evaluate the model trained with learning of characteristic prediction. While there exist characteristic prediction learning models in the number corresponding to the number of pieces of classification criteria candidate information in the present embodiment, a learning process is performed for each characteristic prediction learning model with use of the above-described learning datasets, and an evaluation process is performed with use of the evaluation datasets.

Subsequently, the sequence selector PA1 divides the learning datasets for the learning process into training datasets and validation datasets. For example, the sequence selector PA1 separates the learning datasets into a plurality of groups (G1, G2...GN). The groups are set to have almost the same number of learning datasets. Of the plurality of groups, the sequence selector PA1 selects one group (e.g., Gk) as a group for use in validation (validation group). That is, the sequence selector PA1 uses datasets for learning included in the validation group as validation datasets. The sequence selector PA1 sets the learning datasets included in the rest of the groups as training datasets.

The sequence learner PA2 generates a model trained with learning of characteristic prediction by performing a learning process for the LSTM with use of the training datasets. After the learning process, the sequence learner PA2 validates the LSTM. The sequence learner PA2 inputs the sequence information in the validation datasets into the model trained with learning of characteristic prediction. The sequence learner PA2 acquires prediction scores outputted from the model trained with learning of characteristic prediction as characteristic estimation information. The sequence learner PA2 compares the characteristic estimation information with the characteristic information corresponding to the sequence information inputted, and determines the difference between the characteristic estimation information and the characteristic information by using a predetermined method. The predetermined method is, for example, a method of calculating the average absolute error for all the date in the evaluation datasets.

Determination of the difference between the characteristic information and the characteristic estimation information is not limited to the above-described method. For example, a method of determining mean squared errors, root mean squared errors, coefficients of determination, or the like may be used.

The sequence learner PA2 changes the validation group and repeats the above-described training and validation. The number of repetitions agrees with the number of the divided groups. A group that has once been classified as a validation group is not classified again as a validation group. That is, if learning datasets are divided into N groups of G1 to GN, training and validation are performed N times. Each group is included once in a validation group and used for the above-described validation process.

After the completion of N cycles of training and validation, the sequence learner PA2 uses the N differences obtained to evaluate training for the LSTM with use of all the learning datasets. Specifically, the sequence learner PA2 calculates the average of the N differences. If the average determined is not equal to or lower than a predetermined threshold, the sequence learner PA2 performs the above learning over again. At this time, the sequence learner PA2 changes parameters associated with the entire of the intermediate layer. If the average determined is equal to or lower than a predetermined threshold, the sequence learner PA2 determines that the learning has completed and ends training and validation.

The sequence learner PA2 may perform training and validation with a method differing from the method of replacing a validation group as described above. For example, the sequence learner PA2 does not need to replace a validation group. The sequence learner PA2 may be set so that one learning dataset is included in each group. In this case, the above-described number of groups, N, agrees with the number of learning datasets.

In the present embodiment, a model that outputs characteristic estimation information for inputted data is called a model trained with learning of characteristic prediction.

The sequence selector PA1 calculates an AUC (Area Under an ROC Curve) from binding determination information and characteristic estimation information for each dataset of the evaluation datasets. The sequence selector PA1 performs a learning process and an evaluation process for every piece of classification criteria candidate information, thereby calculating an AUC for a model trained with learning of characteristic prediction for every piece of classification criteria candidate information. The sequence selector PA1 orders the learning result storage 326 to store a piece of classification criteria candidate information with the highest AUC (also referred to as the "selected classification criteria information") and the model trained with learning of characteristic prediction for the piece of classification criteria candidate information with associating with the panning group ID.

The sequence learner PA2 orders the learning result storage 326 to store at least the LSTM part (Figure 14) in the selected trained model generated by the sequence selector PA1 as a model trained with sequence learning.

The virtual sequence generator PA3 generates a plurality of virtual sequences with use of the model trained with sequence learning, and regards a group of a plurality of virtual sequences generated as prediction target sequence information. The sequence information on virtual sequences is sequence information obtained by changing amino acids at one or more positions in the sequence information in learning datasets associated with selected classification criteria information with the character of the sequence information retained.

Specifically, the LSTM in Figure 14 has learned, as a result of learning, conditional probabilities on which amino acid appears at a given position with what probability as a model trained with sequence learning. The virtual sequence generator PA3 generates sequences multiple times according to the probabilities. Specifically, in generating an amino acid sequence, the virtual sequence generator PA3 generates an amino acid of a new virtual sequence at position 1 on the basis of the learned probabilities of appearance of 20 amino acids. At position 2, the virtual sequence generator PA3 generates an amino acid of a new virtual sequence on the basis of the learned conditional probabilities of appearance of amino acids (AA), $P(AA_2|AA_1)$, with dependence on the amino acid at position 1. At position 3, the virtual sequence generator PA3 generates an amino acid of a new virtual sequence with dependence on the amino acids at positions 1 and 2. Subsequently, an amino acid at the next position is sequentially generated on the basis of an expression below determined through learning, and thus a new full-length virtual sequence is generated. This generation of a new virtual sequence is executed for a huge number of cycles. For example, the conditional probability in the following expression is denoted by the probability $P_{T+1}$.

[Expression 2]

$$P(AA_{T+1}|AA_1 \dots AA_T)$$

Here, if mutation information is stored in the mutation information storage 327, the sequence generator PA3 generates prediction target sequence information by inputting into the model trained with sequence learning in such a manner that amino acids other than those at mutation positions (elements of sequence information) represented by the mutation information are fixed and amino acids at mutation positions are set to those outputted from the model trained with sequence learning. Specifically, if the tth position is a mutation position, the sequence generator PA3 generates prediction target sequence information by replacing the tth amino acid with an amino acid represented by the fixed vector value $h_{t-1}$.

Thereby, the information processing system 1 can generate prediction target sequence information in which only amino acids that are likely to bind and present at positions intended to mutate have been changed.

The sequence generator PA3 orders the sequence storage 328 to store the prediction target sequence information generated (see Figure 12).

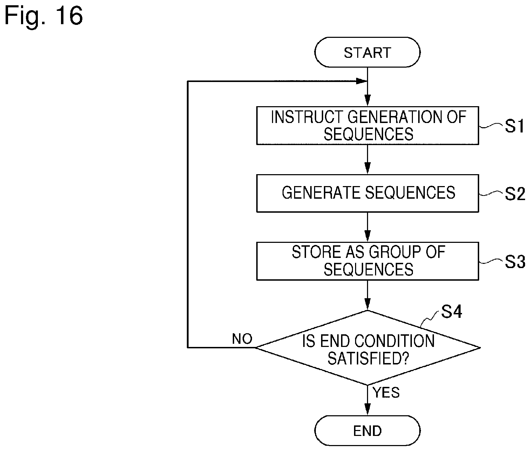

Figure 16 shows a flowchart illustrating an example of operations of the virtual sequence generator PA3 according to the present embodiment.

(Step S1) The virtual sequence generator PA3 inputs information indicating the beginning of an amino acid sequence into the model trained with sequence learning to instruct the model trained with sequence learning to generate a prediction target sequence.

(Step S2) The virtual sequence generator PA3 outputs the vector value ho, as amino acid information, from the output layer ho of the model trained with sequence learning, and inputs the fixed vector value ho, as the vector value $x_1$, into the input layer $X_1$. The virtual sequence generator PA3 outputs the vector value $h_{t-1}$, as amino acid information, from the output layer $h_{t-1}$ of the model trained with sequence learning, and inputs the fixed vector value $h_{t-1}$, as the vector value $x_t$, into the input layer $X_t$; these operations are repeated in the ascending order of t. If information indicating the end of an amino acid sequence is outputted from the output layer $h_M$, the virtual sequence generator PA3 terminates the process. The virtual sequence generator PA3 aligns amino acids represented by the fixed vector values ho to $h_{M-1}$ in order and the sequence after alignment is generated as a prediction target sequence.

(Step S3) The virtual sequence generator PA3 orders the sequence storage 328 to store the information representing the prediction target sequence, which has been generated in step S1, as prediction target sequence information.

(Step S4) The virtual sequence generator PA3 determines whether an end condition is satisfied. The end condition is a preset condition for terminating the process to generate a group of prediction target sequences. The end condition is, for example, such that the number of prediction target sequences generated in step S2 reaches a predetermined number or more. However, another condition may be used as the end condition, and, for example, such that a predetermined number of prediction target sequences of the same sequence have been generated, or a predetermined number of similar prediction target sequences have been generated. Similar prediction target sequences are, for example, sequences for which prediction scores are equal to or higher than a threshold. Alternatively, similar prediction target sequences are prediction target sequences that are located at distances shorter than a predetermined value when being mapped on a vector space by using, for example, the above-described Doc2Vec method.

If the end condition is not satisfied (No), the virtual sequence generator PA3 returns to step S1 and again generates prediction target sequences. If the end condition is satisfied (Yes), on the other hand, the virtual sequence generator PA3 terminates the process in Figure 16.

Through the process in Figure 16, the virtual sequence generator PA3 generates a plurality of prediction target sequences.

Again, with reference to Figure 6, the learner 334 copies the selected trained model generated by the sequence selector PA1, and orders the learning result storage 326 to store the copy as a model trained with learning of characteristic prediction.

The learner 334 may generate a model trained with learning of characteristic prediction by performing a learning process for the LSTM in Figure 14 with use of learning datasets associated with selected classification criteria information (Figure 11). For example, the learner 334 may perform a learning process through supervised learning such as deep learning with use of sequence information and binding determination information. In this case, the learner 334 may perform a learning process with use of learning datasets with binding determination being not "binding", in addition to learning datasets with binding determination being "binding" or in place of some of them.

The controller 335 outputs a prediction score (likelihood) by using the model trained with learning of characteristic prediction for sequence information inputted. That is, the controller 335 gives a prediction score to an antibody on which sequence information has been inputted against the target antigen subjected to panning under the panning group ID corresponding to the model trained with learning of characteristic prediction.

For example, the controller 335 reads out prediction target sequence information from the sequence storage 328. The controller 335 inputs the prediction target sequence information read out, as input data, into the model trained with learning of characteristic prediction and outputs a prediction score. The controller 335 orders the characterization information storage 329 to store the prediction target sequence information and the prediction score given as prediction target antibody information. For the prediction target antibody information in Figure 12, for example, the controller 335 orders to store a prediction score corresponding to the prediction target sequence information.

According to the prediction score in the prediction target antibody information, the output processor 336 outputs the prediction target sequence information of the prediction target antibody information as candidate antibody information. The candidate antibody information represents candidate antibodies having high affinity with the target antigen.

Specifically, the output processor 336 reads out sets of prediction target antibody information from the characterization information storage 329 and sorts them in the descending order of prediction scores. The output processor 336 generates the sets of prediction target sequence information ordered in the descending order of prediction scores as candidate antibody information. The output processor 336 sends the candidate antibody information generated to the user terminal 10 via the communicator 31 through the network NW The output processor 336 may send sets of prediction target antibody information to the user terminal 10, and the user terminal 10 (processor 14) may sort the sets of prediction target antibody information received in the descending order of prediction scores and display on the display 15.

When a target antigen or experiment information (see Figure 4) has been specified in the user terminal 10, the output processor 336 selects a panning group ID associated with the specified target antigen or experiment conditions from target antigens in the experiment information (Figure 7). If experiment conditions have been specified in the user terminal 10, the output processor 336 selects experiment conditions ID that satisfies the specified experiment conditions (Figure 8), and selects a panning group ID associated with the selected experiment conditions ID in the experiment information (Figure 7).

The output processor 336 extracts sets of prediction target antibody information (Figure 12) corresponding to the selected panning group ID. The output processor 336 sorts the extracted sets of prediction target antibody information in the descending order of prediction scores, and sends them to the user terminal 10.

<Operations>

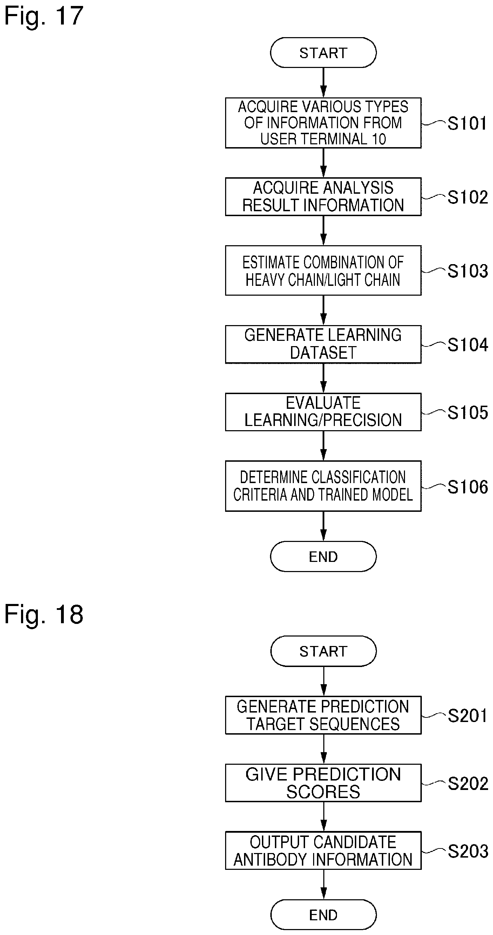

Figure 17 shows a flowchart illustrating an example of operations of the server 30 according to the present embodiment. This diagram illustrates operations of the server 30 in the stage of learning (learning process and evaluation process).

(Step S101) The information acquirer 331 acquires various types of information from the user terminal 10. The information acquirer 331 orders the storage 32 to store the acquired information. Thereafter, step S102 follows.

(Step S102) The information acquirer 331 acquires analysis result information from the next-generation sequencer 20. The information acquirer 331 orders the dataset storage 322 to store, as datasets, the analysis result information acquired in step S101. Thereafter, step S103 follows.

(Step S103) The estimator 332 estimates existing antibody sequences as a combinations of a heavy chain sequence and a light chain sequence on the basis of the datasets stored in step S102. The estimator 332 orders the dataset storage 322 to store the estimated existing antibody sequences. Thereafter, step S104 follows.

(Step S104) The classifier 333 classifies antibodies represented by the existing antibody sequences stored in step S103 into binding antibodies and non-binding antibodies according to classification criteria represented by each piece of classification criteria candidate information. For each piece of classification criteria candidate information, the classifier 333 generates a learning dataset including existing antibody sequences and binding determination information representing classification results, and orders the learning dataset storage 324 to store the learning datasets generated. Thereafter, step S105 follows.

(Step S105) The prediction target sequence generator PA performs a learning process for each piece of classification criteria candidate information on the basis of the learning datasets stored in step S104, thereby generating models trained with learning of characteristic prediction. The prediction target sequence generator PA performs an evaluation process to evaluate the precision of the generated models trained with learning of characteristic prediction. Thereafter, step S106 follows.

(Step S106) The prediction target sequence generator PA selects a model trained with learning of characteristic prediction from the models trained with learning of characteristic prediction that have been generated in step S105 on the basis of evaluation results in the evaluation process in step S105. The prediction target sequence generator PA orders the learning result storage 326 to store the selected trained model picked up and a model trained with sequence learning that has the same LSTM. Thereafter, the operations in the diagram are terminated.

Figure 18 shows a flowchart illustrating another example of operations of the server 30 according to the present embodiment. This diagram illustrates operations of the server 30 in the stage of execution. The stage of execution is a stage in which the information processing system 1 after learning with learning datasets performs prediction and so on with use of the selected trained model.

(Step S201) The prediction target sequence generator PA reads out the model trained with sequence learning that has been stored in step S106 of Figure 17, and generates prediction target sequence information with use of the model trained with learning of characteristic prediction. Thereafter, step S205 follows.

(Step S202) The controller 335 gives prediction scores for prediction target sequences generated in step S201 with use of the model trained with learning of characteristic prediction that has been stored in step S106 of Figure 17. Thereafter, step S203 follows.

(Step S203) According to the prediction scores given in step S202, the output processor 336 outputs the prediction target sequence information of prediction target antibody information as candidate antibody information. The candidate antibody information outputted is displayed on the user terminal 10. Thereafter, the operations in the diagram are terminated.

<Summary>

As described above, in the information processing system 1, the sequence learner PA2 (an example of the "sequence learner") performs a learning process using an LSTM (an example of the "machine learning") on the basis of a plurality of sequences and thereby generates a model trained with sequence learning that has learned the character of sequences represented by sequence information (an example of the "first trained model"). Here, the plurality of sequences to be used for the learning process is sequences of antibodies with the binding determination result being "binding". Accordingly, the model trained with sequence learning has learned the character of sequences that are likely to be determined to bind.

The virtual sequence generator PA3 (an example of the "sequence generator") generates prediction target sequence information (an example of the "virtual sequence information") representing prediction target sequences obtained by mutating at least one of the amino acids (an example of the "constituent elements") constituting a sequence represented by sequence information on antigen-binding molecules.

Thus, the information processing system 1 performs a learning process on the basis of sequences with the binding determination result being "binding", and hence can predict a sequence or amino acids that are likely to be determined to bind from a model trained with sequence learning.

The plurality of sequences to be used for the learning process is sequences of binding antibodies determined to have bound to the target antigen or sequences of antibodies used for the learning process of a selected trained model with the highest AUC value calculated by the sequence selector PA1. However, the present invention is not limited to this, and the sequences may be sequences having a predetermined characteristic (e.g., sequences with a characteristic value of equal to or higher than a threshold or equal to or lower than a threshold) according to characteristics and classification criteria. The binding antibodies determined to have bound to the target antigen may be binding antigen determined to have bound to the target antigen as a result of one round of panning, or binding antibodies subjected to the second or later round of panning, or binding antigen determined to have bound to the target antigen as a result of the second or later round of panning.

The character of sequences to be learned by the model trained with sequence learning is a character including positions of amino acids (an example of the "constituent elements") in the sequences and anteroposterior relationship of amino acids.

Thereby, the information processing system 1 can learn the positional character and character of anteroposterior relationship for amino acid sequences of antigen-binding molecules to be used for learning. In this case, the information processing system 1 can generate prediction target sequence information representing sequences having the same positional character and character of anteroposterior relationship.

The virtual sequence generator PA3 generates prediction target sequence information by changing at least one amino acid in a site set in a sequence and composed of one or more amino acids.

Thereby, the information processing system 1 can generate prediction target sequence information such that the site set has been altered. For example, a user can set a site intended to alter and thereby know prediction target sequence information with the site altered.

The site set in a sequence is included in the sequence of a heavy chain variable region, a light chain variable region, or a constant region of an antibody.

Thereby, the information processing system 1 can alter a site included in a heavy chain variable region, a light chain variable region, or a constant region of an antibody and generate prediction target sequence information with such alteration. For example, a user can set a site included in the sequence of a heavy chain variable region, a light chain variable region, or a constant region of an antibody and thereby know prediction target sequence information with the site altered.

The sequence information to be used for learning is sequence information selected according to results of characterization of antigen-binding molecules or proteins having the sequences represented by the sequence information.

Thereby, the information processing system 1 can generate prediction target sequence information on sequences that are likely to give the same results of characterization. A user can set desired results of characterization and thereby know prediction target sequence information on sequences that give such results of characterization.

In the information processing system 1, the sequence selector PA1 (an example of the "sequence learner": which may be the learner 334) performs a learning process on the basis of sequence information representing sequences including some or all of the sequences of a plurality of antigen-binding molecules, and results of characterization of the antigen-binding molecules represented by the sequences and thereby generates a model trained with learning of characteristic prediction (the "second trained model": which may be a selected trained model).

The controller 335 (an example of the "estimator") estimates prediction scores for characterization of antigen-binding molecules having sequences represented by inputted prediction target sequence information by executing arithmetic processing of the model trained with learning of characteristic prediction. In the information processing system 1, the controller 335 (an example of the "estimator) inputs prediction target sequence information (an example of the "virtual sequence information") generated on the basis of the model trained with sequence learning into a selected trained model, and executes arithmetic processing of the selected trained model to give (an example of "acquiring") prediction scores for characterization of the antigen-binding molecules having the sequences represented by the inputted prediction target sequence information (an example of the "predicted values for characterization").

Thereby, the information processing system 1 can estimate a prediction score for characterization (e.g., for affinity) for each piece of prediction target sequence information generated.

According to the prediction scores estimated by the controller 335, the output processor 336 (an example of the "output") outputs on the basis of the prediction target sequence information and the prediction scores.

Thereby, the information processing system 1 can output prediction target sequences, for example, with giving priority in the descending order of results of characterization.

In the information processing system 1, the virtual sequence generator PA3 generates prediction target sequence information by changing an amino acid constituting a sequence at mutation positions in the sequence set as mutation information.

Thereby, the information processing system 1 can set a mutation position, and generate a virtual sequence obtained by changing the amino acid at the mutation position. Accordingly, the information processing system 1 can operate with fewer candidate sequences than in the case in which mutation is introduced at all positions. For example, the information processing system 1 can generate a virtual sequence with an amino acid at a mutation position changed, where the mutation position is supposed to be important for characteristics such as binding. Thus, the information processing system 1 can narrow down candidate sequences and generate sequences to which an important mutation has been introduced, and can efficiently generate desired sequences.

In the information processing system 1, mutation positions set in a sequence are included in the sequence of a heavy chain variable region, a light chain variable region, or a constant region of an antibody.

This allows, if there is a mutation position that is supposed to be important for characteristics such as binding in the heavy chain variable region or light chain variable region among the variable regions, for example, the information processing system 1 to generate a virtual sequence with the amino acid at the mutation position changed. If there is a mutation position that is supposed to be important for characteristics such as binding in the constant region, for example, the information processing system 1 can generate a virtual sequence with the amino acid at the mutation position changed.

In the information processing system 1, the output processor 336 (an example of the "output") outputs at least one piece of prediction target sequence information among a plurality of pieces of prediction target sequence information inputted into the selected trained model according to prediction scores.

This allows the information processing system 1 to output sets of prediction target sequence information generated, for example, with giving priority to those with a high prediction score. Outputting with giving priority includes outputting only those with high priority, outputting those with high priority in the top, outputting those with high priority in a display mode differing from that for those with low priority, and recommending those with high priority.

In the information processing system 1, the sequence selector PA1 (an example of the "sequence acquirer") selects a plurality of sequences according to sequence information, and analysis result information or binding determination information (an example of "evaluation result information"). The sequence learner PA2 performs a learning process according to the order of the plurality of sequences selected and thereby generates a model trained with sequence learning. Here, the learning process using the LSTM method is machine learning considering the order of sequences.

Thereby, the information processing system 1 can generate virtual sequences for antigen-binding molecules with consideration of the property due to the order of sequences.

In the information processing system 1, the sequence selector PA1 selects a plurality of sequences that show a value of analysis result information or binding determination information being higher than a predetermined value and are determined to have undergone "binding". The sequence learner PA2 performs a learning process for the plurality of sequences selected by using the sequences as inputs and outputs and thereby generates a model trained with sequence learning. Here, the learning process using the LSTM method in the present embodiment is machine learning by using sequences as inputs and outputs.

Thereby, the information processing system 1 can predict and output sequences that are likely to bind. If outputs in machine learning with a supervised model are not sequences (e.g., in the case in which outputs are characterization values), for example, it is needed to further generate sequences through operations to obtain sequences with a high characterization value. By contrast, the information processing system 1 does not need to perform further operations because the outputs from the model trained with sequence learning are sequences, and can immediately obtain sequences that are likely to bind.

In the information processing system 1, the virtual sequence generator PA3 performs machine learning with a deep learning model. Here, the virtual sequence generator PA3 may perform a learning process by using a recursive neural network (RNN), a Gated Recurrent Unit (GRU), a Generative Adversarial Network (GAN), or a Variational Autoencoder (VAE), or a Flow deep generative model as a deep learning model in place of or in addition to the LSTM.

In the information processing system 1, the virtual sequence generator PA3 may perform machine learning by using a probability model in place of or in addition to the LSTM. In this case, the virtual sequence generator PA3 performs machine learning by using a hidden Markov model (HMM) or a Markov model (MM) as a probability model.

In the information processing system 1, the virtual sequence generator PA3 performs machine learning on the basis of sequence information representing constituent elements constituting sequences as the probabilities of appearance of amino acids.

Thereby, the information processing system 1 can process constituent elements in sequence information as probabilities for a plurality of candidate amino acids, and provide constituent elements in sequence information with diversity.

(Second Embodiment)

In the following, the second embodiment of the present invention will be described in detail with reference to drawings.

In the present embodiment, the case in which candidate antibody information is outputted for antigen-binding molecules each of which binds to two or more different target molecules will be described. An antigen-binding molecule that binds to two or more different target molecules means that one antigen-binding domain in the antigen-binding molecule may bind to one target antigen and also to a target antigen differing from it. Antigen-binding molecules each of which binds to two or more different target molecules can be selected through evaluation of affinity with two or more different target molecules by using the above-described library of antigen-binding molecules. Antigen-binding molecules that bind if two or more different target molecules are present can be selected through evaluation of affinity in the presence of two or more different targets. As another mode to select two or more different target molecules, antigen-binding molecules each of which binds to two or more different target molecules but does not simultaneously bind to different target molecules can also be selected. Non-limiting examples of the technique to select antigen-binding molecules each of which does not simultaneously bind to different target molecules include the following technique. Panning is carried out by using one target molecule (target molecule A), and subsequently panning is carried out by using a target molecule differing from the target antigen (target molecule B); in this way, antigen-binding molecules each of which binds to the target molecules A and B can be selected. Subsequently, panning is carried out for the target molecule A with excessive addition of the target B in the panning reaction solution, and antigen-binding molecules for which inhibitory effect to binding activity to the target molecule A are found can be estimated to be antigen-binding molecules each of which does not simultaneously bind to the target molecules A and B. The different target molecules may be different protein antigens, or low-molecular-weight compounds. Selection of antigen-binding molecules each of which binds to two or more different target molecules is not limited to the technique using the library of antigen-binding molecules, and any technique can be used as long as the technique involves different antigen-binding molecules.

The information processing system 1a according to the present embodiment carries out both a sequence of panning in the presence of a small molecule and a sequence of panning in the absence of a small molecule as a plurality of sets of panning with different experiment conditions. In the present embodiment, experiment condition information includes information representing that the concentration of a target antigen is predetermined concentrations and a small molecule is present in a predetermined concentration (whether a small molecule is present or absent) as the composition of the buffer solution. A predetermined concentration is a concentration of a preset value or within a preset range.

The schematic diagram of the information processing system 1a is that obtained by replacing the server 30 in the information processing system 1 (Figure 1) in the first embodiment with a server 30a. The user terminal 10 and next-generation sequencer 20 have the same configurations as in the first embodiment, and hence description is omitted. Hereinafter, configurations identical to those of the first embodiment are provided with identical reference signs, and description is omitted here.

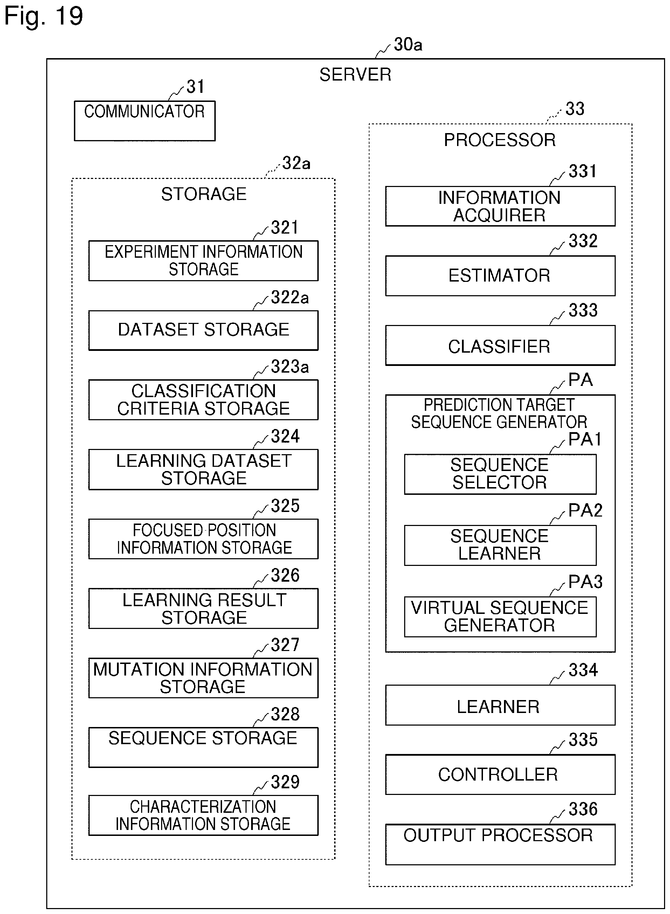

Figure 19 shows a block diagram illustrating an example of the server 30a according to the second embodiment.

The server 30a includes a communicator 31, a storage 32a, and a processor 33. The storage 32a differs from the storage 32 (Figure 6) in the first embodiment with respect to the dataset storage 322a and classification criteria storage 323a. Here, the basic functions of the dataset storage 322a are the same as those of the dataset storage 322. In the following, functions of the dataset storage 322a differing from those of the dataset storage 322 will be described.

The present embodiment shows an example in which sequences of panning in three sets are performed. The three sets are associated with the panning group IDs "P1", "P2", and "P3". Here, the experiment conditions for the panning group of "PI" are such conditions that a target antigen and small molecule are present in predetermined concentrations. The experiment conditions for "P2" are such conditions that no target antigen is present and the small molecule is present in a predetermined concentration. The experiment conditions for "P3" are such conditions that the target antigen is present in a predetermined concentration and no small molecule is present. In addition to the conditions, the experiment conditions in each case include the conditions as shown in Figure 8, and these are identical or generally identical among the sets of panning.

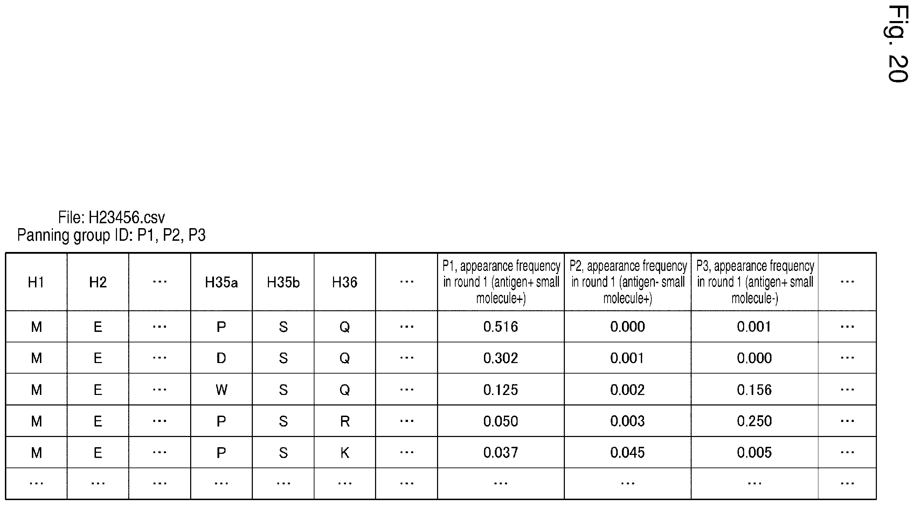

Figure 20 shows a diagram illustrating an example of a dataset according to the present embodiment.

In the dataset in this diagram, panning group ID is associated with "P1, P2, P3", and the file name is "H23456.csv". In short, the dataset in the diagram is a dataset for antibodies of heavy chain sequences, the dataset generated from analysis result information from the sequences of panning in three sets ("P1", "P2", "P3").

In the example illustrated in the diagram, the dataset is a database in which each sequence ID is associated with items of sequence information, appearance frequency in round 1 of PI, appearance frequency in round 1 of P2, and appearance frequency in round 1 of P3 for the antibody.

In the example in Figure 20, "sequence information on antibody heavy chain" corresponding to "sequence ID" of "VH001" represents that the amino acid at position "H1" is "M", the amino acid at position "H2" is "E", the amino acid at position "H35a" is "P", the amino acid at position "H35b" is "S", and the amino acid at position "H36" is "Q". "Evaluation result information on antibody heavy chain" corresponding to "sequence ID" of "VH001" represents that "P1, appearance frequency in round 1" is "0.516", "P2, appearance frequency in round 1" is "0", and "P3, appearance frequency in round 1" is "0.001".

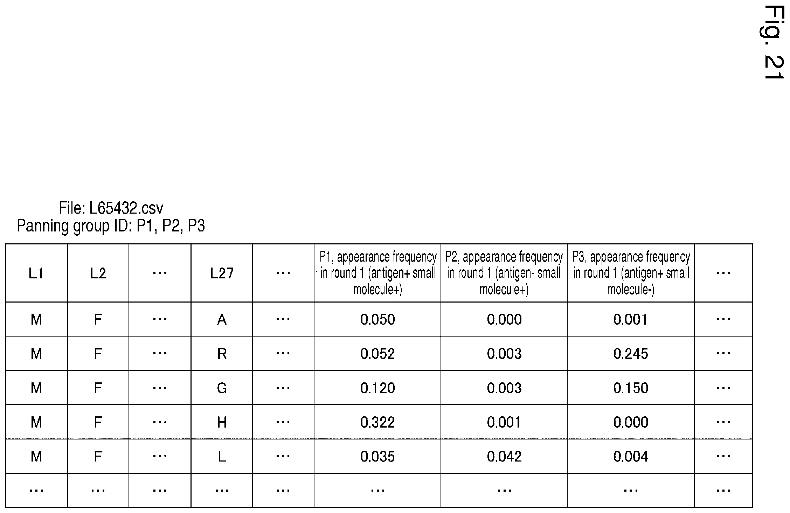

Figure 21 shows a diagram illustrating another example of a dataset according to the present embodiment.

In the dataset in this diagram, panning group ID is associated with "P1, P2, P3", and the file name is "L65432.csv".

In short, the dataset in the diagram is a dataset for antibodies of light chain sequences, the dataset generated from analysis result information from the sequences of panning in three sets ("P1", "P2", "P3 ").

In the example illustrated in the diagram, the dataset is a database in which each sequence ID is associated with items of sequence information, appearance frequency in round 1 of P1, appearance frequency in round 1 of P2, and appearance frequency in round 1 of P3 for the antibody.

The datasets in Figure 20 and Figure 21 differ in whether sequence information on antibodies represents positions in the variable region of antibody heavy chains, or positions in the variable region of antibody light chains.

In the example in Figure 21, "sequence information on antibody light chain" corresponding to "sequence ID" of "VL001" represents that the amino acid at position "L1" is "M", the amino acid at position "L2" is "F", and the amino acid at position "L27" is "A". "Evaluation result information on antibody light chain" corresponding to "sequence ID" of "VL001" represents that "P1, appearance frequency in round 1" is "0.050", "P2, appearance frequency in round 1" is "0", and "P3, appearance frequency in round 1" is "0.01".

Next, the classification criteria storage 323a will be described. Here, the basic functions of the classification criteria storage 323a are the same as those of the classification criteria storage 323. In the following, functions of the classification criteria storage 323a differing from those of the classification criteria storage 323 will be described.

The classification criteria storage 323a stores classification criteria information. The classification criteria information includes a plurality of pieces of classification criteria candidate information. For each piece of classification criteria candidate information (corresponding to criterion 1, 2, 3 in Figure 4), three thresholds are inputted. In the present embodiment, the three thresholds are appearance frequencies (or change rates of appearance frequency) for three panning groups, and appearance frequencies (or change rates) in rounds of the same round number (defined as "round A") can be set. Specifically, appearance frequency in round A of P1 (P1A), appearance frequency in round A of P2 (P2A), appearance frequency in round A of P3 (P3A), and change rates of these appearance frequencies can be set as the thresholds. For example, the criteria are such that "the appearance frequency in P1A is X4 or higher, the change rate of appearance frequency between P1A and P2A (appearance frequency in P1 A/appearance frequency in P2A) is Y4 or higher, and the change rate of appearance frequency between P1A and P3A (appearance frequency in P1A/appearance frequency in P3A) is Z4 or higher".

In the present embodiment, if appearance frequency in P2 or P3 is used as a threshold, the determination criteria are to determine a value equal to or lower than the threshold to be indicative of a binding antibody. In this case, the criteria are, for example, such that "the appearance frequency in P1A is X5 or higher, the appearance frequency in P2A is Y5 or lower, and the appearance frequency in P3A is Z5 or lower".

By setting the classification criteria information as described above, the processor 33 in the present embodiment can output candidate antibodies also for small molecule-dependent antibodies through the same processes as the processor 33 in the first embodiment performs.

<Summary>

As described above, in the information processing system 1a according to the present embodiment, the sequence learner PA2 (an example of the "sequence learner") performs a learning process using an LSTM (an example of the "machine learning") on the basis of sequence information representing sequences including some or all of the sequences of a plurality of antigen-binding molecules and thereby generates a model trained with sequence learning (an example of the "first trained model") that has learned the character of the sequences represented by the sequence information.

Here, the binding determination information is sequences of small molecule-dependent antibodies with the binding determination result being "binding". However, the plurality of sequences to be used for the learning process are appearance frequencies or change rates of appearance frequency between operations of panning. The plurality of sequences to be used for the learning process may be sequences of binding antibodies determined to have bound to the target antigen or sequences of small molecule-dependent antibodies used in a learning process for a selected trained model with the highest AUC value calculated by the sequence selector PA1.

The virtual sequence generator PA3 (an example of the "sequence generator") generates prediction target sequence information representing prediction target sequences obtained by mutating at least one of the amino acids (an example of the "constituent elements") constituting a sequence represented by the sequence information on antigen-binding molecules.

Thus, the information processing system 1a performs a learning process on the basis of sequences for which the binding determination result is "binding" when a small molecule coexists, and hence can predict a sequence or amino acids that are likely to be determined to bind when a small molecule coexists from the model trained with sequence learning.

(Third Embodiment)

In the following, the third embodiment of the present invention will be described in detail with reference to drawings.

In the present embodiment, the case in which a hidden Markov model is used as a characteristic prediction learning model will be described.

The schematic diagram of the information processing system 1b is that obtained by replacing the server 30 in the information processing system 1 (Figure 1) in the first embodiment with a server 30b. The user terminal 10 and next-generation sequencer 20 have the same configurations as in the first embodiment, and hence description is omitted. Hereinafter, configurations identical to those of the first embodiment are provided with identical reference signs, and description is omitted here.

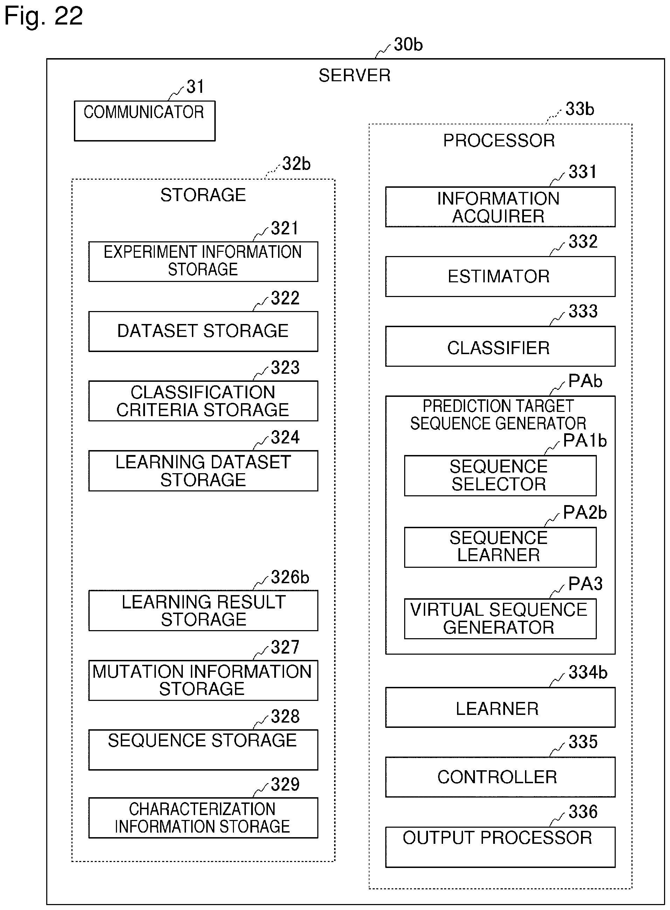

Figure 22 shows a block diagram illustrating an example of the server 30b in the information processing system 1b according to the third embodiment.

The server 30b includes a communicator 31, a storage 32b, and a processor 33b. The storage 32b differs from the storage 32 (Figure 6) in the first embodiment with respect to the absence of the focused position information storage 325 and to the learning result storage 326b. Here, the basic functions of the learning result storage 326b are the same as those of the learning result storage 326. The learning result storage 326b and the learning result storage 326 are different in their characteristic prediction learning models to store therein. The processor 33b differs from the processor 33 (Figure 6) in the first embodiment with respect to the sequence selector PAlb, the sequence learner PA2b, and the learner 334b. The basic functions of the sequence selector PA1b, sequence learner PA2b, and learner 334b are the same as those of the sequence selector PA1, sequence learner PA2, and learner 334, respectively. In the following, functions of the sequence selector PA1b differing from those of the sequence selector PA1, functions of the sequence learner PA2b differing from those of the sequence learner PA2, and functions of the learner 334b differing from those of the learner 334 will be described.

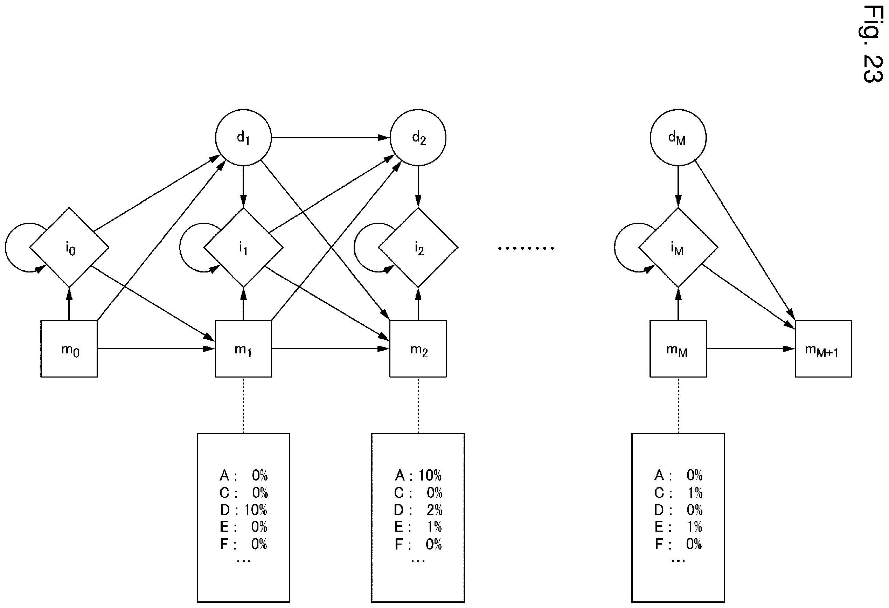

Figure 23 shows a diagram illustrating the summary of the characteristic prediction learning model according to the present embodiment. Here, an example using a hidden Markov model as a characteristic prediction learning model will be described. In the present embodiment, the amino acid sequence of one antibody is regarded as a sequence of amino acids consecutively aligned one by one.

In the example illustrated in Figure 23, each state is indicated by a square, a rhombus, or a circle. An arrow indicates a direction of state-to-state transition. Each arrow is associated with the probability of state transition from the state before transition to the state after transition.

The states are identified with predetermined identifiers. A state indicated by a square represents a state in which an amino acid is present at a certain position (hereinafter, also referred to as a "state of existence"). m is used as the identifier for states of existence. The suffix attached to the identifier denotes the position number. For example, $m_1$ indicates a state in which an amino acid is present at the first position. $m_0$ is a state indicating the beginning of state transition, and $m_{M+1}$ is a state indicating the end of state transition. Each state of existence is associated with information representing the probabilities of appearance of 20 types of amino acid in the state. In the example illustrated in Figure 23, such information associated with each state of existence is shown beneath the state.

A state indicated by a rhombus represents the presence of a state in which an amino acid has been inserted between a certain position and the next position (hereinafter, also referred to as a "state of insertion"). i is used as the identifier for states of insertion. The suffix attached to the identifier denotes the position number to be subjected to insertion. For example, $i_1$ indicates a state in which an amino acid has been inserted after the first position. As with the case of states of existence, each state of insertion is associated with information representing the probabilities of appearance of 20 types of amino acid in the state.

A state indicated by a circle represents a state in which an amino acid at a certain position has been deleted (hereinafter, also referred to as a "state of deletion"). d is used as the identifier for states of deletion. The suffix attached to the identifier denotes the position number with deletion of an amino acid. For example, $d_1$ indicates a state in which an amino acid at the first position has been deleted. In contrast to the above two states, each state of deletion is not associated with information representing the probabilities of appearance of amino acids.

The amino acid sequence of an antibody is generated as a line of amino acids that have appeared (or been inserted) at respective positions through state transitions from state mo to state $m_{M+1}$ (hereinafter, the manner of state transition is also referred to as the "route of state transition"). The route of state transition includes information on the order of transition of states and amino acids that have appeared in each state of existence or state of insertion.

When an amino acid sequence is generated through a certain route of state transition, the probability for the amino acids to be generated through the route (occurrence probability) is calculated. Here, the occurrence probability is the product of all the probabilities of state transition along the route of state transition and the probabilities of appearance of all amino acids that have appeared along the route of state transition.

An amino acid sequence is generated through a plurality of routes of state transition. Thus, the occurrence probability of an amino acid sequence is calculated as the sum total of occurrence probabilities for a plurality of routes of state transition that can generate the sequence.

Subsequently, learning performed by the sequence learner PA2b will be described.

The sequence selector PA1b reads out a learning dataset from the learning dataset storage 324 for each piece of classification criteria candidate information. Among the learning datasets, the sequence selector PA1b uses learning datasets including binding determination information with determination as being a binding antibody (referred to as "partial learning datasets") for learning performed by the sequence learner PA2b.

As in the first embodiment, the sequence selector PA1b divides partial learning datasets into learning datasets (training datasets and validation datasets) and evaluation datasets.

The sequence learner PA2b is trained with sequence information in the training datasets. Here, the sequence learner PA2b learns the probabilities of appearance of amino acids in each state of existence or state of insertion and the probabilities of state-to-state transition.

In the present embodiment, the characteristic estimation information is occurrence probabilities of amino acid sequences. The characteristic estimation information may be any of values based on occurrence probabilities of amino acid sequences, values obtained by subjecting occurrence probabilities to predetermined operations, and so on.

The sequence learner PA2b validates learning results with the validation datasets. On the basis of the validation datasets and learning results, the sequence learner PA2b inputs amino acid sequences included in the validation datasets into the hidden Markov model, and calculates the likelihoods of the sequences. For each amino acid sequence included in the evaluation data, the sequence learner PA21b determines the difference in likelihood between the group of binding sequences and the group of non-binding sequences, and numerical values based on the values are regarded as precision information.

The sequence learner PA2b changes the validation group, and repeats the above-described training and validation. This process is the same as that in the first embodiment, and hence description is omitted here.

The sequence learner PA2b calculates the average of precision information obtained in each repetition. If the average determined is not equal to or lower than a predetermined threshold, the sequence learner PA2b performs the above learning over again. If the average determined is equal to or lower than a predetermined threshold, the sequence learner PA2b orders the learning result storage 326 to store the learning results. The value to be calculated does not need to be an average value. For example, the variance or standard deviation of precision information may be calculated. In the present embodiment, in the example using a hidden Markov model as a characteristic prediction learning model, a hidden Markov model that outputs characteristic estimation information for inputs as a result of learning is called a model trained with learning of characteristic prediction.

The sequence selector PA1b selects the optimum learning model from a plurality of models trained with learning of characteristic prediction. Specifically, the sequence selector PA1b calculates an AUC (Area Under an ROC Curve) from binding determination information and affinity information for each dataset of the evaluation datasets. The sequence selector PA1b performs a learning process and an evaluation process for every piece of classification criteria candidate information, thereby calculating an AUC for a hidden Markov model for every piece of classification criteria candidate information. The sequence selector PA1b orders the learning result storage 326b to store a piece of classification criteria candidate information with the highest AUC (also referred to as the "selected classification criteria information") and the hidden Markov model for the piece of classification criteria candidate information (also referred to as the "selected trained model") with associating with the panning group ID.

The learner 334b copies the selected trained model generated by the sequence selector PAlb, and orders the learning result storage 326b to store the copy as a model trained with learning of characteristic prediction to calculate prediction scores. The learner 334 may generate a model trained with learning of characteristic prediction by performing a learning process for the hidden Markov model in Figure 23 with use of the learning dataset associated with the selected classification criteria information. The learner 334b may perform a learning process by using learning datasets with the binding determination not being "binding" in addition to learning datasets with the binding determination being "binding" or in place of some of them.

<Summary>

As described above, in the information processing system 1b according to the present embodiment, the sequence learner PA2b (an example of the "sequence learner") performs a learning process using a hidden Markov model (an example of the "machine learning") on the basis of a plurality of sequences and thereby generates a model trained with sequence learning (an example of the "first trained model") that has learned the character of the sequences represented by the sequence information. The virtual sequence generator PA3 (an example of the "sequence generator") generates prediction target sequence information (an example of the "virtual sequence information") representing prediction target sequences obtained by mutating at least one of the amino acids (an example of the "constituent elements") constituting a sequence represented by sequence information on antigen-binding molecules.

Thus, the information processing system 1b performs a learning process on the basis of sequences with the binding determination result being "binding", and hence can predict a sequence or amino acids that are likely to be determined to bind from a model trained with sequence learning as a hidden Markov model.

In the information processing system 1b, the sequence selector PA1b (an example of the "sequence learner": which may be the learner 334b) performs a learning process on the basis of sequence information representing sequences including some or all of the sequences of a plurality of antigen-binding molecules, and results of characterization of the antigen-binding molecules represented by the sequences and thereby generates a model trained with learning of characteristic prediction (the "second trained model": which may be a selected trained model).

As described above, the virtual sequence generator PA3 (an example of the "sequence generator") may generate prediction target sequence information representing virtual sequences obtained by mutating at least one of the amino acids (an example of the "constituent elements") constituting a sequence represented by sequence information on antigen-binding molecules using a model trained with sequence learning as a hidden Markov model.

In this case, the information processing system 1b can generate virtual sequences with a higher characteristic even with machine learning using a hidden Markov model.

In the information processing system 1b, the controller 335 (an example of the "estimator") inputs a plurality of pieces of prediction target sequence information generated by the virtual sequence generator PA3 into a model trained with learning of characteristic prediction (hidden Markov model; an example of the "second trained model"), and executes arithmetic processing of the model trained with learning of characteristic prediction to give (an example of "acquiring") a prediction score for the affinity of each of the plurality of virtual sequences (an example of the "predicted value for characterization").

Thereby, for each of virtual sequences generated, the information processing system 1b can estimate a prediction score for the affinity by using a hidden Markov model.

(Fourth Embodiment)

In the following, the fourth embodiment of the present invention will be described in detail with reference to drawings.

In the present embodiment, an example in which a plurality of characteristics for antigen-binding molecules is used in characterization will be described. In the present embodiment, the case in which an LSTM is used as a learning model for the model trained with sequence learning ("first trained model") and a random forest is used as a learning model for the model trained with learning of characteristic prediction ("second trained model": which may be a selected trained model) will be described.

The schematic diagram of the information processing system 1c according to the present embodiment is that obtained by replacing the user terminal 10 and the server 30 in the information processing system 1 (see Figure 1) in the first embodiment, except the next-generation sequencer 20, with a user terminal 10c and a server 30c, respectively. Hereinafter, configurations identical to those of the first embodiment are provided with identical reference signs, and description is omitted here.

In the present embodiment, an example in which a plurality of characteristics for antigen-binding molecules is used in characterization will be described. In the information processing system 1c, for one or more pieces of characteristic information selected in the user terminal 10c, pieces of sequence information that satisfy a selection condition set in the user terminal 10c are selected. Here, the selection condition is, for example, such a condition that the characteristic represented by characteristic information is good (e.g., the characteristic value is higher than a threshold), which is a condition that can be set for each piece of characteristic information.

The server 30c performs machine learning on the basis of the selected pieces of sequence information and thereby generates a model trained with sequence learning (an example of the "first trained model"). This machine learning is performed by using the LSTM (Figure 14) described as a learning model in the first embodiment.

Thus, the model trained with sequence learning is trained with pieces of sequence information that satisfy the selection condition set by a user for each piece of characteristic information selected by a user. That is, the server 30c can use a group of sequences that satisfy the selection condition of each piece of characteristic information as a group of sequences having a desired property for learning. In this case, the server 30 can generate virtual sequences that are likely to satisfy the selection condition for each piece of characteristic information, for example, as a group of many virtual sequences.

In the present embodiment, the case in which a random forest is used as a learning model for the model trained with learning of characteristic prediction (an example of the "second trained model") to give prediction scores will be described.

information Processing System>

Now, details of the present embodiment will be described.

In the information processing system 1c, the server 30c learns with use of sequence information inputted from the user terminal 10c and representing amino acid sequences of antibodies (an example of the antigen-binding molecule) and characteristic information representing the characteristics of the antibodies. The server 30c sends output information to the user terminal 10c on the basis of the input information from the user terminal 10c and learning results.

As characteristic information on antibodies, for example, characteristic information representing the characteristics relating to activity against an antigen and characteristic information representing the characteristics relating to physical properties of antibodies are inputted into the user terminal 10c. The user terminal 10c sends information in which the sequence information and the characteristic information are associated with each other to the server 30c. As conditions for selecting sequence information to be used for learning, a selection condition is inputted into the user terminal 10c for each of one or more characteristics. Sets of information required to generate prediction target sequence information (template sequence information, mutation condition information) are inputted into the user terminal 10c. The user terminal 10c sends the sets of inputted information to the server 30c.

The user terminal 10c associates the sets of characteristic information on one or more characteristics with the sequence information, and sends the resultant to the server 30c. For example, the user terminal 10c may associate two sets of characteristic information individually with the same sequence information and send the resultants. In this case, if the server 30c has received sequence information, the server 30c associates newly received characteristic information with the previously received sequence information, too.

The template sequence information is information representing an amino acid sequence that serves as a template to generate prediction target sequence information. In the present embodiment, as described later, prediction target sequences are generated by introducing a mutation into an amino acid sequence that serves as a template. At this time, the template sequence information represents an original sequence (also referred to as a "template sequence") into which a mutation is to be introduced. The template sequence is one of sequences included in the sequence information associated with the sets of characteristic information.

The mutation condition information is information representing conditions in introducing a mutation to a template sequence. The mutation condition information includes, for example, information representing the upper limit number of mutations to be introduced into a template sequence in generating one prediction target sequence.

The server 30c receives sequence information, selection conditions, and sets of characteristic information via the network NW or a storage medium, and stores the sets of information received.

The server 30c learns on the basis of sequence information that satisfies a selection condition for each piece of characteristic information, and generates a model trained with sequence learning and store it. The server 30c learns on the basis of sequence information and characteristic information for each characteristic, and generates a model trained with learning of characteristic prediction and stores it.

The server 30c receives information required to generate prediction target sequence information via the network NW or a storage medium, and stores it. The server 30c performs machine learning on the basis of sequence information for which the pieces of characteristic information satisfy selection conditions among the sets of sequence information stored and thereby generates a model trained with sequence learning. The server 30c generates prediction target sequence information on the basis of the model trained with sequence learning, and orders to store it.

On the other hand, the server 30c performs machine learning on the basis of the stored sequence information and characteristic information, and thereby generates a model trained with learning of characteristic prediction. On the basis of the model trained with learning of characteristic prediction, the server 30c predicts one or more characteristic scores for respective characteristics (an example of the characterization information) for the inputted prediction target sequence information. Here, the one or more characteristic scores are characteristic scores for the sets of characteristic information used to compare with the selection conditions.

According to the one or more characteristic scores predicted, the server 30c sends candidate antibody information representing candidate antibodies expected to bind to the target antigen to the user terminal 10c.

The user terminal 10c displays the candidate antibody information according to the characteristic scores received.

Thereby, the information processing system 1c can present candidate antigen-binding molecules with higher consideration of characteristics to be considered in being developed as a medicine than in the case without any characteristic information on antigen-binding molecules. Thus, the information processing system 1c can present desired antigen-binding molecule information.

<User Terminal>

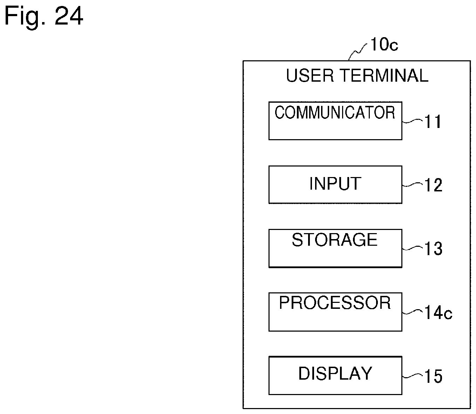

Figure 24 shows a block diagram illustrating an example of the user terminal 10c according to the present embodiment.

The user terminal 10c includes a communicator 11, an input 12, a storage 13, a processor 14c, and a display 15.

The basic functions of the processor 14c are the same as those of the processor 14 (Figure 3) in the first embodiment. In the following, functions of the processor 14c differing from those of the processor 14 will be described.

The processor 14c sends various types of information such as input information inputted from the input 12 (e.g., one or more pieces of characteristic information, sequence information, template sequence information, mutation condition information) to the server 30c via the communicator 11c. The server 30c stores relationship information (e.g., a trained model, a table) between input information and output information in advance, and generates output information for input information. The processor 14c receives the output information generated by the server 30c via the communicator 11c. The processor 14c orders the display 15 to display the output information received (an example of the output).

If the storage 13 stores relationship information, the processor 14c may read out the relationship information for input information to generate output information, and order the display 15 to display the output information.

<Server>

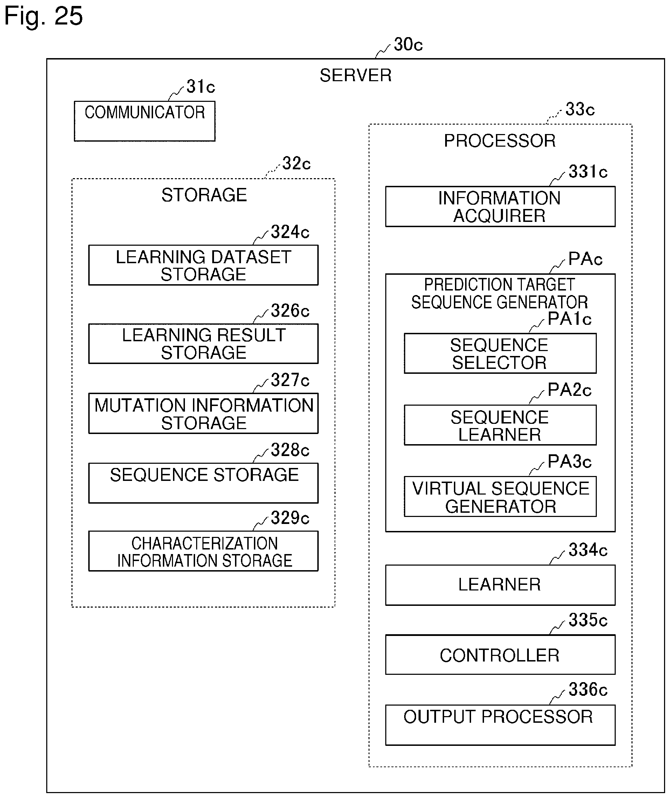

Figure 25 shows a block diagram illustrating an example of the server 30c according to the present embodiment.

The server 30c includes a communicator 31c, a storage 32c, and a processor 33c. The basic functions of each are the same as those in the case of the server 30 (Figure 6) in the first embodiment. In the following, functions of the communicator 31c, storage 32c, and processor 33c differing from those of the communicator 31, storage 32, and processor 33 will be described.

The communicator 31c is a communication module that performs various types of communication via the network NW. The communicator 31c performs various types of communication, for example, between itself and the user terminal 10c.

<Storage of Server>

The storage 32c includes a learning dataset storage 324c, a learning result storage 326c, a mutation information storage 327c, a sequence storage 328c, and a characterization information storage 329c.

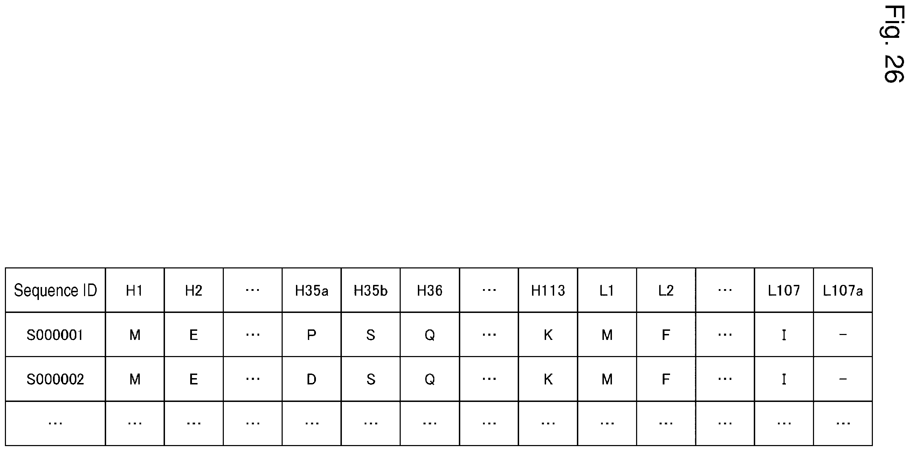

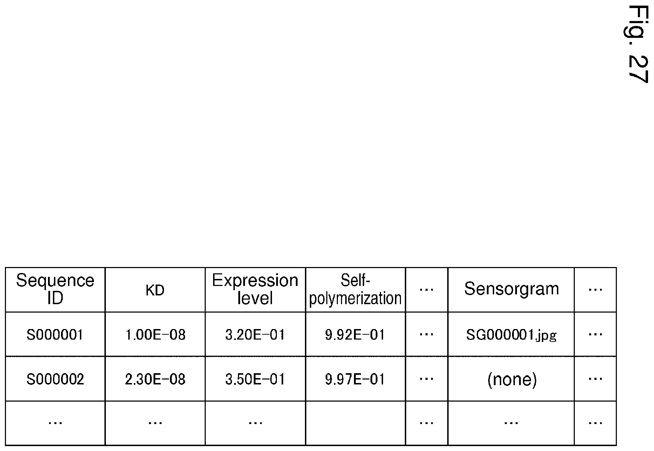

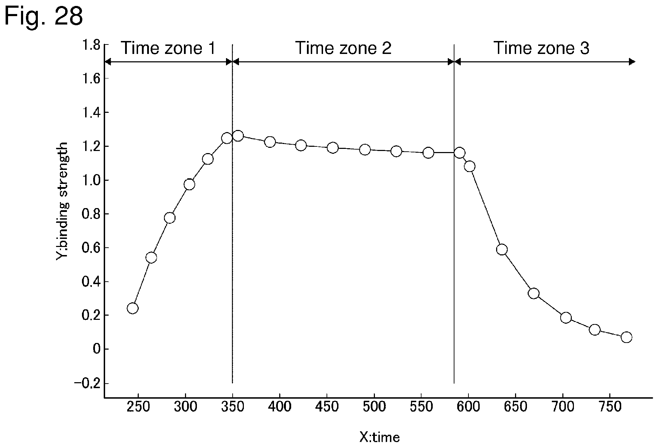

The learning dataset storage 324c stores sequence information (see Figure 26) and pieces of characteristic information (see Figure 27, Figure 28). These sets of information are included in input information from the user terminal 10c, and inputted by the processor 33c.

The learning result storage 326c stores a model trained with sequence learning and a characteristic prediction learning model as learning results from the learner 334c.

The mutation information storage 327c stores mutation information. The mutation information is, for example, template sequence information and mutation condition information included in input information from the user terminal 10c, and inputted by the processor 33c. The mutation information includes mutation position information representing mutation positions as a result of processes by the prediction target sequence generator PAc. Details of processes by the prediction target sequence generator PAc are detailed later. However, the present invention is not limited to this, and the mutation information may be set in advance in the mutation information storage 327c.

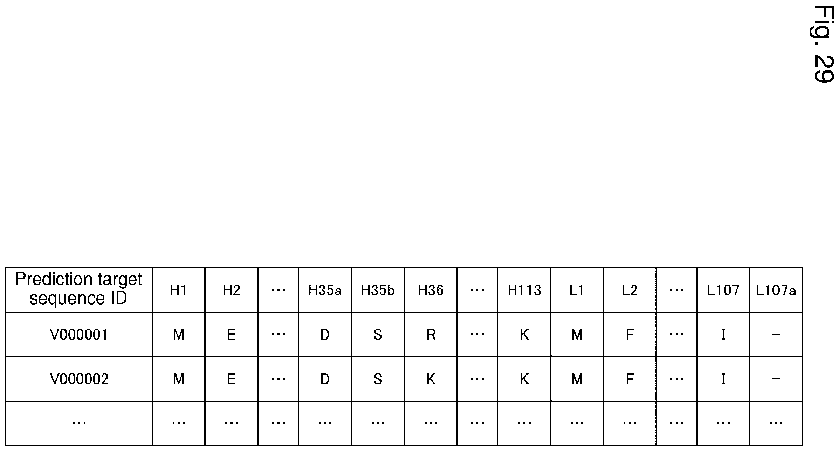

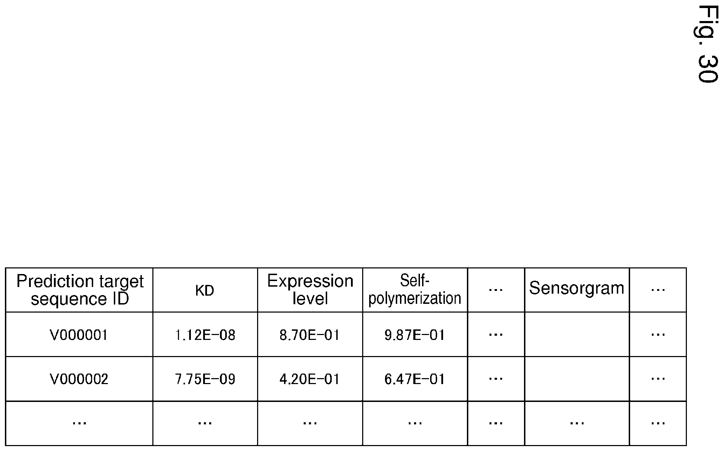

The characterization information storage 329c stores characterization information (see Figure 30) in which a prediction score given by the processor 33c with use of the model trained with learning of characteristic prediction is associated with each prediction target sequence (see Figure 29).

In the following, an example of the sequence information, pieces of characteristic information, prediction target sequence information, and characterization information stored by the storage 32c will be described with reference to Figures 26 to 30.

Figure 26 shows a diagram illustrating an example of sequence information according to the present embodiment.

In the sequence information in the example illustrated in the diagram, items of a sequence ID and sequence information on an antibody (HI, H2, ..., H35a, H35b, H36, ...H113, L1, L2...L107, L107a) are associated. Here, "Sequence ID" indicates an identifier to identify sequences of antibodies. "H1", "H2", "H35a", "H35b", "H36", "H113", "L1", "L2", "L107", and "L107a" have been associated in advance with positions of amino acids in antibodies.

The example illustrated in the diagram represents that the antibody identified by "Sequence ID" of "S000001" has an amino acid sequence in which the amino acid at position "H1" is "M", the amino acid at position "H2" is "E", the amino acid at position "H35a" is "P", the amino acid at position "H35b" is "S", the amino acid at position "H36" is "Q", the amino acid at position "H113" is "K (lysine)", the amino acid at position "L1" is "M", the amino acid at position "L2" is "F", the amino acid at position "L107" is "I (isoleucine)", and the amino acid at position "L107a" is "- (absent)".

Figure 27 shows a diagram illustrating an example of characteristic information according to the present embodiment.

In the characteristic information in the example illustrated in the diagram, items of a sequence ID, a KD, an expression level, self-polymerization, a sensorgram, and structure information are associated. Here, "KD" indicates the dissociation constant of an antibody. "Expression level" indicates the expression level when an antibody is produced. "Self-polymerization" indicates the degree of self-polymerization of an antibody. "Sensorgram" indicates data (sensorgram) representing a result of measurement of the interaction between the target antigen and an antibody by using SPR (surface plasmon resonance).

The example illustrated in the diagram represents that the characteristics of the sequence identified by "Sequence ID" of "S000001" are such that "KD" is "1.00E-08", "Expression level" is "3.20E-01", "Self-polymerization" is "9.92E-01", and "Sensorgram" is data shown in "SG000001.jpg".

For some sequences, characteristic information on all the items indicating characteristics (such as KD, expression level, self-polymerization, sensorgram) may be absent. In the example illustrated in the diagram, for example, no characteristic information on the item "Sensorgram" is present for the sequence information identified by "Sequence ID" of "S000002".

Figure 28 shows a graph representing an example of a sensorgram according to the present embodiment.

The sensorgram in Figure 28 is an example of data shown in the item Sensorgram in the characteristic information in Figure 27.

The example illustrated in the graph represents a result of measurement of the interaction between a target antigen and an antibody by using SPR in three time zones under different conditions. In the graph, the horizontal axis shows elapsed time, and the vertical axis shows intensity of binding. Time zone 1 shows a time zone in which reaction between the antibody and the antigen is carried out in a neutral reaction solution until the binding reaches saturation. Time zone 2 shows a time zone in which, after the binding has reached saturation, the binding is retained while the solution conditions are kept unchanged. Time zone 3 shows a time zone in which the pH of the reaction solution is changed to be acidic and thereafter the binding antibody and antigen gradually become dissociated.

Figure 29 shows a diagram illustrating an example of prediction target sequence information according to the present embodiment.

In the prediction target sequence information in the example illustrated in the diagram, items of a prediction target sequence ID and sequence information on an antibody (HI, H2, ..., H35a, H35b, H36, ...H113, L1, L2...L107, L107a) are associated. The prediction target sequence ID is an identifier to identify prediction target sequences. The sequence information on an antibody shows the same sequence information on an antibody in Figure 26.

The example illustrated in the diagram represents that the antibody identified by "Prediction target sequence ID" of "V000001" has an amino acid sequence in which the amino acid at position "H1" is "M", the amino acid at position "H2" is "E", the amino acid at position "H35a" is "D (aspartic acid)", the amino acid at position "H35b" is "S", the amino acid at position "H36" is "R (arginine)", the amino acid at position "H113" is "K", the amino acid at position "L1" is "M", the amino acid at position "L2" is "F", the amino acid at position "L107" is "I", and the amino acid at position "L107a" is "- (absent)".

Figure 30 shows a diagram illustrating an example of evaluation result information according to the present embodiment.

In the evaluation result information in the example illustrated in the diagram, items of a prediction target sequence ID, a KD, an expression level, self-polymerization, and a sensorgram are associated. Here, "KD" indicates an evaluation result for the dissociation constant of an antibody. "Expression level" indicates an evaluation result for the expression level when an antibody is produced. "Self-polymerization" indicates an evaluation result for the degree of self-polymerization of an antibody. "Sensorgram" indicates an evaluation result for the interaction between the target antigen and an antibody with a sensorgram.

The example illustrated in the diagram represents that the evaluation result for the sequence identified by "Prediction target sequence ID" of "V000001" is such that "KD" is "1.12E-08", "Expression level" is "8.70E-01", and "Self-polymerization" is "9.87E-01". The fields for the item "Sensorgram" are blank because evaluation results have not been obtained yet.

An evaluation value for a sensorgram is a value obtained by scoring the shape of the graph of the sensorgram. In an example, an evaluation score is a value obtained by adding a value for a term favorably scored as the maximum value of the graph is higher, a value for a term favorably scored as the decline of the graph after a certain period of time is more drastic, and so on.

<Processor of Server>

Again, with reference to Figure 25, details of the processor 33c will be described.

The processor 33c is a processor such as a central processor (CPU). On the basis of input information inputted from the communicator 31c and information stored in the storage 32c, for example, the processor 33c generates output information for input information. The processor 33c sends the output information generated to the user terminal 10c via the communicator 31c.

Specifically, the processor 33c acquires sequence information representing amino acid sequences of antibodies and characteristic information representing characteristics of antibodies from the user terminal 10c via the communicator 31c, and stores, as learning datasets, the sets of acquired information in the storage 32c.

Thereafter, the processor 33c selects a learning dataset including characteristic information on a characteristic selected by a user. The processor 33c learns the selected learning dataset on the basis of the sequence information and characteristic information on the characteristic, and thereby generates a model trained with learning of characteristic prediction. The processor 33c orders the storage 32c to store the generated model trained with sequence learning and model trained with learning of characteristic prediction.

The processor 33c acquires input information from the user terminal 10c via the communicator 31c. The input information is, for example, mutation information and so on. On the basis of the input information and the information and learning results stored in the storage 32c, the processor 33c generates candidate antibody information representing candidate antibodies that bind to the target antigen according to the degree of biding to the target antigen. The processor 33c sends the candidate antibody information generated to the user terminal 10c via the communicator 31c.

<Configuration of Processor>

In Figure 25, the processor 33c includes an information acquirer 331c, a prediction target sequence generator PAc, a learner 334c, a controller 335c, and an output processor 336c.

The information acquirer 331c acquires sequence information (see Figure 26) and characteristic information (see Figure 27) from information received from the user terminal 10c, and orders the learning dataset storage 324c to store the sets of information. The information acquirer 331c acquires mutation information from information received from the user terminal 10c, and orders the mutation information storage 327c to store it.

The prediction target sequence generator PAc and the learner 334c acquire information in which sequence information and one or more pieces of characteristic information are associated from the learning dataset storage 324c. The prediction target sequence generator PAc and the learner 334c do not acquire any information for sequence information for which no characteristic information on the characteristic is present. The prediction target sequence generator PAc and the learner 334c use the sets of information, as learning datasets, for a learning process to learn the character of sequences and a learning process and evaluation process to give prediction scores. Here, sequence information that satisfies a selection condition for each characteristic is used in the learning process to learn the character of sequences.

<Learning Process for Generation of Prediction Target Sequences>

The prediction target sequence generator PAc selects learning datasets. As a learning process to generate prediction target sequences (a learning process to learn the character of sequences), the prediction target sequence generator PAc performs a learning process with use of an LSTM, which has been described in the first embodiment, as a learning model on the basis of the sequence information in the selected learning datasets.

Here, for the learning datasets, for example, a user selects one or more characteristics from characteristics in characterization of antigen-binding molecules and sets a selection condition for each characteristic. Examples of the characterization include evaluation of affinity, evaluation of pharmacological activity, evaluation of physical properties, evaluation of kinetics, and evaluation of safety for antigen-binding molecules. Examples of characteristics for evaluation of affinity include binding activity, examples of characteristics for evaluation of pharmacological activity include pharmacological activity, and examples of characteristics for evaluation of physical properties include thermal stability, chemical stability, solubility, viscosity, photostability, long-term storage stability, and non-specific adsorptivity. Among learning datasets, the sequence selector PA1 selects learning datasets whose characteristic information satisfies the selection condition.

On the basis of the sequence information in the selected learning datasets, the prediction target sequence generator PAc performs a learning process to learn the character of sequences, and thereby generates a model trained with sequence learning.

For example, a user may select one or more characteristics for any one of evaluation of affinity, evaluation of pharmacological activity, evaluation of physical properties, evaluation of kinetics, and evaluation of safety or any combination of them, and set a selection condition for each characteristic. In this case, among learning datasets, the prediction target sequence generator PAc selects learning datasets in which the characteristic information satisfies the selection condition for any one of evaluation of affinity, evaluation of pharmacological activity, evaluation of physical properties, evaluation of kinetics, and evaluation of safety or any combination of them. On the basis of the sequence information in the selected learning datasets, the prediction target sequence generator PAc performs a learning process to learn the character of sequences, and thereby generates a model trained with sequence learning.

[Selection Condition]

For example, the following condition is a selection condition that can be set for each characteristic.

When the characteristic is binding activity, the selection condition is such a condition that intensity of binding is high (e.g., the KD (dissociation rate constant) is equal to or higher than a threshold). As described above, intensity of binding is the total intensity of non-covalent interactions between one or more binding sites of a molecule (e.g., an antibody) and a binding partner for the molecule (e.g., an antigen).

When the characteristic is stability such as thermal stability and chemical stability of antigen-binding molecules, the selection condition is such a condition that stability is high (equal to or higher than a threshold). Although stability differs among evaluation methods to measure stability, denaturation midpoint (Tm), which is an index of thermal stability, can be used for determination, and high Tm is expected as high thermal stability. Evaluation can be made by measuring the decomposition, chemical modification, or association of the antigen-antibody molecule before and after treatment intended for the evaluation of stability such as heat treatment, exposure to a low-pH environment, exposure to light, stirring with a machine, and long-term storage, and high stability is expected if the antigen-binding molecule undergoes less decomposition, chemical modification, or association. When the characteristic is for evaluation of non-specific binding based on evaluation of binding to the extracellular matrix (ECM), the selection condition is such a condition that binding intensity to the ECM is low (equal to or lower than a threshold). Low binding intensity to the EMC is expected as low non-specific binding.

The protein expression level of the antigen-binding molecule can be measured in such a manner that a gene encoding the antigen-binding molecule is introduced into expression cells, and the concentration of the antigen-binding molecule in the culture supernatant is measured after the expression cells are cultured for a certain period of time, and the case in which the concentration of the antigen-binding molecule in the culture supernatant is high is expected as "high expression level". In this case, the selection condition is such a condition that the concentration is high (equal to or higher than a threshold).

<Learning Process to Give Prediction Scores>

As a learning process to give prediction scores, the learner 334c performs a learning process in which sequence information in learning datasets is input variables and character information is output variables.

Specifically, the learner 334c selects learning datasets each including a value of characteristic information for the characteristic selected by a user. The learner 334c learns on the basis of the sequence information and characteristic information in the selected learning datasets, and thereby generates a model trained with learning of characteristic prediction. The sequence information to be used to generate the model trained with learning of characteristic prediction includes sequence information for which the characteristics do not satisfy the selection conditions. For this reason, the model trained with learning of characteristic prediction can also precisely give prediction scores with bad characteristics (e.g., low characteristic values) that do not satisfy the selection conditions. However, the sequence information to be used to generate the model trained with learning of characteristic prediction does not need to include sequence information for which the characteristics do not satisfy the selection conditions.

The prediction target sequence generator PAc performs machine learning using an LSTM as a learning model for the model trained with sequence learning. On the other hand, the learner 334c performs machine learning using a random forest as a learning model for the model trained with learning of characteristic prediction. In this way, the learning model used by the prediction target sequence generator PAc and the learning model used by the learner 334c may be of different types.

In the following, details of a learning process and evaluation process in which the learner 334 uses a random forest as a learning model for the model trained with learning of characteristic prediction will be described. However, the present invention is not limited to this, and another learning model may be used as a characteristic prediction learning model in the learning process and evaluation process.

<Learning Process and Evaluation Process>

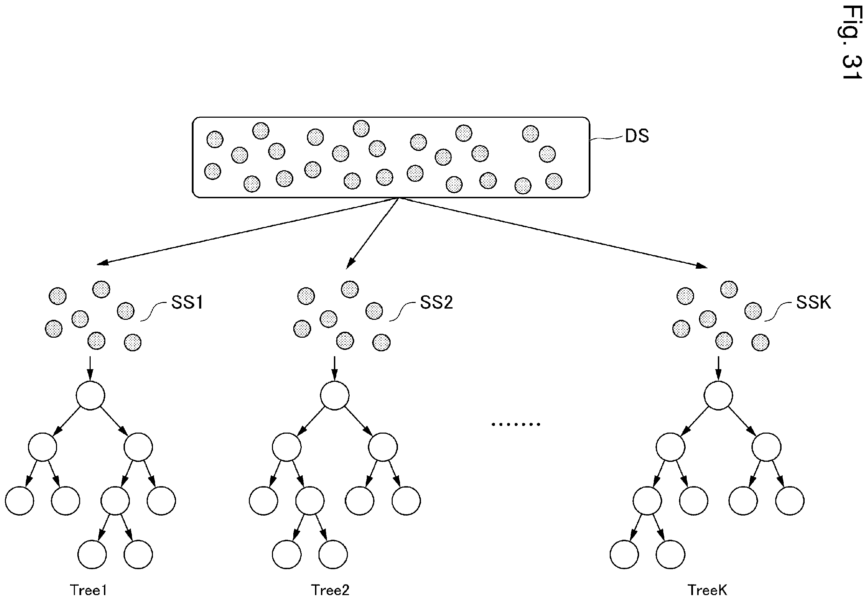

Figure 31 shows an illustration for describing an example of the learning process according to the present embodiment. Figure 31 shows an example of the learning process to generate the model trained with learning of characteristic prediction, and an example of the learning process when characteristic information can be expressed as numeric values.

In Figure 31, a learning dataset is represented by a hatched circle.

The learner 334c extracts learning datasets (hereinafter, also referred to as "subsets") with a predetermined number of data (e.g., 100 data) from learning datasets DS, for example, at random. The learner 334c repeats such extraction for predetermined cycles (e.g., K cycles) to generate K subsets: SS1 to SSK. The learner 334c generates a decision tree Treek (k = 1 to K) for each subset SSk (k = 1 to K).

Here, the learner c sets elements of subject sequence information (elements of a sequence), that is, information on amino acids at respective positions as independent variables. The learner 334c sets characteristic information as dependent variables. For the independent variables and dependent variables set, the learner 334c generates decision trees by using the subsets (an example of "learning").

In Figure 31, each decision tree is composed of a plurality of nodes (open circles) and edges (arrows) connecting nodes. If two nodes are connected with an arrow, the node at the start of the arrow is referred to as a parent node and the node at the end of the arrow is referred to as a child node. Each node has at most one parent node. A node without any parent node is referred to as a root node.

In the stage of execution, input data as subjects of estimation (prediction target sequence information on antibodies) are classified from a root node to any node with no child node (hereinafter, also referred to as a "leaf node") along direction of arrows. Which arrow is employed for input data depends on the determination criterion associated with the node. As a result of learning, the determination criterion is associated with an independent variable, that is, information on an amino acid at each position. For example, the determination criterion at a certain node is a criterion for information on an amino acid at a position with sequence information of "H95a", and such that classification proceeds to the direction of the right arrow if the amino acid is L (leucine), and to the direction of the left arrow if the amino acid is I (isoleucine).

Since leaf nodes have no next child node, a determination criterion is not associated with leaf nodes. At each leaf node, characteristic estimation information is associated with the antibody represented by input data after going through nodes. The characteristic estimation information is a result of estimation of characteristic information for input data that have reached a leaf node. The characteristic estimation information to be associated with each leaf node in each decision tree Treek is determined with the subset SSk.

In the stage of learning, the characteristic estimation information is determined on the basis of learning datasets classified into leaf nodes. For example, the characteristic estimation information is a value based on a statistic of characteristic information included in learning datasets that have reached leaf nodes. The characteristic estimation information is, for example, the average value of characteristic information. The characteristic estimation information may be, for example, the maximum value or minimum value of characteristic information, or a value determined, for example, on the basis of the average value, maximum value, minimum value, or standard deviation.

Next, the learner 334c performs an evaluation process for the plurality of decision trees generated, Tree1 to TreeK.

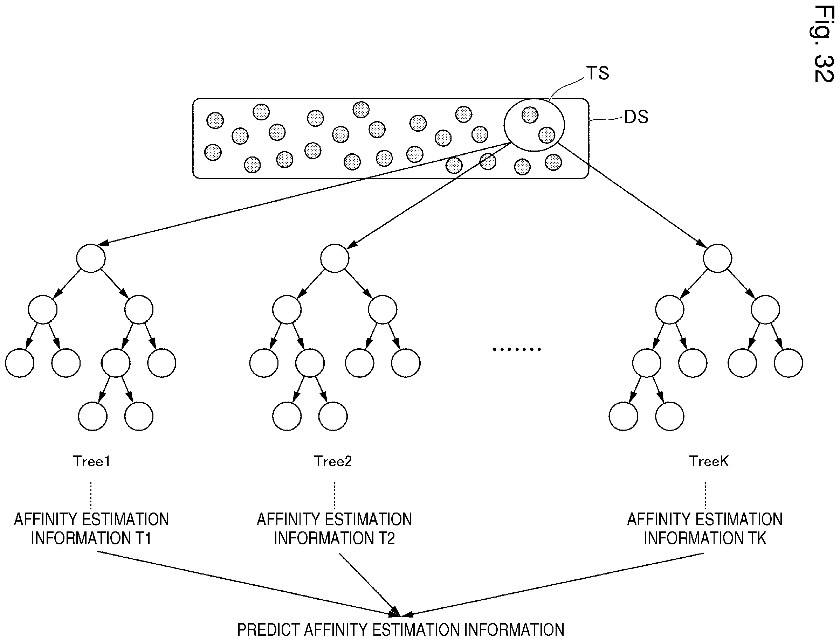

Figure 32 shows an illustration for describing an example of the evaluation process according to the present embodiment. As Figure 31, Figure 32 shows an example of the learning process for the case in which characteristic information can be expressed as numeric values.

The learner 334c selects one or more learning datasets that are not included in any of the subsets SS1 to SSK (also referred to as "evaluation datasets") from learning datasets DS. Figure 32 is a diagram for the case in which two evaluation datasets TD are selected.

For each dataset of the evaluation datasets TD, the learner 334c inputs the sequence information into each of the decision trees Tree1 to TreeK, and acquires K pieces of characteristic estimation information T1 to TK. The learner 334c calculates a representative value based on the pieces of characteristic estimation information T1 to TK as characterization information. The representative value is, for example, the average value of the pieces of characteristic estimation information T1 to TK, but the present invention is not limited to this, and the representative value may be the maximum value or minimum value. For each dataset of the evaluation datasets TD, the learner 334c compares the characteristic information and the characteristic estimation information, and the difference between the characteristic information and the characteristic estimation information by using a predetermined method. The predetermined method is, for example, a method of calculating the average absolute error for all the data in the evaluation datasets TD. The learner 334c determines whether the difference between the characteristic information and the characteristic estimation information determined by using the predetermined method falls within a predetermined range. If the difference falls within a predetermined range, the learner 334c determines completion of learning, and terminates the learning process and evaluation process. If the difference does not fall within a predetermined range, the learner 334c performs learning over again.

Determination of the difference between the characteristic information and the characteristic estimation information is not limited to the above-described method. For example, a method of determining mean squared errors, root mean squared errors, coefficients of determination, or the like may be used.

In the present embodiment, when characteristic information can be expressed as numeric values, a model that outputs characterization information based on pieces of characteristic estimation information T1 to TK for input data with use of a collection of decision trees Tree1 to TreeK is called a trained model.

When characteristic information is an image in learning to generate the model trained with learning of characteristic prediction, the learner 334c extracts feature values in the image in accordance with a method stored in advance in the storage 32c, and the above-described processes are carried out by using the extracted feature values as characteristic information. In addition, determination criteria for whether the extracted feature values are superior or not are also stored in advance in the storage 32c. For extraction of feature values and determination criteria therefor, for example, a known method using machine learning is used.

When the image is a graph or the like, for example, the slope of a graph or coefficients of a function representing an approximate curve for a graph is calculated as a feature value. In the case of a graph as illustrated in Figure 28, for example, an approximate curve is calculated for each time zone, and coefficients of the function representing the approximate curve are determined as feature values. In the example shown in the graph, for example, the slope of an approximate line is used as a feature value for Time zone 1 and Time zone 2. For Time zone 3, exponential approximation is carried out and the half life is used as a feature value.

After learning, the prediction target sequence generator PAc and the learner 334c order the learning result storage 326c to store the model trained with sequence learning and the model trained with learning of characteristic prediction, respectively.

<Generation Process for Prediction Target Sequences>

In the following, a generation process for prediction target sequences that is performed by the prediction target sequence generator PAc will be described.

The prediction target sequence generator PAc includes a sequence selector PA1c, a sequence learner PA2c, and a virtual sequence generator PA3c.

The sequence selector PA1c acquires sequence information on antibodies that exhibits a characteristic superior to that of the antibody with the template sequence on the basis of information stored in the learning dataset storage 324c in learning to generate the model trained with sequence learning and template sequence information stored in the mutation information storage 327c. For example, the sequence selector PA1c reads out template sequence information from in the mutation information storage 327c. The sequence selector PA1c selects sequence information matching the template sequence information from the learning dataset storage 324c. The sequence selector PA1c acquires characteristic information associated with the selected sequence information (hereinafter, also referred to as "reference characteristic information"). From the characteristic information stored as selection conditions in the learning dataset storage 324c, the sequence selector PA1c selects characteristic information that exhibits a superior characteristic to the reference characteristic information (hereinafter, also referred to as "improved characteristic information"). Here, the expression "exhibits a superior characteristic" indicates that the characteristic information is superior to the reference characteristic information for a predetermined characteristic item. The determination criterion for being superior or not is based on determination criteria stored in advance in the storage 32c. The sequence selector PA1c orders the mutation information storage 327c to store sequence information corresponding to the improved characteristic information.

Although the sequence selector PA1c selects a group of sequences having a better characteristic value than the template in this example, however, the sequence selector PA1c may select a group of sequences $\alpha$ times better than that of the template or the top N sequences (N is a natural number) in terms of their characteristic values and use as sequence information corresponding to the improved characteristic information.

The sequence learner PA2c acquires the sequence information corresponding to the improved characteristic information from the mutation information storage 327c. The sequence learner PA2c performs machine learning with use of the sequence information acquired. The learning process is the same as that in the first embodiment, and hence description is omitted here. As a result of machine learning, the sequence learner PA2c generates a model trained with sequence learning.

The virtual sequence generator PA3c generates prediction target sequences with use of the model trained with sequence learning generated by the sequence learner PA2c. The method for generating prediction target sequences is the same as that in the first embodiment, and hence description is omitted here (see Figure 16). The virtual sequence generator PA3c orders the sequence storage 328c to store the prediction target sequence information generated.

<Giving Prediction Scores>

The controller 335c reads out the prediction target sequence information from the sequence storage 328c. The controller 335c inputs the prediction target sequence information read out as input data into the model trained with learning of characteristic prediction, and outputs prediction scores. The controller 335c orders the characterization information storage 329c to store, as characterization information, the prediction target sequence information and the prediction score given. For the prediction target sequence information in Figure 29, for example, the controller 335c orders to store prediction scores corresponding to the prediction target sequence information. The model trained with learning of characteristic prediction may be a Gaussian process, and each prediction score given by the Gaussian process may be reliability of prediction.

The controller 335c can also determine characterization information on a characteristic that is estimated, for example, by using a plurality of characteristics outputted and not included in learning. Such a characteristic is, for example, information representing the viscosity of an antibody or the possibility of humanization of an antibody. The method for estimating such characterization information is determined and stored in advance in the storage 32c.

According to the prediction scores for the prediction target sequence information, the output processor 336c outputs the prediction target sequence information as candidate antibody information. The candidate antibody information represents candidate antibodies having a high characteristic.

Specifically, for example, the output processor 336c reads out characterization information from the characterization information storage 329c, and performs ordering in the descending order of prediction scores. Since a prediction score is present for each characteristic, an ordering results are present for each characteristic. The output processor 336c integrates the ordering results for prediction target sequence information on the respective characteristics to perform ordering for the total characteristics. The ordering for the total characteristics is performed, for example, on the basis of values obtained by averaging orders for the respective characteristics. The method for ordering for the total characteristics is not limited to the described method. For example, the sum of orders for respective characteristics or the sum of predetermined scores corresponding to orders may be used. Alternatively, weighting of characteristics may be performed to determine candidate antibody information. Specifically, by weighting a characteristic of concern, candidate antibody information with sequences being superior in the characteristic becomes more likely to be determined.

The output processor 336c generates prediction target sequence information ordered in the descending order of the total characteristics as candidate antibody information. The output processor 336c sends the candidate antibody information generated to the user terminal 10c via the communicator 31c through the network NW. The output processor 336c may send the prediction target antibody information to the user terminal 10c, and the user terminal 10c (processor 14c) may sort the received prediction target antibody information by using the above-described method and display on the display 15.

<Operations>

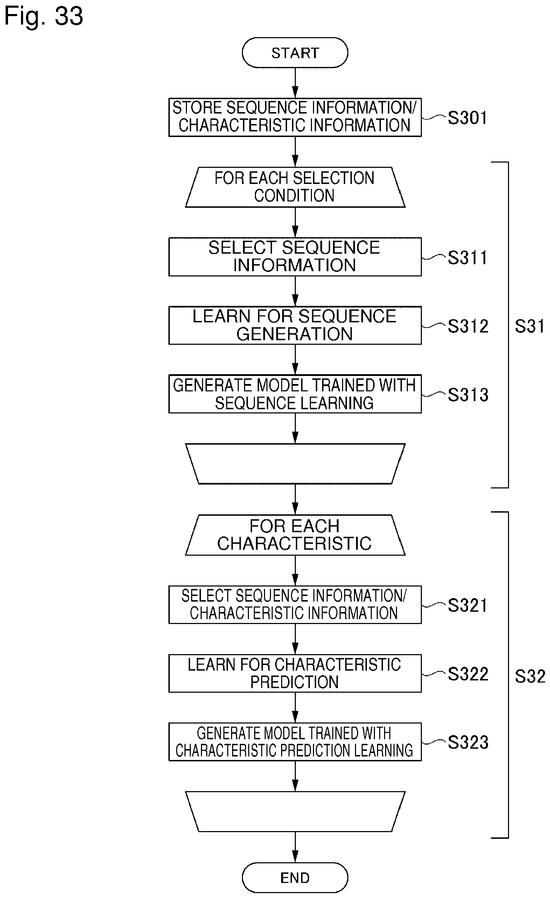

Figure 33 shows a flowchart illustrating an example of operations of the server 30c according to the present embodiment. This diagram illustrates operations of the server 30c in the stage of learning (learning process and evaluation process).

(Step S301) The information acquirer 331c acquires various types of information from the user terminal 10c. The information acquirer 331c orders the storage 32c to store the acquired information. Thereafter, step S311 follows. Processes in steps S311 to S313 are performed for each selection condition. These processes are the learning process S31 to generate prediction target sequences.

(Step S311) For learning datasets that satisfy a selection condition among learning datasets stored in step S301, the sequence selector PA1c selects sequence information corresponding to improved characteristic information as sequence information on the learning datasets. Thereafter, step S312 follows.

(Step S312) The sequence learner PA2c performs a learning process to generate prediction target sequences with use of the sequence information selected in step S311. Thereafter, step S313 follows.

(Step S313) The sequence learner PA2c orders the learning result storage 326c to store a model trained with sequence learning generated through the learning process in step S312. Thereafter, step S321 follows. The processes in steps S321 to S323 are performed for each of one or more characteristics. These processes are the learning process S32 to give prediction scores.

(Step S321) The learner 334c selects sequence information and characteristic information for learning datasets with values of one or more characteristics for selection conditions (characteristics in improved characteristic information) among learning datasets stored in step S301. Thereafter, step S322 follows.

(Step S322). The learner 334c performs a learning process to give prediction scores for each of one or more characteristics with use of the learning datasets selected in step S321. Thereafter, step S323 follows.

(Step S323) The learner 334c orders the learning result storage 326c to store the model trained with learning of characteristic prediction generated through the learning process in step S322. Thereafter, the operations in the diagram are terminated.

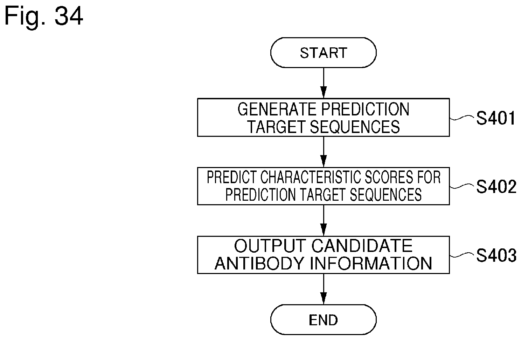

Figure 34 shows a flowchart illustrating another example of operations of the server 30c according to the present embodiment. This diagram illustrates operations of the server 30c in the stage of execution. The stage of execution is a stage in which the information processing system 1c after learning with learning datasets generates prediction target sequences with use of the model trained with sequence learning, and gives prediction scores with use of the model trained with learning of characteristic prediction.

(Step S401) The virtual sequence generator PA3 generates prediction target sequence information with use of the model trained with sequence learning generated in step S313 of Figure 33. The prediction target sequence generator PA3c orders the sequence storage 328c to store the prediction target sequence information generated. Thereafter, step S402 follows.

(Step S402) The controller 335c gives prediction scores for the prediction target sequence information generated in step S401 with use of the model trained with learning of characteristic prediction generated in step S323 of Figure 33. Thereafter, step S403 follows.

(Step S403) The output processor 336c performs ordering for the total characteristics on the basis of one or more prediction scores given in step S402. The output processor 336c outputs prediction target sequence information as candidate antibody information on the basis of the ordering for the total characteristics. The outputted candidate antibody information is displayed on the user terminal 10c. Thereafter, the operations in the diagram are terminated.

As described above, in the information processing system 1c according to the present embodiment, the sequence learner PA2c (an example of the "sequence learner") performs a learning process (an example of the "machine learning") using an LSTM on the basis of a plurality of sequences, and thereby generates a model trained with sequence learning (an example of the "first trained model") that has learned the character of sequences represented by sequence information.

Here, the plurality of sequences used for the learning process is sequences that satisfy a selection condition set by a user for each piece of characteristic information.

The virtual sequence generator PA3c generates prediction target sequence information (an example of the "virtual sequence information") representing virtual sequences obtained by mutating at least one of the amino acids (an example of the "constituent elements") constituting a sequence represented by sequence information on antigen-binding molecules.

Thereby, the information processing system 1c can generate virtual sequences that are likely to satisfy a selection condition set for each piece of characteristic information selected.

In the information processing system 1c, the controller 335 (an example of the "estimator") inputs the prediction target sequence information generated on the basis of the model trained with sequence learning into a selected trained model (decision trees; an example of the "second trained model"), and executes arithmetic processing of the selected trained model give (an example of "acquiring") prediction scores for characterization of the antigen-binding molecules having the sequences represented by the inputted prediction target sequence information (an example of the "predicted values for characterization").

Thereby, information processing system 1c can estimate a prediction score for characterization (e.g., for affinity) by using decision trees for each piece of prediction target sequence information generated.

(Fifth Embodiment)

In the following, the fifth embodiment of the present invention will be described with reference to drawings.

In the present embodiment, an information processing system 1d actually measures the characteristics of antibodies having prediction target sequences predicted (e.g., antibodies represented by candidate antibody information). On the basis of sequence information representing the sequences of the antibodies and characteristic information representing the characteristics measured, the information processing system 1d performs further machine learning for a model trained with sequence learning or a model trained with learning of characteristic prediction. With use of the model trained with sequence learning after performing the further machine learning, the information processing system 1d generate new prediction target sequence information.

In the present embodiment, the information processing system 1d repeats the sequence of processes. That is, in the present embodiment, the information processing system 1d performs, as one cycle, learning on the basis of learning datasets, generation of prediction target sequences on the basis of learning results, measurement with use of antibodies represented by candidate sequence information, and addition of measurement results and sequence information to learning datasets, and repeats this cycle.

The schematic diagram of the information processing system 1d according to the present embodiment is that obtained by replacing the server 30c in the information processing system 1c in the fourth embodiment with a server 30d. The user terminal 10d has the same configurations as in the fourth embodiment, and hence description is omitted. Hereinafter, configurations identical to those of the fourth embodiment are provided with identical reference signs, and description is omitted here.

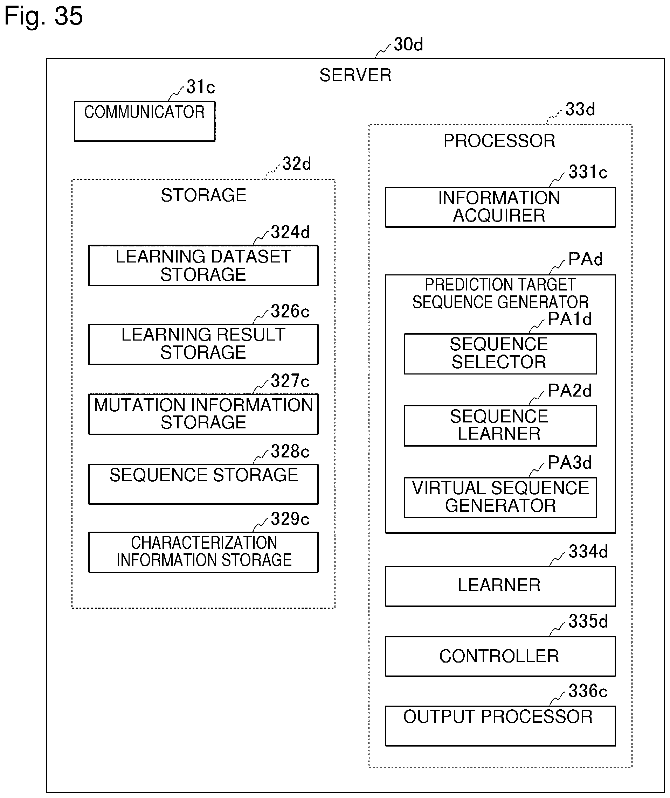

Figure 35 shows a block diagram illustrating an example of the server 30d according to a fifth embodiment.

The server 30d includes a communicator 31c, a storage 32d, and a processor 33d.

The storage 32d differs from the storage 32c in the fourth embodiment (Figure 25) with respect to that the learning dataset storage 324d is present in place of the learning dataset storage 324c. Here, the basic functions of the learning dataset storage 324d are the same as those of the learning dataset storage 324c. In the following, functions of the learning dataset storage 324d differing from those of the learning dataset storage 324c will be described.

The learning dataset storage 324d stores sequence information and characteristic information. These sets of information are included in input information from the user terminal 10c, and inputted by the processor 33d. Here, the sequence information includes cycle number information indicating the cycle in which the prediction target sequence generator PAd has performed prediction.

<Configuration of Processor>

The processor 33d in Figure 35 differs from the processor 33c in the fourth embodiment (Figure 25) with respect that the learner 334d, prediction target sequence generator PAd, and controller 335d are present in place of the learner 334c, prediction target sequence generator PAc, and controller 335c, respectively. Here, the basic functions of the learner 334d, prediction target sequence generator PAd, and controller 335d are the same as those of the learner 334c, prediction target sequence generator PAc, and controller 335c, respectively. In the following, functions of the learner 334d, prediction target sequence generator PAd, and controller 335d differing from those of the learner 334c, prediction target sequence generator PAc, and controller 335c, respectively, will be described.

The prediction target sequence generator PAd and the learner 334d acquire sequence information and one or more pieces of characteristic information from the learning dataset storage 324d. The prediction target sequence generator PAd and the learner 334d do not acquire any information for sequence information for which no characteristic information on the characteristic is present. The prediction target sequence generator PAd and the learner 334d use a part of the sets of information, as learning datasets, for a learning process to learn the character of sequences and a learning process and evaluation process to give prediction scores. Here, sequence information that satisfies a selection condition for each characteristic is used in the learning process to learn the character of sequences.

<Operations of Information Processing System 1d>

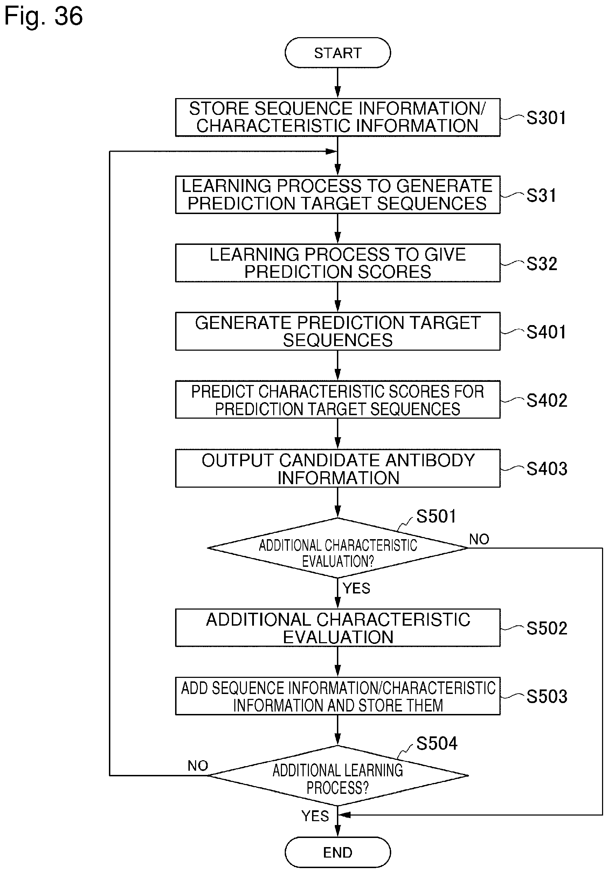

Figure 36 shows a flowchart illustrating an example of operations of the information processing system 1d according to the present embodiment. In Figure 36, identical reference signs are provided to the same processes as to those in Figures 33 and 34.

(Step S301) The information acquirer 331c acquires various types of information from the user terminal 10c. The information acquirer 331c orders the storage 32d to store the acquired information. Thereafter, step S31 follows.

(Step S31) The sequence selector PAld and the sequence selector PAld perform the learning process S31 in Figure 33 (processes in steps S311 to S313 performed for each selection condition) to generate prediction target sequences, and thereby generate a model trained with sequence learning. Thereafter, step S32 follows.

(Step S32) The learner 334d performs the learning process S32 in Figure 33 (processes in processing steps S321 to S323 performed for each of one or more characteristics) to give prediction scores, and thereby generates a model trained with learning of characteristic prediction. Thereafter, step S401 follows.

(Step S401) The virtual sequence generator PA3d generates prediction target sequence information with use of the model trained with sequence learning generated in step S31. The virtual sequence generator PA3d orders the sequence storage 328c to store the prediction target sequence information generated. Thereafter, step S402 follows.

(Step S402) The controller 335d gives prediction scores for the prediction target sequence information generated in step S401 with use of the model trained with learning of characteristic prediction generated in step S323 in Figure 33. Thereafter, step S403 follows.

(Step S403) The output processor 336c performs ordering for the total characteristics on the basis of one or more prediction scores given in step S402. The output processor 336c outputs prediction target sequence information as candidate antibody information on the basis of the ordering for the total characteristics. The outputted candidate antibody information is displayed on the user terminal 10c.

(Step S501) The controller 335d determines whether additional characterization is performed or not, for example, according to input from the user terminal 10c. In the additional characterization, panning for a plurality of antibodies and the target antigen is further performed, and the analysis result information is outputted from the next-generation sequencer 20. In the additional characterization, an antibody library containing candidate antibodies represented by the candidate antibody information is preferably used. If determination is made to perform the additional characterization (Yes), step S502 follows; if determination is made not to perform the additional characterization (No), the operations in the diagram are terminated.

(Step S502) The additional characterization is performed, and as a result the next-generation sequencer 20 outputs analysis result information. This analysis result information preferably includes, as antibodies, candidate antibodies represented by the candidate antibody information. Thereafter, step S503 follows.

(Step S503) The information acquirer 331d acquires various types of information from the user terminal 10c. The information acquirer 331c adds the acquired information to the information stored in step S301 or previous step S503, and orders the storage 32d to store the resultant information. This information includes sequence information and one or more pieces of characteristic information as analysis result information outputted in step S502. Thereafter, step S504 follows.

(Step S504) The controller 335d determines whether an additional learning process is performed or not, for example, according to input from the user terminal 10c. If determination is made to perform the additional learning process (Yes), step S31 is done over again; if determination is made not to perform the additional learning process (No), the operations in the diagram are terminated.

Here, the sequence selector PAld and the learner 334d each select information including learning datasets from the acquired information as follows. The sequence selector PAld and the learner 334d acquire the upper limit number of learning datasets from the storage 32d. The upper limit number is, for example, the number of learning datasets used in the first cycle of learning. The upper limit number is determined in advance for each learning process (a learning process to learn the character of sequences, a learning process to give prediction scores), that is, for each trained model (a model trained with sequence learning, a model trained with learning of characteristic prediction), and stored in the storage 32d.

The sequence selector PAld and the learner 334d learn with learning datasets in a number smaller than the upper limit number in at least two different cycles. In other words, the number ratio of learning datasets in previous cycles to all learning datasets is reduced. Thereby, the information processing system 1d can gradually reduce the influence of characterization in previous cycles with reflecting characterization by the latest panning. In this case, the information processing system 1d can converge prediction target sequences or prediction scores outputted from each trained model without causing large divergence, in some cases.

For example, the sequence selector PAld and the learner 334d each refer to sequence information and preferentially acquire sequences generated in a cycle near the current cycle as learning datasets. From sequences generated in a certain cycle (defined as the Mth cycle), the sequence selector PAld and the learner 334d each select sequences to be included as learning datasets and sequences not to be included as learning datasets if inclusion of all the learning datasets leads to excess over the upper limit number. For example, the sequence selector PAld and the learner 334d each perform ordering of sequences generated in the Mth cycle on the basis of characteristic information corresponding to the sequences. The method of ordering is, for example, based on the average of orders obtained through ordering performed for respective characteristics. The method of ordering is not limited to this. The sequence selector PAld and the learner 334d may select learning dataset in each cycle with the same method, and learning datasets selected by one may be used by the other.

The sequence selector PAld and the learner 334d each acquire sequences in higher ranks superior in characteristics as sequences to be included in learning datasets on the basis of results of ordering. The learner 334d performs the above process until the number of learning datasets reaches the upper limit number.

The sequence learner PA2d and the learner 334d each performs a learning process on the basis of the acquired learning datasets. The learning process is the same as the method described for the fourth embodiment, and hence description is omitted here. The learner 334d orders the learning result storage 326c to store learning results.

Selection conditions may be different among cycles, and a user may select characteristics and set selection conditions for the characteristics selected in each cycle. For example, selection conditions in a later cycle may be stricter (e.g., higher characteristic values) than selection conditions in an earlier cycle, or rather looser (e.g., lower characteristic values) than selection conditions in earlier cycles. Characteristics for selection conditions in a later cycle may be partially or totally different from characteristics for selection conditions in an earlier cycle.

The prediction target sequence generator PAd generates prediction target sequence information. At this time, the prediction target sequence generator PAd generates part of prediction target sequence information on the basis of information acquired from the mutation information storage 327c. This method is the same as the method as in the fourth embodiment, and hence description is omitted here.

The prediction target sequence generator PAd may associate each piece of prediction target sequence information generated with the date and time of generation or cycle number when the piece of prediction target sequence information was generated. In this case, the output processor 336c or the user terminal 10 can output in the order of generation of pieces of prediction target sequence information or the order of cycles with generation thereof, or per cycle. The output processor 336c or the user terminal 10 may classify pieces of prediction target sequence information by the date of generation or cycle number and output the pieces of prediction target sequence information in modes different among classes. For example, the user terminal 10 displays pieces of prediction target sequence information generated in the latest cycle with addition of a character string or image (e.g., "NEW") indicating being new.

The prediction target sequence generator PAd may generate, as prediction target sequences, sequences that are not included in previous cycles. Here, an example using Bayesian optimization will be described.

Bayesian optimization is a technique to determine the maximum value of a function of unknown shape (e.g., the affinity of an antibody in this case). Input in training is an antibody sequence and affinity, and an acquisition function is outputted when a new virtual sequence is inputted as test data after training. The acquisition function is, for example, the maximum value of the affinity range of the sequence expected with consideration of uncertainty inferred from the data before and at the current time point. Examples of possible acquisition functions include the upper confidence bound and the expected improvement. Sequences with a high acquisition function can be selected and proposed through experiment. Saito et al., ACS Synth Biol. 2018 Sep 21;7(9):2014-2022. is an example, though this is not for antibodies. Examples of available algorithms include GP-UCB and Thompson sampling.

As described above, in the information processing system 1d according to the present embodiment, the output processor 336c (an example of the "output") outputs at least one piece of prediction target sequence information (candidate antibody information) among a plurality of pieces of prediction target sequence information inputted into the selected trained model (an example of the "second trained model") according to prediction scores.

On the basis of prediction target sequence information outputted by the output processor 336c, additional characterization is performed for antibodies represented by the prediction target sequence information, and the analysis result information is stored as learning datasets. The sequence learner PA2d (an example of the "sequence learner") performs further machine learning on the basis of the prediction target sequence information outputted by the output processor 336c, and thereby generates a new version of the model trained with sequence learning.

The learner 334d (an example of the "learner") performs further machine learning on the basis of the prediction target sequence information outputted by the output processor 336c, and binding determination information on antigen-binding molecules represented by the prediction target sequence information (an example of "evaluation result information on characterization"), and thereby generates a selected trained model (or a model trained with learning of characteristic prediction: the "second trained model").

Thereby, the information processing system 1d can perform further machine learning with use of prediction target sequence information with high characteristics and binding determination information therefor. In some cases, the information processing system 1d can increase the proportion or number of pieces of sequence information with strong bindability as learning datasets. In this case, the information processing system 1 can generate virtual sequences with higher characteristics.

With setting of mutation positions, the information processing system 1d can generate sequence information on virtual sequences having higher characteristics from sequence information obtained by mutating amino acids at the mutation positions. In this case, the information processing system 1d can converge sequence information obtained by mutating amino acids at mutation positions into sequence information on virtual sequences having higher characteristics, in some cases.

(Hardware Configuration)

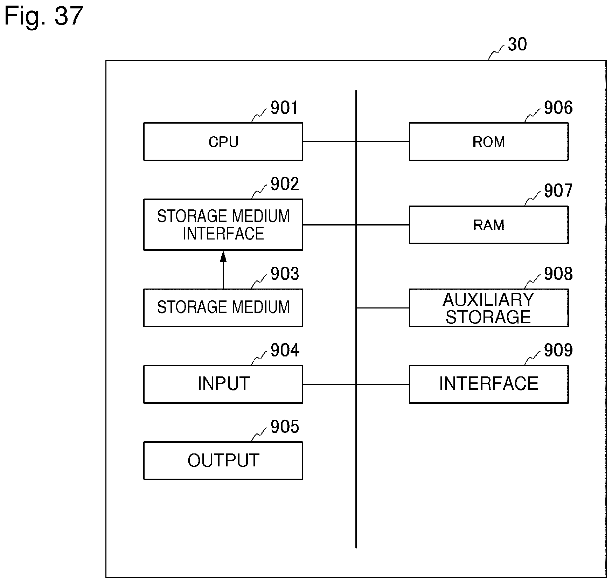

Figure 37 shows a block diagram illustrating an example of the hardware configuration of the server 30 according to the embodiments.

The server 30 includes a CPU 901, a storage medium interface 902, a storage medium 903, an input 904, an output 905, a ROM 906, a RAM 907, an auxiliary storage 908, and an interface 909. The CPU 901, storage medium interface 902, storage medium 903, input 904, output 905, ROM 906, RAM 907, auxiliary storage 908, and interface 909 are connected to each other via a bus.

The CPU 901 mentioned here refers to a processor in a broad sense, and the term includes not only a device what is called a CPU in a narrow sense but also a GPU, a DSP, and so on. Implementation of the CPU 901 mentioned here is not limited to implementation with one processor, and the CPU 901 may be implemented by combining identical processors or different types of processors.

The CPU 901 reads out and executes programs stored in the auxiliary storage 908, ROM 906, and RAM 907, and reads out various data stored in the auxiliary storage 908, ROM 906, and RAM 907 and writes various data into the auxiliary storage 908 and RAM 907, thereby controlling the server 30. The CPU 901 reads out various data stored in the storage medium 903 and writes various data into the storage medium 903 via the storage medium interface 902. The storage medium 903 is a portable storage medium such as a magneto-optical disk, a flexible disk, and a flush memory, and stores various data.

The storage medium interface 902 is an interface to read out from and write into the storage medium 903.

The input 904 is an input device such as a mouse, a keyboard, a touch panel, a volume control button, a power button, a setting button, and an infrared-receiver.

The output 905 is an output device such as a display and a speaker.

The ROM 906 and RAM 907 store programs to operate functional units of the server 30 and various data.

The auxiliary storage 908 is a hard disk drive, a flush memory, or the like, and stores programs to operate functional units of the server 30 and various data.

The interface 909 has a communication interface, and is connected to the network NW through wireless communication or wired communication.

For example, the processor 33 in the functional configuration of the server 30 in Figure 6 corresponds to the CPU 901 in the hardware configuration illustrated in Figure 35. For example, the storage 32 in the functional configuration of the server 30 in Figure 6 corresponds to the ROM 906, RAM 907, or auxiliary storage 908, or any combination of them in the hardware configuration illustrated in Figure 35. For example, the communicator 31 in the functional configuration of the server 30 in Figure 6 corresponds to the interface 909 in the hardware configuration illustrated in Figure 35.

The user terminal 10 and next-generation sequencer 20 also include the same hardware configurations, and hence description of the hardware configurations of the user terminal 10 and next-generation sequencer 20 is omitted here.

In the above-described first to third embodiments, an example in which the server 30 (30a, 30b) outputs candidate antibody information representing candidate antibodies having affinity with the target antigen has been described. The server 30 (30a, 30b) may output experiment conditions under which the best evaluation result information can be obtained for an antibody having a certain sequence based on the information stored through the above-described method. The server 30 (30a, 30b) obtains a learning model through the above-described method in each of rounds under different experiment conditions. Then, the server 30 (30a, 30b) acquires experiment conditions as learning datasets, too. The server 30 (30a, 30b) associates experiment conditions, a learning model, and evaluation result information for each sequence. On the basis of the associated information, the server 30 (30a, 30b) performs learning with use of an existing learning model. At that time, the server 30 (30a, 30b) sets the learning model and evaluation result information as independent variables, and sets the experiment conditions as dependent variables. On the basis of learning results and inputted sequence information, the server 30 (30a, 30b) determines experiment conditions under which the best evaluation result information is outputted, and outputs the information.

Thereby, experiment conditions in performing panning can be optimized, and the character of antibodies evaluated to have higher affinity can be clarified to a higher degree. In this way, the information processing system 1 (1a, 1b) can achieve reduced processing time or processing loads. Accordingly, the information processing system 1 (1a, 1b) can provide desired antibody information.

In the above-described first to third embodiments, an example in which prediction target sequences are generated on the basis of sequence information on binding sequences and mutation information has been described, but the method for generating prediction target sequences is not limited to this. For example, prediction target sequences may be generated on the basis of sequence information on binding sequences. At that time, the prediction target sequence generator PA (also PAb, PAc, and PAd; the same is applied hereinafter) determines probabilities of appearance of amino acids at respective positions in binding sequences. The prediction target sequence generator PA determines information on amino acids whose probabilities of appearance are equal to or higher than a threshold at each position. The prediction target sequence generator PA assigns any of the amino acids at each position to generate prediction target sequences.

In the above-described first to third embodiments, an example in which learning is performed with use of learning datasets acquired from a plurality of operations of panning, but the present disclosure is not limited to this. Learning may be performed with use of learning datasets acquired from one operation of panning. In this case, classification criteria information is a threshold for appearance frequency in the operation of panning.

In the above-described first to third embodiments, an example in which the next-generation sequencer 20 separately outputs sequence information on antibody heavy chains and sequence information on antibody light chains, and the server 30 estimates combinations of an antibody heavy chain and an antibody light chain has been described, but the present invention is not limited to this. If the next-generation sequencer 20 can acquire sequence information including antibody heavy chains and light chains, for example, combinations of an antibody heavy chain and an antibody light chain have been determined, and thus the above-described combination estimation process is not performed.

In the above-described first to third embodiments, the dataset storage 322 may be ordered to store heavy chain sequences (or light chain sequences) as existing antibody sequences. For example, the server 30 may order the dataset storage 322 to store heavy chain sequences (or light chain sequences) as existing antibody sequences. In this case, the server 30 performs a learning process on the basis of heavy chain sequences (or light chain sequences), and thereby generates a model trained with sequence learning. The server 30 performs a learning process on the basis of probabilities of appearance, and thereby generates a model trained with learning of characteristic prediction.

The server 30 generates prediction target sequence information representing heavy chain sequences (or light chain sequences) with use of the model trained with sequence learning. The server 30 inputs each piece of prediction target sequence information generated into the model trained with learning of characteristic prediction, and gives a prediction score for each piece of prediction target sequence information representing heavy chain sequences (or light chain sequences).

On the other hand, the server 30 estimates a light chain sequence (or a heavy chain sequence) to be combined with each heavy chain sequence (or each light chain sequence) represented by prediction target sequence information. Estimation may be carried out with the above-described technique performed by the estimator 332 or another technique. A user may select a light chain sequence (or a heavy chain sequence) to be combined with each heavy chain sequence (or each light chain sequence). The server 30 may generate candidate antibody information by combining each heavy chain sequence (or each light chain sequence) represented by prediction target sequence information and a light chain sequence (or a heavy chain sequence) estimated to be combined with the heavy chain sequence (or the light chain sequence).

In the above-described first to third embodiments, an example in which learning is performed on the basis of all the sequences acquired from the next-generation sequencer 20 in each operation of panning, but the present disclosure is not limited to this. For example, on the basis of acquired sequence information, the sequence information is classified into a plurality of clusters, for each of which learning may be performed.

In the above-described first to third embodiments, evaluation by the information processing system using panning has been described as an example of evaluation of affinity. However, the present invention is not limited to this, and any evaluation of affinity may be acceptable that evaluates affinity between a target antigen and antibodies, and evaluation other than panning may be used.

In the above-described first to third embodiments, the case in which the information processing system uses appearance frequencies of antibodies as evaluation result information has been described as an example. However, the present invention is not limited to this, and appearance frequencies of sequences for each operation of panning, appearance frequencies of amino acids at respective positions for each panning, and so on are acceptable as evaluation result information.

In the above-described first to third embodiments, an example in which the sequence selector PA1 (also PA1b, PA1c, and PA1d; hereinafter, PA1 represents them) and the learner 334 (also 334b, 334c, and 334d; hereinafter, 334 represents them) determines selected classification criteria information and a selected trained model on the basis of AUCs calculated in performing an evaluation process, but the present disclosure is not limited to this. For example, determination may be made on the basis of the correlation between the dissociation constant (KD) and affinity information. Specifically, sequences with KD known in advance are first prepared as evaluation datasets (KD known is also referred to as "known KD"). The storage 32 (32a, 32b) stores relationship information between affinity information and KD. The sequence selector PA1 and the learner 334 calculate affinity information for sequences with use of evaluation datasets. The sequence selector PA1 and the learner 334 convert affinity information into KD (converted KD is also referred to as "calculated KD") on the basis of the calculated affinity information and the relationship information between the affinity information and KD. The sequence selector PA1 and the learner 334 calculate the correlation coefficient for a combination between calculated KD and known KD for each trained model. The sequence selector PA1 and the learner 334 associate classification criteria candidate information with the highest correlation coefficient and a trained model corresponding to the classification criteria candidate information with a panning group ID, and order the learning result storage 326 to store resultants as selected classification criteria information and a selected trained model.

In the above embodiments, examples in which the sequence selector PA1, the sequence learner PA2, and the learner 334 perform a learning process with use of an LSTM or a hidden Markov model have been described, but the present disclosure is not limited to this. For example, the sequence selector PA1, the sequence learner PA2, and the learner 334 may use a random forest described in the fourth and the fifth embodiments.

For example, the virtual sequence generator PA3 can quickly generate many sequences if the sequence learner PA2 uses a learning model with unsupervised learning. Specifically, the sequence learner PA2 generates a model trained with sequence learning to classify sequence information on the basis of characteristic values with use of unsupervised learning. The virtual sequence generator PA3 can generate prediction target sequences having the same characteristic by generating sequences belonging to the same category as a certain sequence belongs.

In the case of a supervised model, the sequence learner PA2 performs machine learning with datasets including sequence information and results of characterization. In this case, the virtual sequence generator PA3 inputs sequence information generated into a supervised model, and selects sequence information with high characterization values outputted from the supervised model as prediction target sequence information. Here, the virtual sequence generator PA3 may, for example, randomly generate amino acids as constituent elements and generate sequence information representing sequences obtained by aligning the generated amino acids as sequence information into the supervised model. The sequence information with high characterization values may be sequence information with characterization values being higher than a threshold, or sequence information with higher characterization values in top ranks.

In the above-described fourth and fifth embodiment, an example in which the server 30c (30d) performs learning and prediction by handling sequence information as character strings in each of which characters representing amino acids of an antibody are aligned has been described, but sequence information is not limited to this. For example, the server 30c (30d) may convert the amino acid sequence of an antibody into a collection of physical property values of individual amino acids constituting the sequence and handle the collection. Specifically, the server 30c (30d) receives character strings of amino acid sequences from the user terminal 10c. The server 30c (30d) converts them into physical property values on the basis of information in which a character string is associated with physical property values.

Here, physical property values of amino acids are numeric values indicating physicochemical or biochemical characteristics of amino acids, and are, for example, characteristic values registered in AAindex. Specifically, physical property values of an amino acid are the volume of the amino acid, the number of atoms, the length of the side chain, the surface area, the electric charge, the degree of hydrophobicity, the region (inside or surface) that frequently appears in proteins, the type of secondary structure often employed, the angle in forming a $\beta$ strand, the change in energy in dissolving in water, the melting point, the heat capacity, the NMR data, and so on. The server 30c (30d) acquires a predetermined combination of physical property values (also referred to as a "group of position physical property values")

for each amino acid as sequence information. That is, the sequence information is information in which position physical property values for each amino acid constituting each antibody are integrated (groups of physical property values).

Information on the combination of physical property values (information indicating what combination of physical property values is used) may be stored in advance in the server 30c (30d), or be information inputted by the user terminal 10c and stored by the server 30c (30d). The combination of physical property values may differ among characteristics.

Information on physical property values corresponding to each amino acid constituting amino acid sequences is not needed to be stored in the server 30c (30d). For example, such information may be stored in the user terminal 10c, and amino acid sequences may be converted in advance into groups of physical property values. In this case, the server 30c (30d) receives groups of physical property values after conversion as sequence information. The information on physical property values corresponding to each amino acid constituting amino acid sequences may be acquired from the network NW by the server 30c (30d) or the user terminal 10c.

The server 30c (30d) may reduce amino acid sequences by using a predetermined method before converting the amino acid sequences into physical property values. The predetermined method is, for example, a method using an autocorrelation function.

Also when the controller 335c predicts characteristic scores of prediction target sequences, the server 30c (30d) temporarily converts amino acid sequences into characteristic quantities. The controller 335c predicts characteristic scores on the basis of the characteristic quantities after conversion and learning results.

The server 30c (30d) may estimate the three-dimensional structure from each amino acid sequence and use information based on the estimated three-dimensional structures (also referred to as "structure information") as sequence information. The structure information is information representing hydrophobic regions, positively charged regions, and negatively charged regions. Such information may be three-dimensionally expressed or two-dimensionally expressed by projection.

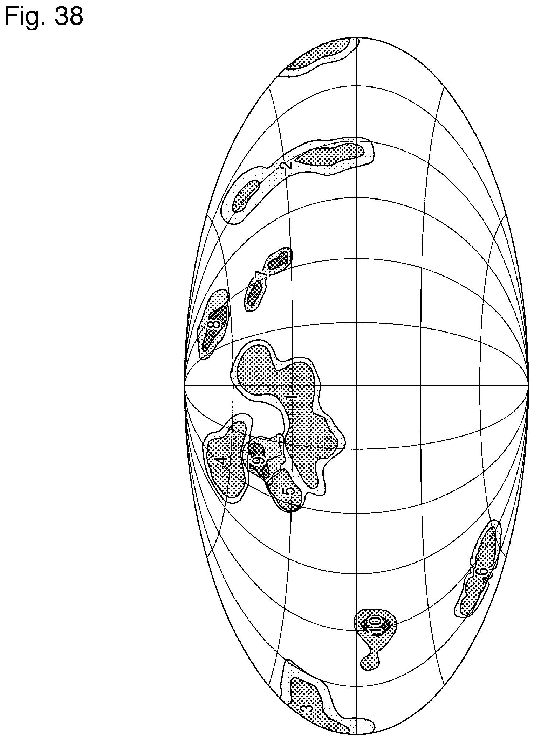

Figure 38 shows a diagram illustrating an example of structure information.

The example illustrated in the diagram is a spherical projection map of the properties of the surface of an antibody molecule (hydrophobic regions, positively charged regions, negatively charged regions) on the basis of results of analytical calculation of the three-dimensional structure of an antibody. The center is a binding surface to an antigen. In the diagram, the regions 1 to 6 each indicate that the surface is a hydrophobic region. The regions 7 to 9 each indicate that the surface is a positively charged region. The region 10 indicates that the surface is a negatively charged region.

The server 30c (30d) extracts feature values from structure information on amino acids by using a predetermined method. The feature values are information representing positions of properties of surfaces of antibody molecules, sizes of regions, and so on. The server 30c (30d) performs learning on the basis of the extracted feature values and characteristic information. Estimation of structure information may be carried out in advance by the user terminal 10c. Alternatively, a configuration in which sequence information is sent to the network NW and the user terminal 10c or the server 30c receives the corresponding structure information from the network NW may be used.

In the above-described fourth and fifth embodiments, an example in which characteristic information on characteristics against an antigen has been used, but characteristic information is not limited to this. For example, characteristic information on characteristics against another antigen may be used. This characteristic information is characteristic information on physical properties of antibodies.

In the above-described fourth and fifth embodiments, no limitation has been set for characteristics (characteristic information) to be used in learning and prediction, but limitation may be set to characteristics (characteristic information) to be used. For example, such information may be inputted a user of the user terminal 10c and sent to the server 30c (30d).

In the above-described fourth and fifth embodiments, an example in which a trained model is produced through learning for each characteristic has been described, but the present disclosure is not limited to this. For example, one trained model may be produced through learning for a plurality of characteristics at once. In this case, characterization information on a plurality of characteristics is outputted from the trained model.

In the above-described embodiments, an example in which the prediction target sequence generator PA generates prediction target sequences with use of an LSTM has been described, but the present disclosure is not limited to this. For example, the prediction target sequence generator PA may specify positions to which mutations are introduced on the basis of acquired sequence information. In the following, this method will be described.

In the above-described first to third embodiments, the prediction target sequence generator PA reads out learning datasets with the binding determination being "binding" from learning datasets (Figure 11) associated with selected classification criteria information. The prediction target sequence generator PA generates prediction target sequence information from the sequence information in the learning datasets read out by changing amino acids at one or more positions. However, the present invention is not limited to this, and the prediction target sequence generator PA may randomly generate prediction target sequence information.

If mutation information is stored in the mutation information storage 327, the prediction target sequence generator PA generates prediction target sequence information from the sequence information in the learning datasets read out by changing amino acids at positions represented by the mutation information (elements of the sequence information).

Thereby, the information processing system 1 can generate prediction target sequence information in which only amino acids that are likely to bind and present at positions intended to mutate have been changed.

The sequence generator PA orders the sequence storage 328 to store the prediction target sequence information generated.

In the above-described fourth and fifth embodiments, the sequence selector PAlc (PA1d) and the sequence learner PA2c (PA2d) acquire sequence information with sequences represented by improved characteristic information from sequence information stored in the learning dataset storage 324c (324d). The sequence selector PA1, the sequence learner PA2, and the learner 334 compare each amino acid sequence in the acquired sequence information and the amino acid sequence in template sequence information, and specify mutation positions in the amino acid sequence. The sequence selector PA1, the sequence learner PA2, and the learner 334 order the mutation information storage 327c to store mutation position information representing specified mutation positions as one type of mutation information.

The prediction target sequence generator PAc (PAd) generates prediction target sequence information on the basis of template sequence information and mutation information. For example, the sequence selector PAlc (PA1d) reads out template sequence information, mutation position information, and mutation condition information from the mutation information storage 327c. The sequence selector PAlc (PA1d) determines whether the number of mutation positions represented by the mutation position information is larger than the upper limit number of mutations represented by the mutation condition information. If the number of mutation positions is equal to or smaller than the upper limit number, the sequence selector PAlc (PA1d) determines that all the mutation positions are points to be mutated. If the number of mutation positions is larger than the upper limit number, the sequence selector PAc (PA1d)1 randomly selects upper limit number of positions to be mutated from mutation positions. The sequence selector PAlc (PA1d) generates prediction target sequence information from template sequence information by changing amino acids specified by the positions to be mutated. The sequence selector PAlc (PA1d) orders the sequence storage 328c to store the generated prediction target sequence information.

In the described method, the case in which the server 30c determines places to be mutated has been described, but the present disclosure is not limited to this. For example, mutation information representing specific positions to be mutated may be inputted by a user of the user terminal 10c and sent to the server 30c (30d). In this case, the server 30c (30d) stores the received mutation information in the mutation information storage 327c. So as to include the positions to be mutated, the server 30c (30d) determines positions to be mutated.

In the above-described fourth and fifth embodiments, an example in which in outputting candidate antibody information, the server 30c (30d) determines candidate antibody information on the basis of ordering for each characteristic has been described, but output of candidate antibody information is not limited to this. For example, the user terminal 10c may perform determination of candidate antibody information with use of a plurality of characteristics. In this case, the server 30c sends results of ordering for respective characteristics (prediction target sequences and results of ordering for respective characteristics) to the user terminal 10c.

In the above-described fourth and fifth embodiments, an example in which the server 30c (30d) generates prediction target sequences has been described, but the present disclosure is not limited to this. For example, prediction target sequences may be inputted by a user of the user terminal 10c and sent to the server 30c (30d).

In the above-described fourth and fifth embodiments, an example in which in learning of an LSTM, the server 30c (30d) stores in advance parameters associated with the entire of the intermediate layer and the server 30c (30d) appropriately changes the parameters in re-learning has been described, but the present disclosure is not limited to this. For example, parameters may be inputted by a user of the user terminal 10c. In this case, for example, the server 30c (30d) sends information for demanding parameters associated with the entire of the intermediate layer, as necessary, to the user terminal 10c. The user terminal 10c displays the received information on the display 15, and sends information inputted by a user of the user terminal 10c to the server 30c (30d). The server 30c (30d) determines the parameters on the basis of the received information.

In the above-described fourth and fifth embodiments, an example in which sequence information and characteristic information to be used for learning datasets are determined on the basis of cycle number, but the present disclosure is not limited to this. For example, sequence information and characteristic information with superior characteristics may be preferentially used for learning datasets. In this case, the server 30c stores information representing characteristics of concern in the storage 32c, or receives the information from the user terminal 10c.

For example, the server 30c may determine sequence information and characteristic information to be used for learning datasets on the basis of information representing date and time when sequence information and characteristic information has been acquired. Specifically, the server 30c acquires pieces of sequence information and characteristic information to be used for learning datasets in order from the latest date and time of acquisition until a predetermined upper limit number is reached. Here, date and time of acquisition is date and time when the server 30c acquired a piece of information, or date and time when the server 30c acquired a piece of characteristic information through actual measurement or the like.

For example, the server 30c may perform learning on the basis of all the pieces of sequence information and characteristic information acquired before the learning. At that time, the server 30c may perform weighing depending on cycle number. Specifically, pieces of sequence information generated in later cycles and the corresponding pieces of characteristic information may be regarded as being more important.

In the above-described embodiments, an example using antibodies, as an example of antigen-binding molecules, has been described, but the antigen-binding molecules are not limited to this. That is, the antigen-binding molecule is used in the broadest sense. Specifically, the antigen-binding molecule includes various molecular types that exhibit binding activity against an antigen. If the antigen-binding molecule is a molecule in which an antigen-binding domain and an Fc region are bonded together, for example, examples thereof include complete antibodies and antibody fragments. Antibodies can include single monoclonal antibodies (including agonist and antagonist antibodies), human antibodies, humanized antibodies, and chimeric antibodies. Preferred examples in using a fragment of an antibody include antigen-binding domains and antigen-binding fragments (e.g., VHH, Fab, F(ab')2, scFv, and Fv). The antigen-binding molecule in the present disclosure can include scaffold molecules formed as a library for construction of antigen-binding domains by using only a partial structure of three-dimensional structure, as a scaffold, such as existing stable $\alpha\beta$/ barrel protein structure.

In the above-described first to third embodiments, an example in which the user terminal 10, the next-generation sequencer 20, and the server 30 are connected together via the network NW has been described, but the present disclosure is not limited to this. For example, the server 30 and the next-generation sequencer 20 may be integrated together. The user terminal 10 and the server 30 may be integrated together. Although an example in which the server 30 converts nucleotide sequences acquired from the next-generation sequencer into amino acid sequences has been described, the present disclosure is not limited to this. For example, a converter configured to perform a process to convert nucleotide sequences into amino acid sequences may be present outside of the server 30. In this case, the converter converts nucleotide sequences in information inputted from the next-generation sequencer 20 into amino acid sequences, and outputs the information after conversion to the server 30. The next-generation sequencer 20 is not limited to a next-generation sequencer. For example, the next-generation sequencer 20 may be another sequencer.

In the above-described fourth and fifth embodiments, an example in which the user terminal 10c and the server 30c (30d) are connected together via the network NW has been described, but the present disclosure is not limited to this. For example, the server 30c and the user terminal 10c may be integrated together. For example, a device configured to perform the learning process by the server 30c (30d) may be provided outside of the server 30c (30d). For example, a device configured to perform the prediction process by the server 30c (30d) may be provided outside of the server 30c (30d).

In the above embodiments, the prediction target sequence generator PA and the learner 334 select one amino acid to generate the fixed vector value $h_{t-1}$ according to probabilities of appearance. For example, the prediction target sequence generator PA and the learner 334 output in such a manner that an amino acid with a probability of appearance of 40% is selected four times every 10 times.

Thereby, the prediction target sequence generator PA and the learner 334 can allow various amino acids to appear, for example, as compared with the case in which one amino acid with the maximum value is selected at each position, and thus can output a wide variety of prediction target sequences or candidate antibody information.

In the above-described embodiments, the prediction target sequence generator PA and the learner 334 may be included in another device. The sequence selector PA1, sequence learner PA2 (also PA2b, PA2c, and PA2d; hereinafter, PA2 represents them) and the virtual sequence generator PA3 (also PA3b, PA3c, and PA3d; hereinafter, PA3 represents them) may be each included in another device.

In the above-described embodiments, the server 30 (also 30a, 30b, 30c, and 30d; hereinafter, 30 represents them) is not needed to include the classifier 333, and the sequence selector PA1 is not needed to generate an LSTM. In this case, the sequence selector PA1 selects sequence information in learning datasets in which the characteristic value satisfies a predetermined condition among learning datasets. The sequence learner PA2 performs a learning process on the basis of the sequence information selected by the sequence selector PA1, and thereby generates a model trained with sequence learning. The server 30 is not needed to include the learner 334b. In this case, some or all of the prediction target sequences generated by the virtual sequence generator PA3 are generated as candidate antibody information in the server 30. The output processor 336 outputs the candidate antibody information generated. The server 30 is not needed to include the estimator 332.

Generating a group of virtual sequences through machine learning and predicting values thereto such as prediction scores in the above embodiments is significant against the presence of sequences that are not successfully expressed in phage display experiment and cause difficulty in binding experiment. This is advantageous in that evaluation for sequences that cause difficulty in experiment becomes feasible by a computer.

Generating a group of virtual sequences with an LSTM in the above embodiments is significant with respect to the fact that simple enumeration causes explosion of the number of combinations, and even high-performance computers occasionally fail to handle it. If 19 types of amino acid are assigned to Hch and Lch, at 20 positions in total, in all possible patterns, for example, there are an extremely huge number of combinations, $19^{20} = 3.76 \times 10^{25}$, and it is very difficult to evaluate all the combinations with a computer. Therefore, it is important in the above embodiments to learn sequences with "a good property" and generate a group of sequences "that are likely to have a good property" with use of an LSTM.

[Examples]

In the following, examples according to the first embodiment will be described. Sequence information was acquired for the case in which a phage display library to an antigen K was subjected to panning. The sequence information is included in analysis result information from analysis with a next-generation sequencer (NGS). Since the sequence information in analysis result information was obtained after panning, the sequence information is sequence information on a group of antibodies that bind to the antigen K.

A learning process was performed for an LSTM with use of the group of sequences represented by the sequence information after panning, and a group of virtual sequences that are likely to bind was generated as prediction target sequences with use of the LSTM after the learning process.

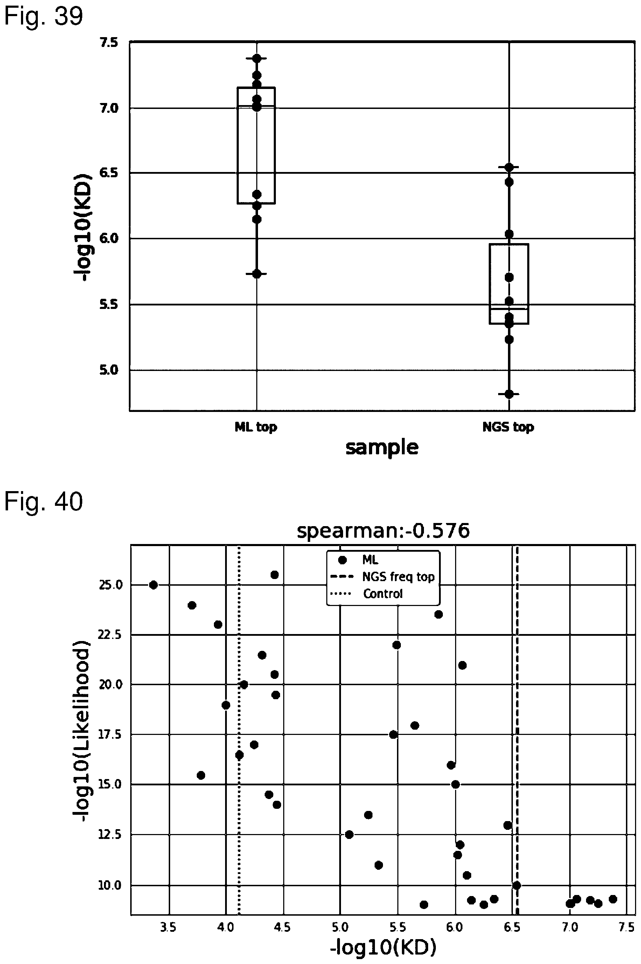

Figure 39 shows a graph representing the relationship between sequences and characteristics according to the present example. In Figure 39, the horizontal axis shows sequence types and the vertical axis shows characteristic values. Characteristic values are negative common logarithms of dissociation constants (KD) (-logio(KD)), which are indicative of affinity.

In Figure 39, the relationship between sequences and characteristic values was plotted for each of the group of sequences of "ML top" and the group of sequences of "NGS top". Among the prediction target sequences generated with use of the LSTM after the learning process, the group of sequences of "ML top" is a group of top 10 sequences in terms of likelihood P (prediction score). The group of sequences of "NGS top" is a group of top 10 sequences in terms of appearance frequency included in the analysis result information from the next-generation sequencer.

In Figure 39, a boxplot is shown for characteristic values (-logio(KD)) in each of the group of sequences of "ML top" and the group of sequences of "NGS top".

Comparison between the group of sequences of "ML top" and the group of sequences of "NGS top" finds that the group of sequences of "ML top" exhibits higher characteristic values than the group of sequences of "NGS top" does. That is, it is understood that prediction target sequences are sequences having stronger binding ability than sequences from analysis by the next-generation sequencer. Thus, the server 30 successfully generated a group of virtual sequences (prediction target sequences) having stronger binding ability than sequences from analysis by the next-generation sequencer 20 (sequences used for the learning process) with use of the LSTM after the learning process. In addition, scoring of predicted antibody sequences based on likelihood P was found to be effective.

Figure 40 shows a graph representing the prediction precision for characteristics of sequences according to the present example. The vertical axis in Figure 40 shows predicted values for affinity, which are negative common logarithms of likelihood P ($-\log_{10}(P)$) (in Figure 40, shown as -logio(likelihood)). The horizontal axis in Figure 40 shows actual measurements of affinity, which are negative common logarithms of dissociation constants (KD) (-logio(KD)).

In Figure 40, predicted values (vertical axis) and actual measurements (horizontal axis) are plotted for some of the prediction target sequences generated with use of the LSTM after the learning process. In this graph, the absolute value of the correlation coefficient between predicted values and actual measurements was 0.576. As demonstrated by the graph, the higher the likelihood P (since being negative common logarithms ($-\log_{10}(P)$), the lower the value in the vertical axis was), the stronger the affinity was (the value in the horizontal axis higher). That is, the information processing system 1 (server 30) achieved high prediction precision in terms of prediction of sequences with strong binding. Thus, sequences having high binding ability can be predicted by generating a group of virtual sequences (prediction target sequences) by using likelihood P as an index.

In Figure 40, "NGS freq top" indicated by a chain line shows the dissociation constant (KD) of the sequence with the highest frequency obtained from the next-generation sequencer after panning. "Control" indicated by a dotted line shows the dissociation constant (KD) of the template sequence to produce the group of sequences for panning. As can be seen from the graph, with use of the LSTM, the information processing system 1 successfully generated sequences with stronger binding than the sequence the most concentrated after panning as prediction target sequences. As shown above, a group of virtual sequences having strong binding ability was successfully generated with the LSTM according to the first embodiment, and scoring of predicted antibody sequences based on likelihood P is effective.

In the following, an example according to the third embodiment will be described.

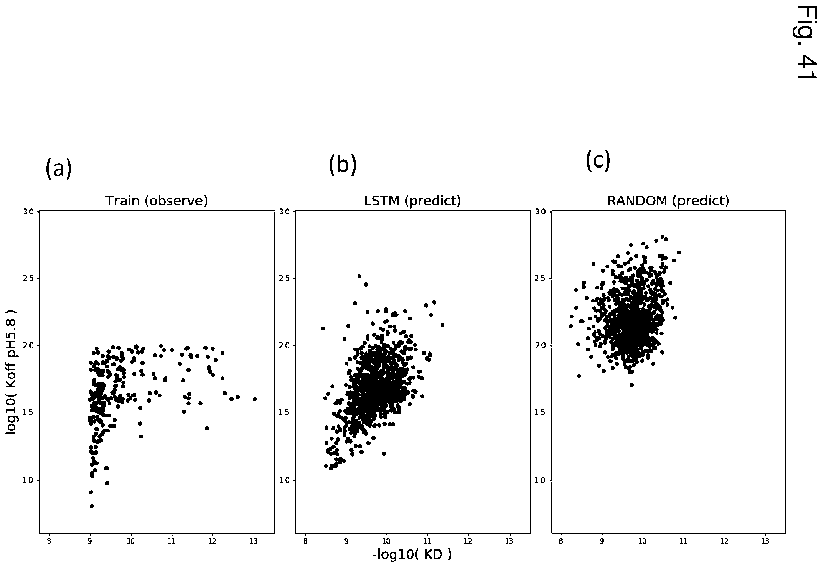

Figure 41 shows plots demonstrating the similarity between training sequences and virtual sequences according to the present example. The training sequences are sequences used for training of the LSTM, which are included in training datasets. The vertical axis in Figure 41 shows negative common logarithms of dissociation rates (acidic koff) when the pH of the reaction solution was acidic, which are common logarithms of dissociation rates when the pH was 5.8 (acidic) (-logio(koff pH 5.8)) in Figure 41. The horizontal axis in Figure 41 shows negative common logarithms ($-\log_{10}(kD)$) of dissociation constants when the pH of the reaction solution was neutral (neutral KD).

Figure 41(a) is a plot of characteristic values (actual measurements) for training sequences. These sequences plotted are 251 sequences that satisfy -logio(KD) > 9 and logio(koff) < 2. Neutral KD and acidic koff are actual measurements.

Figure 41(b) is a plot of characteristic values (predicted values) for sequences generated by the LSTM. This is a plot for a group of 1000 virtual sequences outputted from a trained model after machine learning (LSTM) was performed with use of the training datasets shown in Figure 41(a). Neutral KD and acidic koff are predicted values. Figure 41(c) is a plot for a new group of virtual sequences generated by listing mutated residues contained in the sequences shown in Figure 41(a) and randomly shuffling and combining them. Neutral KD and acidic koff are predicted values.

Comparison between Figure 41(a) and Figure 41(c) finds that the generated sequences in Figure 41(c) were present in a range of acidic koff largely different from that for the training sequences in Figure 41(a). The reason is, for example, thought to be that, if shuffling is performed, combinations of mutations that "improve neutral KD and acidic koff through synergistic effect" are broken and only one mutation is employed. On the other hand, the generated sequences in Figure 41(b) were present in a range of acidic koff similar to that for the training sequences in Figure 41(a), in contrast to the case in Figure 41(c). In this way, the group of virtual sequences (prediction target sequences) obtained by using the LSTM exhibited the nature of the training sequences more intensely than those obtained by changing sequences at mutation positions at random.

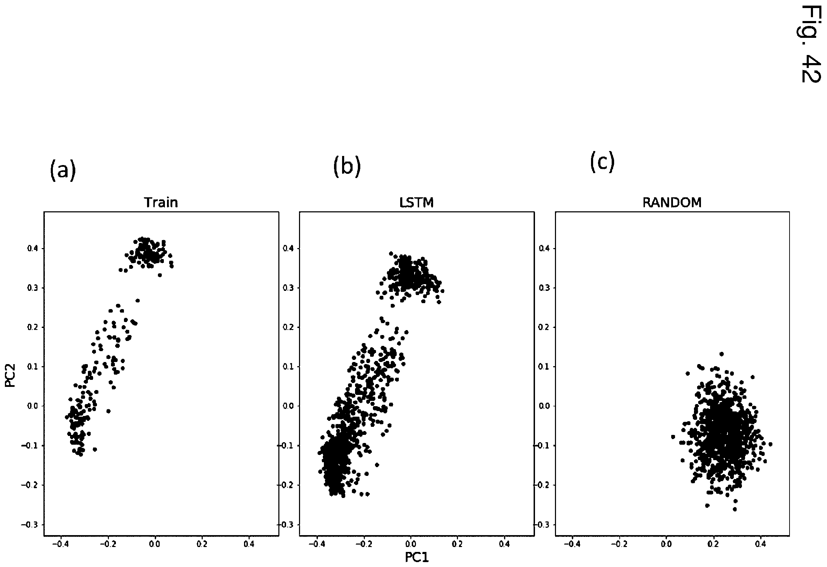

Figure 42 shows other plots demonstrating the similarity between training sequences and virtual sequences according to the present Example. In Figure 42, the horizontal axis shows first principal components in principal component analysis, and the vertical axis shows second principal components. Principal component analysis was carried out by mapping sequences on a vector space by using the Doc2Vec method. The model used here for the Doc2Vec method was a model for which a learning process had been performed with use of sequences from the protein sequence database Uniprot (http://www.uniprot.org/).

Figure 42(a) is a plot of principal component values for training (Train) sequences. These sequences are the same as the sequences plotted in Figure 41(a).

Figure 42(b) is a plot of principal component values for sequences generated by the LSTM. These sequences are the same as the sequences plotted in Figure 41(b).

Figure 42(c) is a plot of principal component values for sequences obtained by changing sequences at mutation positions at random. These sequences are the same as the sequences plotted in Figure 41(c).

Since each numeric vector in Figures 42(a) to Figure 42(c) is a vector representing the characteristic of an actually existing amino acid sequence, amino acid sequences resembling each other are expressed as similar vectors.

Comparison between Figure 42(a) and Figure 42(b) found that sequences in the figures exhibited close values, in contrast to comparison with those in Figure 42(c). In short, it was found that the sequences generated by the LSTM resembled the amino acid sequences used in training. By contrast, comparison between Figure 42(a) and Figure 42(c) found that sequences in the figures exhibited non-close values, in contrast to comparison with those in Figure 42(b). In short, it was found that the sequences generated by the LSTM do not resemble the amino acid sequences used in training. It can be understood from this that the sequences generated by the LSTM resembled the original training sequences with respect to their amino acid sequences, and thus reflected the character of the training sequences.

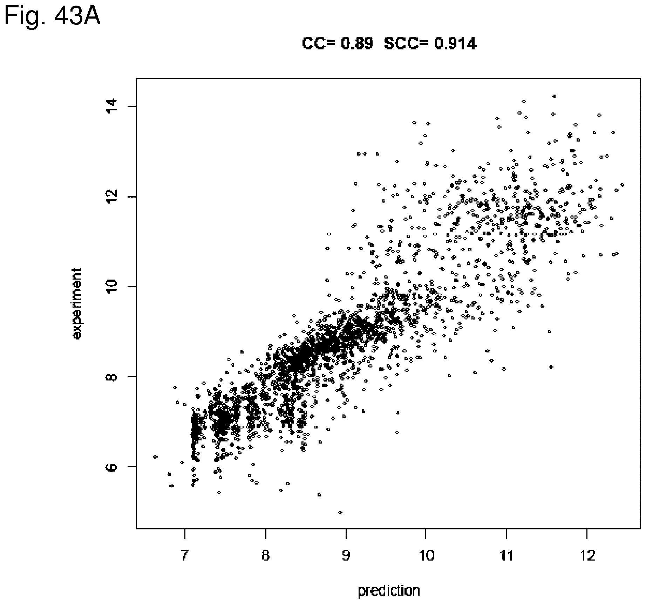

Figure 43A to Figure 43I show plots representing the correlation between predicted values and actual measurements for characteristics of sequences according to the Example. Combinations of mutated sequences and experimental quantities (actual measurements) were listed for each type of experimental quantities, and the resultant was used as learning datasets. The mutated sequences are sequence expected for improvement of binding and physical properties through introducing a few mutations into a template sequence for which antigen binding has been confirmed. For the mutated sequences, data obtained by converting amino acid sequences into character strings were used.

The types of experimental quantities were dissociation constants (KD), dissociation rates (koff), expression levels, monomer% in SEC (size-exclusion chromatography), half widths of monomer peaks in SEC, amounts bound to ECM (extracellular matrix), amounts of an antibody captured in thermal acceleration test, amounts of an antigen bound in thermal acceleration test, and affinity scores.

Monomer% in SEC is the ratio of antibody molecules with the amino acid sequence present as a monomer. Half widths of monomer peaks in SEC are those when amounts of eluted molecules in chromatography are shown in the vertical axis and time (molecular weight) is shown in the horizontal axis. The amount bound to ECM are amounts specifically or non-specifically bound to the extracellular matrix not being a targeted antigen. The amount of an antibody captured in thermal acceleration test is a value that indicates, when samples are stored under a high-temperature condition (50°C) and refrigeration (4°C) for a certain period of time are subjected to surface plasmon resonance (SPR) measurement, the amount of the sample fixed under the high-temperature condition in terms of % of capture molecules on a sensor chip as compared with the sample under refrigeration, and primarily serves as an index of the stability of parts other than antigen-biding sites. The amount of an antigen bound in thermal acceleration test is a value that indicates, when samples are stored under a high-temperature condition (50°C) and refrigeration (4°C) for a certain period of time are subjected to surface plasmon resonance (SPR) measurement, the amount of an antigen bound under the high-temperature condition in terms of % per amount of the sample captured as compared with the sample under refrigeration, and primarily serves as an index of the stability of antigen binding. The affinity score is defined from raw data of analysis using a Biacore as an index of good balance between binding strength at pH 7.4 and the rate of dissociation at pH 5.8. Specifically, the affinity score is obtained from the shape of a sensorgram in the Biacore by summation of three scores of "Score for degree of rapid binding at neutral pH", Score for degree of continuous stable binding at neutral pH", and "Score for degree of occurrence of rapid dissociation at acidic pH" each multiplied by an appropriate coefficient.

Learning datasets were extracted from an antibody database in Chugai Pharmaceutical Co., Ltd. on April 26, 2019. Machine learning using a random forest was performed with use of learning datasets for each type of experimental quantities to generate a trained model. Predicted values for experimental quantities were given by using the trained model generated. Here, training samples and test samples are separately evaluated by the Out of bag method for evaluation of precision. Thus, results in Figure 43A to Figure 43I were obtained.

Figure 43A shows a plot demonstrating the correlation between predicted values and actual measurements for dissociation constants (KD).

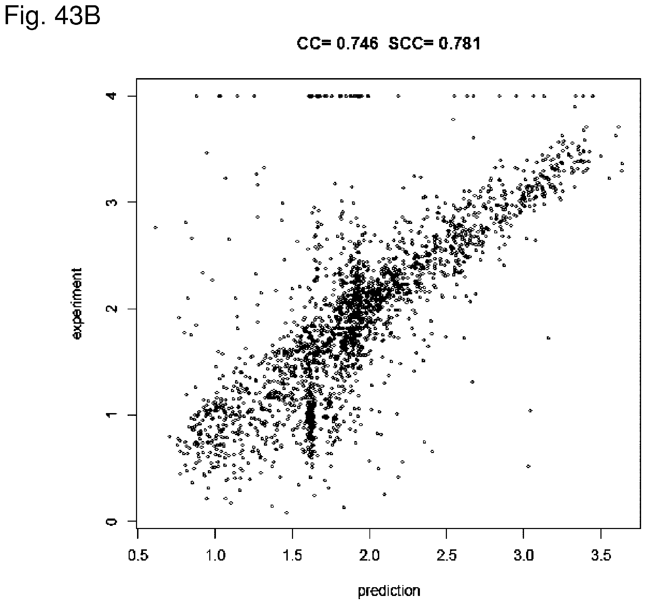

Figure 43B shows a plot demonstrating the correlation between predicted values and actual measurements for dissociation rates (koff).

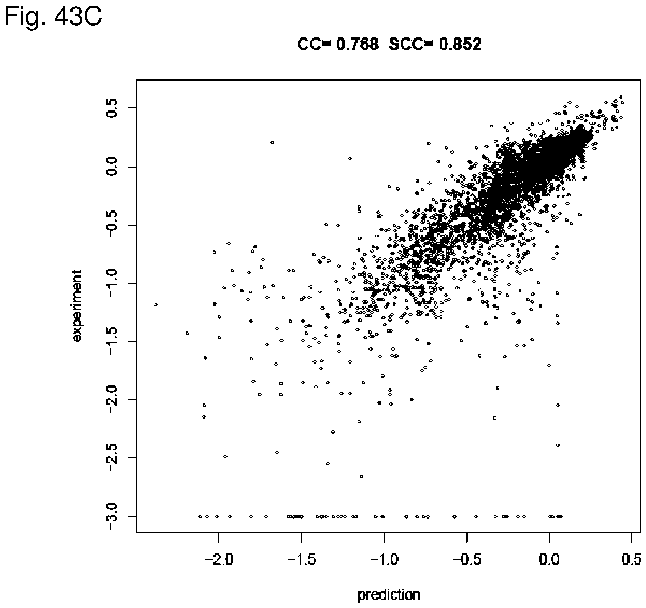

Figure 43C shows a plot demonstrating the correlation between predicted values and actual measurements for expression levels.

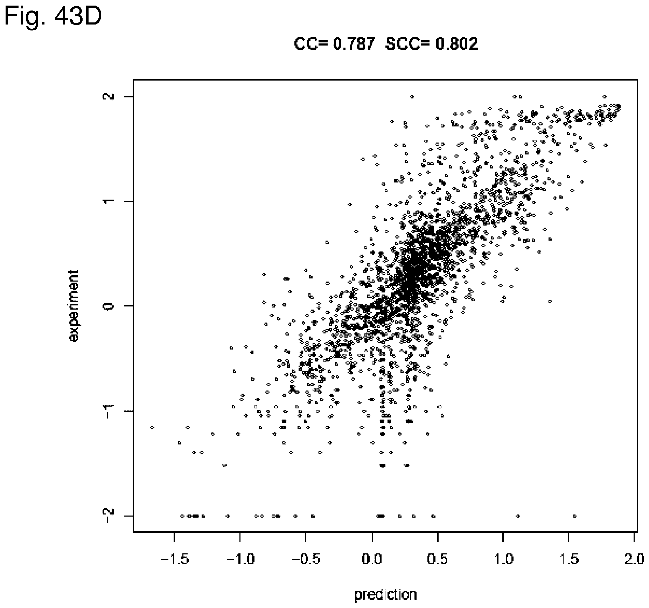

Figure 43D shows a plot demonstrating the correlation between predicted values and actual measurements for monomer % in SEC.

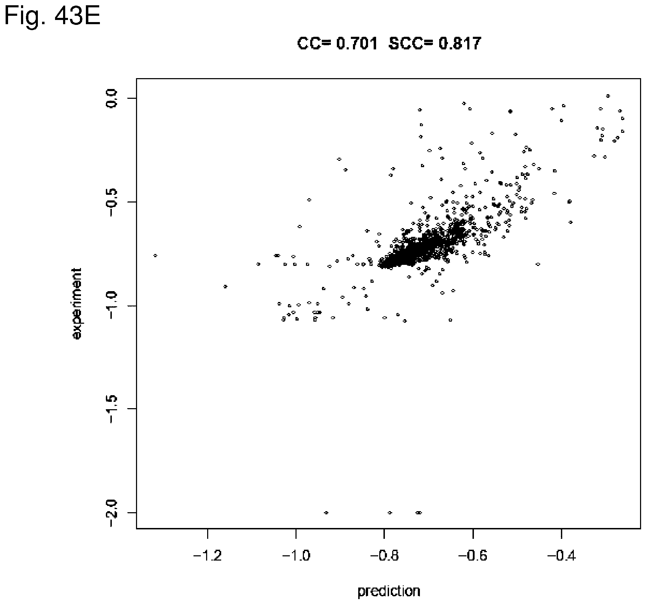

Figure 43E shows a plot demonstrating the correlation between predicted values and actual measurements for half widths of monomer peaks in SEC.

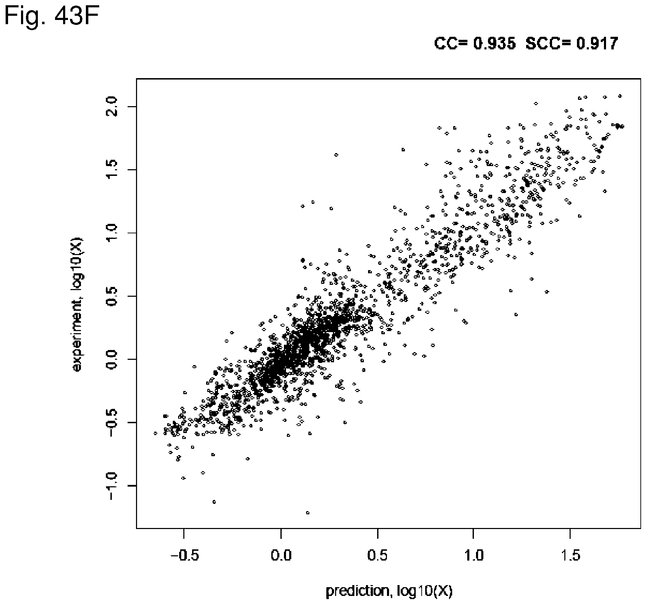

Figure 43F shows a plot demonstrating the correlation between predicted values and actual measurements for amounts bound to the ECM.

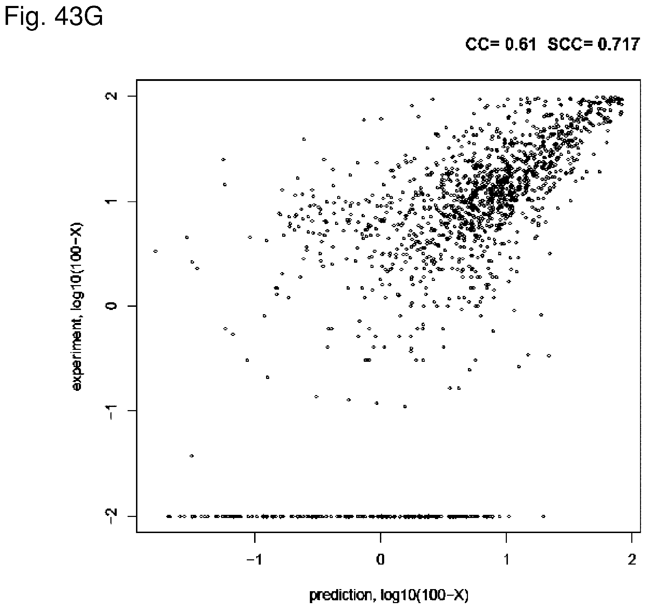

Figure 43G shows a plot demonstrating the correlation between predicted values and actual measurements for amounts of an antibody captured in thermal acceleration test.

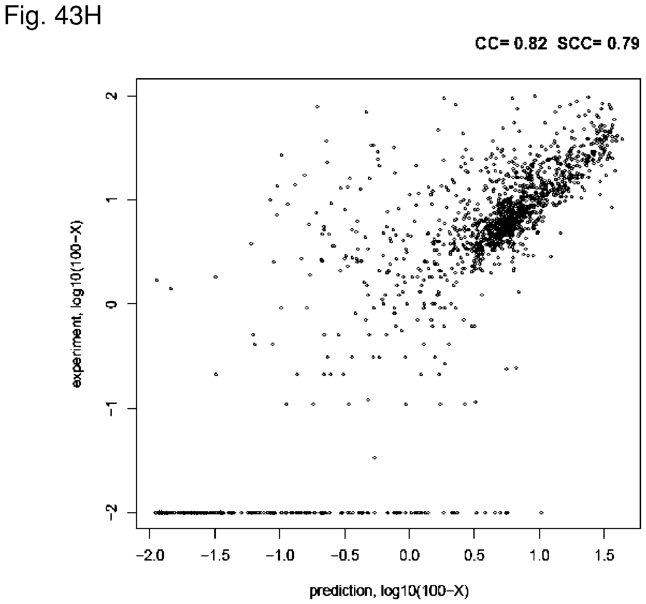

Figure 43H shows a plot demonstrating the correlation between predicted values and actual measurements for amounts of an antigen bound in thermal acceleration test.

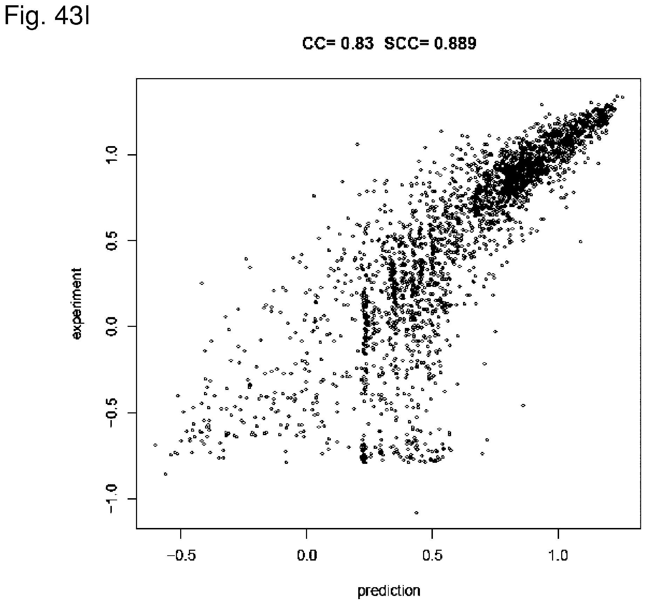

Figure 43I shows a plot demonstrating the correlation between predicted values and actual measurements for affinity scores.

In Figure 43A to Figure 43I, correlation coefficients (CC) and Spearman correlation coefficients were values shown in the plots.

The distributions, correlation coefficients, or selective correlation coefficients in Figure 43A to Figure 43I revealed that there was correlation between predicted values and actual measurements for each characteristic (type of experimental quantities). That is, it was demonstrated that predicted values given by the above embodiment have good precision.

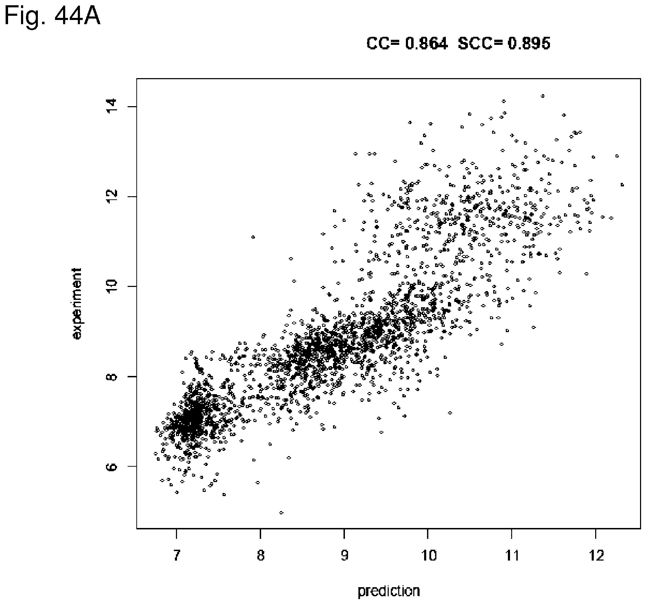

Figure 44A to Figure 44I each show a plot demonstrating the correlation between predicted values and actual measurements for a characteristic of sequences according to another example. In the present example, data obtained by converting amino acid sequences into numeric vectors by the Doc2Vec method were used for mutated sequences. The present example and the example in Figure 43A to Figure 43I are different in whether amino acid sequences of mutated sequences were numeric vectors or character strings.

Figure 44A shows a plot demonstrating the correlation between predicted values and actual measurements for dissociation constants (KD).

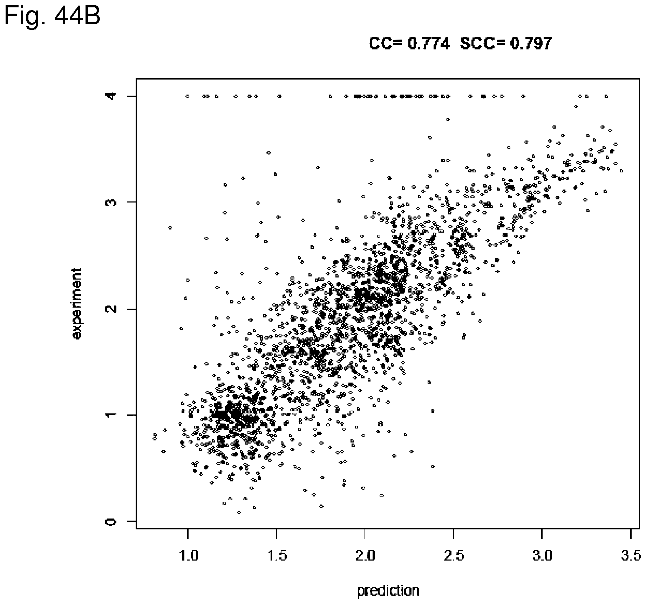

Figure 44B shows a plot demonstrating the correlation between predicted values and actual measurements for dissociation rates (koff).

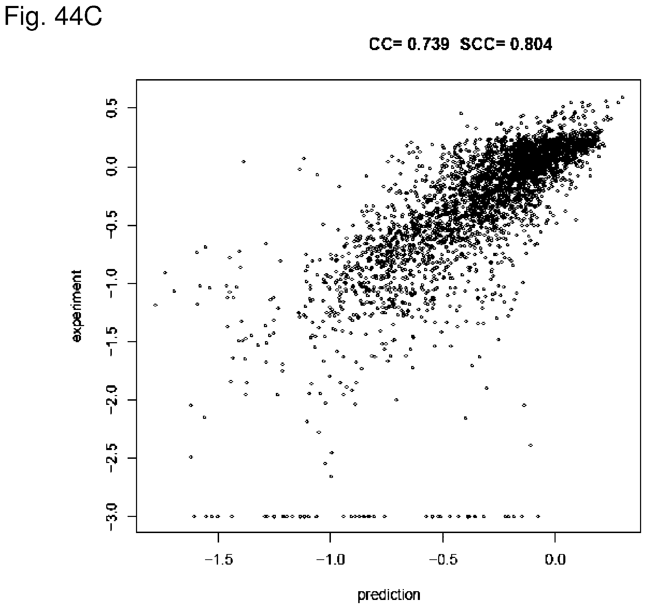

Figure 44C shows a plot demonstrating the correlation between predicted values and actual measurements for expression levels.

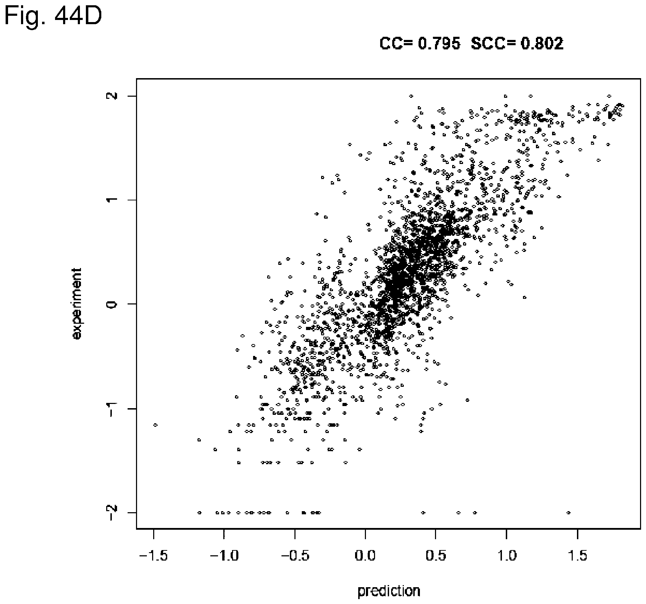

Figure 44D shows a plot demonstrating the correlation between predicted values and actual measurements for monomer % in SEC.

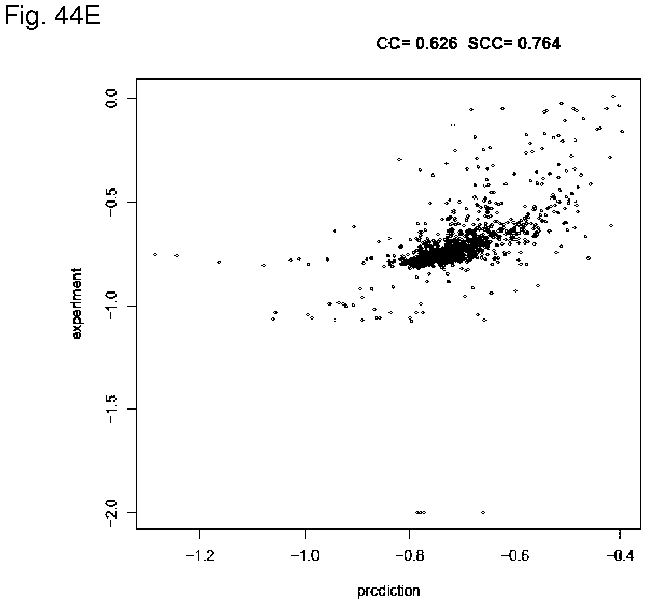

Figure 44E shows a plot demonstrating the correlation between predicted values and actual measurements for half widths of monomer peaks in SEC.

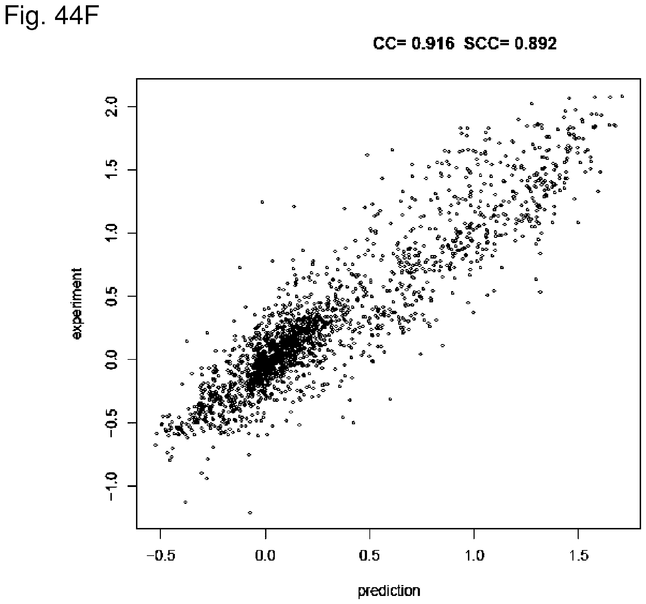

Figure 44F shows a plot demonstrating the correlation between predicted values and actual measurements for amounts bound to the ECM.

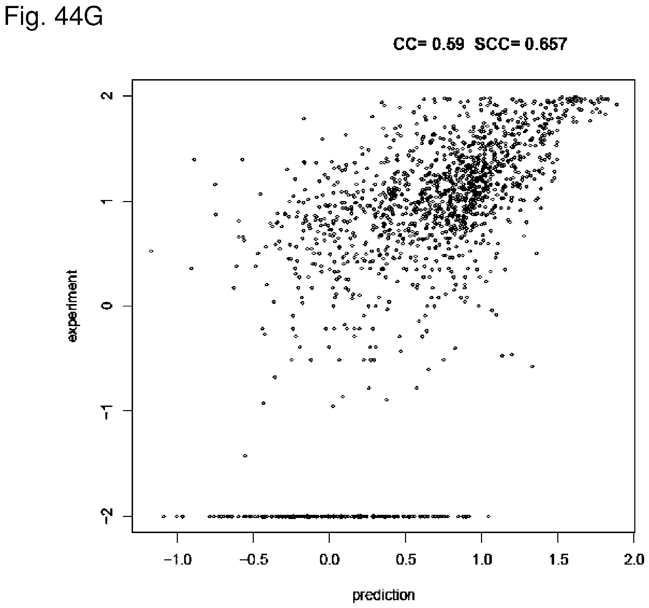

Figure 44G shows a plot demonstrating the correlation between predicted values and actual measurements for amounts of an antibody captured in thermal acceleration test.

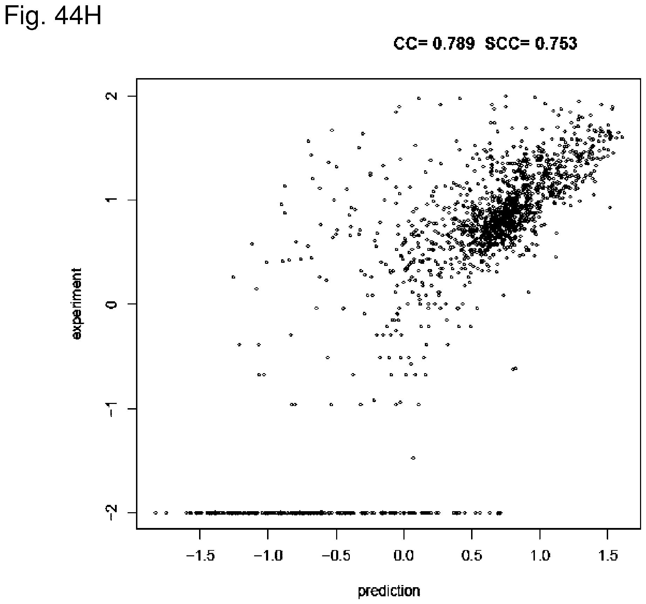

Figure 44H shows a plot demonstrating the correlation between predicted values and actual measurements for amounts of an antigen bound in thermal acceleration test.

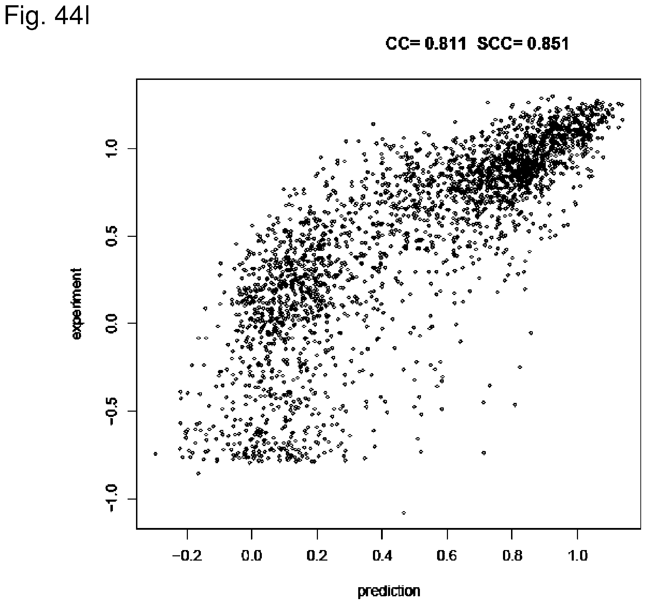

Figure 44I shows a plot demonstrating the correlation between predicted values and actual measurements for

EP 3 982 369 A1

affinity scores.

In Figure 44A to Figure 44I, correlation coefficients (CC) and selective correlation coefficients were values shown in the plots.

The distributions, correlation coefficients, or selective correlation coefficients in Figure 44A to Figure 44I revealed that there was correlation between predicted values and actual measurements for each characteristic (type of experimental quantities). That is, it was demonstrated that predicted values given by the above embodiment have good precision.

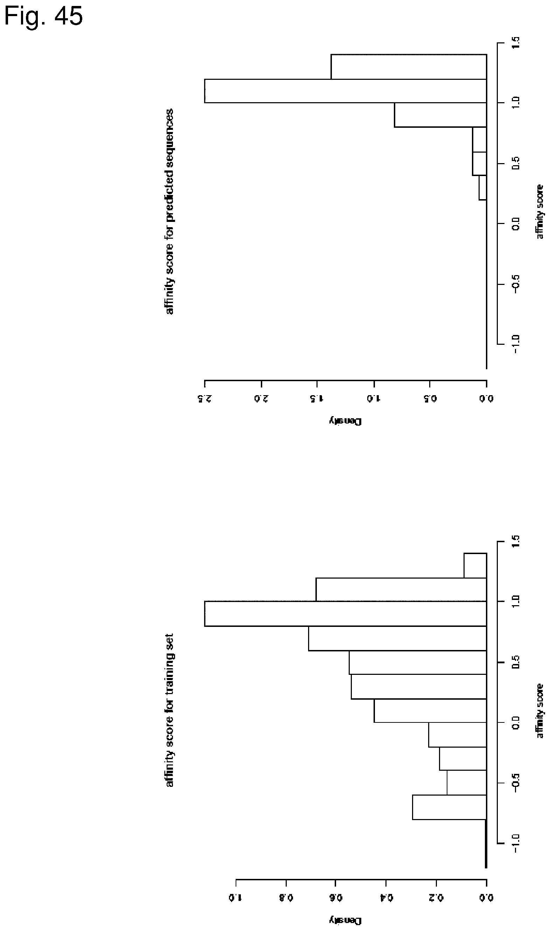

Figure 45 shows graphs for describing an improved characteristic of sequences according to the present example. The characteristic is represented as affinity scores, and larger values indicates strong avidity and smaller values indicates weak avidity. The vertical axis shows the density function (density), which indicates appearance frequency (number of sequences).

In the present Example, a learning process was performed for an LSTM with use of 2,636 combinations of a mutated sequence and an affinity score as learning datasets, and a group of virtual sequences was generated as prediction target sequences with use of the LSTM after the learning process. For the prediction target sequences, 81 sequences having a good predicted value for the affinity score were generated by using the random forest method. These 81 sequences are prediction target sequences having a prediction score equal to or higher than a threshold and information on the sequences is candidate antibody information in the above-described embodiment.

The graph "affinity score for training set" shows the distribution of affinity scores for 2,636 sequences in datasets for learning. On the other hand, the graph "affinity score for predicted sequences" shows the distribution of affinity scores for the generated 81 sequences (among prediction target sequences, sequences having a prediction score equal to or higher than a threshold).

Comparison of these distributions finds that predicted sequences (81 sequences) were more distributed in the high affinity score side than the sequences in the datasets for learning. In short, it is understood that the predicted sequences were improved in distribution of affinity as compared with the sequences in the datasets for learning.

In the above embodiment, sequences having a good predicted value of the affinity score may be generated as prediction target sequences by using the gradient boosting method in place of or in addition to the random forest method. Also in this case, distribution like that in Figure 45 was successfully obtained for sequences generated.

Thus, Figure 45 demonstrates that the LSTM according to the third embodiment succeeded in generating a group of virtual sequences having strong binding ability, and the technique of the present embodiment is effective.

[Supplement]

(1) An aspect of the present invention is an information processing system including: a sequence learner configured to perform machine learning on the basis of sequence information representing sequences including some or all of the sequences of a plurality of antigen-binding molecules and thereby generate a trained model that has learned a character of the sequences; and a sequence generator configured to generate virtual sequence information obtained by mutating at least one of constituent elements constituting a sequence represented by the sequence information on the basis of the trained model.

The constituent elements refer to elements that constitute a sequence and elements that constitute a molecule. When sequence information is sequence information on an amino acid sequence, for example, the constituent elements are amino acids. When sequence information is sequence information on a nucleotide sequence, for example, the constituent elements are nucleotides.

(2) An aspect of the present invention is an information processing system including: a sequence learner configured to perform machine learning on the basis of sequence information representing sequences including some or all of the sequences of a plurality of proteins and thereby generate a trained model that has learned a character of the sequences; and a sequence generator configured to generate virtual sequence information obtained by mutating at least one of constituent elements constituting a sequence represented by the sequence information on the basis of the trained model.

That is, each antigen-binding molecule in each of the above embodiments may be a protein.

(3) In the information processing system as an aspect of the present invention, the character of the sequences is a character including positions of the constituent elements in the sequences and anteroposterior relationship of the constituent elements.

(4) In the information processing system as an aspect of the present invention, the sequence generator generates the virtual sequence information by changing at least one of the constituent elements in preset sites including one or more of the constituent elements in a sequence.

That is, in each of the above embodiments, the server 30 may set sites specified with a plurality of positions of amino

59

acids and change at least one of the amino acids inside the sites.

(5) In the information processing system as an aspect of the present invention, the plurality of sites is included in a sequence of a heavy chain variable region, a light chain variable region, or a constant region of an antibody.

(6) In the information processing system as an aspect of the present invention, the sequence information is sequence information selected according to results of characterization of antigen-binding molecules or proteins with the sequences represented by the sequence information.

(7) In the information processing system as an aspect of the present invention, the sequence learner performs the machine learning with use of a deep learning model or a probability model.

(8) In the information processing system as an aspect of the present invention, the sequence learner performs the machine learning with use of a deep learning model, and performs the machine learning with use of, as the deep learning model, a Long short-term memory (LSTM), a recursive neural network (RNN), a Gated Recurrent Unit (GRU), a Generative Adversarial Network (GAN), or a Variational Autoencoder (VAE), or a Flow deep generative model.

(9) In the information processing system as an aspect of the present invention, the sequence learner performs the machine learning with use of a probability model, and performs the machine learning with use of, as the probability model, a hidden Markov model (HMM) or a Markov model (MM).

(10) An aspect of the present invention is an information processing system including: a learner configured to perform machine learning on the basis of sequence information representing sequences including some or all of the sequences of a plurality of antigen-binding molecules or proteins and results of characterization of antigen-binding molecules or proteins represented by the sequences and thereby generate a second trained model; and an estimator configured to input virtual sequence information generated on the basis of a first trained model being the trained model according to any one of (1) to (9) into the second trained model, execute arithmetic processing of the second trained model, and thereby estimate predicted values for characterization of antigen-binding molecules or proteins with sequences represented by the inputted virtual sequence information.

(11) The information processing system as an aspect of the present invention comprises an output configured to, according to the predicted values estimated by the estimator, output on the basis of virtual sequence information and the predicted values.

(12) In the information processing system as an aspect of the present invention, the sequence learner to generate the first trained model performs machine learning on the basis of the virtual sequence information and thereby generates a new version of the first trained model, and/or the learner to generate the second trained model performs machine learning on the basis of the virtual sequence information and results of characterization of antigen-binding molecules or proteins with sequences represented by the virtual sequence information and thereby generates a new version of the second trained model.

(13) In the information processing system as an aspect of the present invention, the learner performs the machine learning on the basis of the sequence information represented by character strings, numeric vectors, or physical property values of constituent elements constituting sequences.

(14) In the information processing system as an aspect of the present invention, the sequence information represents amino acid sequences or nucleic acid sequences.

(15) In the information processing system as an aspect of the present invention, the sequence information represents sequences including the sequence of an antigen-binding domain of an antibody.

(16) In the information processing system as an aspect of the present invention, the sequence information represents sequences including the sequence of a constant region of an antibody.

(17) An aspect of the present invention is an information processing device including: a sequence learner configured to perform machine learning on the basis of sequence information representing sequences including some or all of the sequences of a plurality of antigen-binding molecules or proteins and thereby generate a trained model; and a sequence generator configured to generate virtual sequence information obtained by mutating at least one of constituent elements constituting a sequence represented by the sequence information on the basis of the trained model.

(18) An aspect of the present invention is an information processing method in an information processing system, the method including: a sequence learning step of performing machine learning on the basis of sequence information representing sequences including some or all of the sequences of a plurality of antigen-binding molecules or proteins and thereby generating a trained model that has learned a character of the sequence information; and a sequence generation step of generating virtual sequence information obtained by mutating at least one of constituent elements constituting a sequence represented by the sequence information on the basis of the trained model.

(19) An aspect of the present invention is a program configured to allow a computer in an information processing system to execute: a sequence learning procedure of performing machine learning on the basis of sequence information representing sequences including some or all of the sequences of a plurality of antigen-binding molecules or proteins and thereby generating a trained model; and a sequence generation procedure of generating virtual sequence information obtained by mutating at least one of constituent elements constituting a sequence represented by the sequence information on the basis of the trained model.

(20) An aspect of the present invention is a trained model generated by performing machine learning on the basis of sequence information representing sequences including some or all of the sequences of a plurality of antigen-binding molecules or proteins, and used for mutating at least one of constituent elements constituting a sequence represented by the sequence information on the basis of the trained model.

(21) An aspect of the present invention is an antigen-binding molecule or protein having a virtual sequence obtained, on the basis of a trained model generated by performing machine learning on the basis of sequence information representing sequences including some or all of the sequences of a plurality of antigen-binding molecules or proteins, by mutating at least one of constituent elements constituting a sequence represented by the sequence information.

(22) An aspect of the present invention is a method for producing an antigen-binding molecule or protein with use of the information processing system according to any one of (10) to (16) described above, wherein the antigen-binding molecule or protein is represented by a virtual sequence, and a predicted value for characterization has been estimated for the virtual sequence.

(A1) In the information processing system as an aspect of the present invention, the characterization is performed on the basis of appearance frequencies in sequence information representing sequences including some or all of the sequences of a plurality of different antigen-binding molecules.

(A2) In the information processing system as an aspect of the present invention, appearance frequencies in the sequence information are appearance frequencies in sequence information representing sequences of antigen-binding molecules (e.g., binding antibodies) selected from different antigen-binding molecules according to results of screening (e.g., panning) based on characterization.

(A3) In the information processing system as an aspect of the present invention, appearance frequencies in the sequence information are selected according to probabilities of appearance before and after screening (e.g., round-to-round change rates) for antigen-binding molecules (e.g., binding antibodies) selected from different antigen-binding molecules according to results of screening (e.g., panning) based on characterization.

(A4) In the information processing system as an aspect of the present invention, the characterization is performed on the basis of comparison of appearance frequencies of different antibody-binding molecules after screening based on one or more characterizations (e.g., rounds, panning).

(A5) In the information processing system as an aspect of the present invention, each of the antigen-binding molecules is an antigen-binding molecule containing two or more different sequences (e.g., a heavy chain sequence and a light chain sequence), and, on the basis of appearance frequencies of the different sequences constituting the antigen-binding molecule, a sequence estimator (e.g., the estimator 332) estimates an antigen-binding molecule with a combination of the sequences.

(A6) In the information processing system as an aspect of the present invention, the characterization is evaluation of affinity between each of the antigen-binding molecules and a target molecule as a molecule to be targeted.

(A7) In the information processing system as an aspect of the present invention, the evaluation of affinity is evaluation of affinity between each of the antigen-binding molecules and two or more different target molecules.

(A8) In the information processing system as an aspect of the present invention, each of the antigen-binding molecules is an antigen-binding molecule capable of binding to two or more different target molecules.

(A9) In the information processing system as an aspect of the present invention, the evaluation of affinity is evaluation of affinity with each of the antigen-binding molecules in the presence of two or more different target molecules.

(A10) In the information processing system as an aspect of the present invention, each of the antigen-binding molecules is an antigen-binding molecule that does not simultaneously bind to two or more different target molecules.

(A11) In the information processing system as an aspect of the present invention, the different antigen-binding molecules include a plurality of antigen-binding molecules.

(A12) The information processing system as an aspect of the present invention includes a sequence information acquirer (e.g., the next-generation sequencer 20) configured to acquire sequence information representing sequences of different antigen-binding molecules.

(A13) In the information processing system as an aspect of the present invention, a next-generation sequencer (e.g., the next-generation sequencer 20) is used as the sequence information acquirer.

(B1) In the information processing system as an aspect of the present invention, the characterization is evaluation of affinity, evaluation of physical properties, evaluation of pharmacological activity, evaluation of safety, evaluation of kinetics, or evaluation of production suitability for the antigen-binding molecules.

(B2) In the information processing system as an aspect of the present invention, the characterization is at least two of evaluation of affinity, evaluation of physical properties, evaluation of pharmacological activity, evaluation of safety, evaluation of kinetics, and evaluation of production suitability for the antigen-binding molecules.

(B3) The information processing system as an aspect of the present invention includes an output (e.g., the output processor 336) configured to output evaluation values based on results of evaluation of affinity, evaluation of physical properties, evaluation of pharmacological activity, evaluation of safety, evaluation of kinetics, or evaluation of production suitability for the antigen-binding molecules.

(B4) In the information processing system as an aspect of the present invention, the evaluation values outputted by the output are evaluation values based on image data acquired in characterization.

(B5) In the information processing system as an aspect of the present invention, the evaluation values outputted by the output are evaluation values based on time-series data acquired in characterization.

(C1) An aspect of the present invention is an information processing system including: a learning dataset acquirer configured, for characterization of a plurality of antigen-binding molecules against a target antigen, to acquire sequence information representing sequences including some or all of the sequences of the plurality of antigen-binding molecules and learning datasets according to evaluation result information on the characterization of the antigen-binding molecules; a learner configured to learn on the basis of the learning datasets; a controller configured to predict characterization information representing characterization of an antigen-binding molecule represented by input sequence information inputted against the target antigen on the basis of learning results from the learner; and an output configured, according to the predicted characterization information, to output candidate antigen-binding molecule information representing candidate antigen-binding molecules having a characteristic against the target antigen, wherein, for antigen-binding molecules evaluated in a first cycle of the characterization to have a characteristic against the target antigen, the learning dataset acquirer acquires the learning datasets on a second cycle of the characterization.

(C2) An aspect of the present invention is an information processing system including: a learning dataset acquirer configured, for characterization of a plurality of antigen-binding molecules against a target antigen, to acquire sequence information representing sequences including some or all of the sequences of the plurality of antigen-binding molecules and learning datasets according to evaluation result information on the characterization of the antigen-binding molecules; a learner configured to learn on the basis of the learning datasets; a controller configured to predict characterization information representing characterization of an antigen-binding molecule represented by input sequence information inputted against the target antigen on the basis of learning results from the learner; and an output configured, according to the predicted characterization information, to output candidate antigen-binding molecule information representing candidate antigen-binding molecules having a characteristic against the target antigen, wherein the learning datasets include information based on appearance frequencies for respective pieces of the sequence information.

In the information processing system as an aspect of the present invention, the learning datasets include the evaluation result information on measurement of measurement subjects including the plurality of antigen-binding molecules, the measurement conducted per antigen-binding molecule. In the information processing system as an aspect of the present invention, the learning datasets include a set of the sequence information and the evaluation result information each acquired from measurement by a sequencer. In the information processing system as an aspect of the present invention, the learning datasets include a set of the sequence information and the evaluation result information each acquired from measurement by a sequencer, and the sequencer acquires the sequence information and the evaluation result information through measurement for measurement subjects including the plurality of antigen-binding molecules, the measurement conducted per antigen-binding molecule. In the information processing system as an aspect of the present invention, the learning datasets include evaluation result information based on appearance frequencies measured for the antigen-binding molecules, without measurement for dissociation information representing dissociation of each of the antigen-binding molecules.

(C3) In the information processing system as an aspect of the present invention, the learning dataset acquirer acquires a first set of the learning datasets on a first cycle of the characterization and a second set of the learning datasets on a second cycle of the characterization, and a plurality of antigen-binding molecules in the second cycle of the characterization includes antigen-binding molecules evaluated in the first cycle of the characterization to have a characteristic against the target antigen.

(C4) In the information processing system as an aspect of the present invention, the plurality of antigen-binding molecules in the second cycle of the characterization is antigen-binding molecules deriving from removing at least one type of antigen-binding molecules among antigen-binding molecules evaluated in the first cycle of the characterization to have a low characteristic against the target antigen, or deriving from reducing such antibody types.

(C5) In the information processing system as an aspect of the present invention, the sequence information in the second set of the learning datasets derives from removing sequence information representing sequences of at least one type of antigen-binding molecules from the sequence information in the first set of the learning datasets.

(C6) In the information processing system as an aspect of the present invention, the learning dataset acquirer acquires, as the evaluation result information, appearance frequency information representing the number of antigen-binding molecules, among the plurality of antigen-binding molecules, that have bound to the target antigen, and the estimator predicts, for an antigen-binding molecule represented by input sequence information inputted, the appearance frequency information on the antigen-binding molecule for the target antigen, the output outputs the candidate antibody information according to the predicted appearance frequency information, and the plurality of antigen-binding molecules in the second cycle of the characterization includes antigen-binding molecules evaluated to have affinity with the target antigen on the basis of appearance frequency information in the first cycle of the characterization.

(C7) In the information processing system as an aspect of the present invention, the learning dataset acquirer acquires evaluation condition information representing evaluation conditions in the characterization, sequence information representing sequences including some or all of the sequences of the antigen-binding molecules, and learning datasets according to the evaluation result information.

(C8) The information processing system as an aspect of the present invention includes: a criteria storage configured to store, for each cycle of the characterization in a sequence of the characterization, the cycle number in the sequence of the characterization and criteria for characteristics in characterization at the cycle number; and a classifier configured to generate the evaluation result information and characteristic determination information according to the criteria for each cycle number, wherein the learning dataset acquirer acquires sequence information representing sequences including some or all of the sequences of the antigen-binding molecules, and the learning datasets according to the characteristic determination information on the antigen-binding molecules.

(C9) The information processing system as an aspect of the present invention includes: a dataset storage configured to store heavy chain sequence information representing sequences of heavy chain moieties of the antibodies and the evaluation result information on the heavy chain moieties, and light chain sequence information representing sequences of light chain moieties of the antibodies and the evaluation result information on the light chain moieties; and a combination estimator configured to estimate combinations of the heavy chain moieties and the light chain moieties on the basis of the evaluation result information on the heavy chain moieties and the evaluation result information on the light chain moieties, wherein the learning dataset acquirer acquires sequence information representing sequences including some or all of the sequences of antigen-binding molecules containing the combinations and learning datasets according to the evaluation result information on the characterization of the antigen-binding molecules.

(C10) An aspect of the present invention is an information processing system including: a learning dataset acquirer configured to acquire, for characterization of a plurality of antigen-binding molecules against a target antigen, sequence information representing sequences including some or all of the sequences of the antigen-binding molecules and learning datasets according to evaluation result information on the characterization of the antigen-binding molecules from a sequencer; a sequence information generator configured to generate input sequence information to be inputted into a trained learning device that has performed machine learning to estimate a characteristic of each of the antigen-binding molecules against the target antigen as the degree of the characteristic of an antigen-binding molecule against the target antigen; an estimator configured to input the input sequence information generated by the sequence information generator into the learning device and execute arithmetic processing of the trained learning device to acquire characterization information representing the degree of the characteristic of each of the antigen-binding molecules against the target antigen from the learning device; and an output configured to output candidate antigen-binding molecule information representing candidate antigen-binding molecules having a characteristic against the target antigen, according to the predicted characterization information, wherein the sequence information generator generates, on the basis of sequence information on the antigen-binding molecules having a characteristic against the target antigen, the input sequence information by mutating one or more amino acids of an amino acid sequence included in the sequence information.

(C11) An aspect of the present invention is an information processing method including: a learning dataset acquisition step of acquiring, for evaluation of affinity between each of a plurality of antibodies and a target antigen, sequence information representing sequences including some or all of the sequences of the antibodies and learning datasets according to evaluation result information on the evaluation of affinity for the antibodies; a learning step of learning on the basis of the learning datasets; a control step of predicting, for an antibody represented by input sequence information inputted, affinity information representing affinity between the antibody and the target antigen on the basis of learning results in the learning step; and an output step of outputting candidate antibody information representing candidate antibodies having affinity with the target antigen according to the predicted affinity information, wherein a first set of the learning datasets on a first cycle of the evaluation of affinity and a second set of the learning datasets on a second cycle of the evaluation of affinity are acquired in the learning dataset acquisition step, and a plurality of antibodies in the second cycle of the evaluation of affinity includes antibodies evaluated to have affinity with the target antigen in the first cycle of the evaluation of affinity.

(C12) An aspect of the present invention is an information processing device including: a learning dataset acquirer configured to acquire, for evaluation of affinity between each of a plurality of antibodies and a target antigen, sequence information representing sequences including some or all of the sequences of the antibodies and learning datasets according to evaluation result information on the evaluation of affinity for the antibodies; and a learner configured to learn on the basis of the learning dataset, wherein the learning dataset acquirer acquires a first set of the learning datasets on a first cycle of the evaluation of affinity and a second set of the learning datasets on a second cycle of the evaluation of affinity, and a plurality of antibodies in the second cycle of the evaluation of affinity includes antibodies evaluated to have affinity with the target antigen in the first cycle of the evaluation of affinity.

(C13) An aspect of the present invention is an information processing device including: a controller configured to read out, for evaluation of affinity between each of a plurality of antibodies and a target antigen, learning results obtained by using sequence information representing sequences including some or all of the sequences of the antibodies and learning datasets according to evaluation result information on the evaluation of affinity for the antibodies, from a storage, and predict, for an antibody represented by input sequence information inputted, affinity information representing affinity between the antibody and the target antigen on the basis of the learning results; and an output configured to output candidate antibody information representing candidate antibodies having affinity with the target antigen according to the predicted affinity information, wherein the learning datasets include a first set of the learning datasets on a first cycle of the evaluation of affinity and a second set of the learning datasets on a second cycle of the evaluation of affinity, and a plurality of antibodies in the second cycle of the evaluation of affinity includes antibodies evaluated to have affinity with the target antigen in the first cycle of the evaluation of affinity.

(C14) An aspect of the present invention is a program configured to allow one or more computers to execute: a learning dataset acquisition procedure of acquiring, for evaluation of affinity between each of a plurality of antibodies and a target antigen, sequence information representing sequences including some or all of the sequences of the antibodies and learning datasets according to evaluation result information on the evaluation of affinity for the antibodies; and a learning procedure of learning on the basis of the learning datasets, wherein a first set of the learning datasets on a first cycle of the evaluation of affinity and a second set of the learning datasets on a second cycle of the evaluation of affinity are acquired in the learning dataset acquisition procedure, and a plurality of antibodies in the second cycle of the evaluation of affinity includes antibodies evaluated to have affinity with the target antigen in the first cycle of the evaluation of affinity.

(C15) An aspect of the present invention is a program configured to allow one or more computers to execute: a control procedure of reading out, for evaluation of affinity between each of a plurality of antibodies and a target antigen, learning results obtained by using sequence information representing sequences including some or all of the sequences of the antibodies and learning datasets according to evaluation result information on the evaluation of affinity for the antibodies, from a storage, and predicting, for an antibody represented by input sequence information inputted, affinity information representing affinity between the antibody and the target antigen on the basis of the learning results; and an output procedure of outputting candidate antibody information representing candidate antibodies having affinity with the target antigen according to the predicted affinity information, wherein the learning datasets include a first set of the learning datasets on a first cycle of the evaluation of affinity and a second set of the learning datasets on a second cycle of the evaluation of affinity, and a plurality of antibodies in the second cycle of the evaluation of affinity includes antibodies evaluated to have affinity with the target antigen in the first cycle of the evaluation of affinity.

(C16) An aspect of the present invention is a trained model trained, for evaluation of affinity between each of a plurality of antibodies and a target antigen, with sequence information representing sequences including some or all of the sequences of the antibodies and learning datasets learning datasets according to evaluation result information on the evaluation of affinity for the antibodies, in order to allow a computer to function to: predict, for an antibody represented by input sequence information inputted, affinity information representing affinity between the antibody and the target antigen; and output candidate antibody information representing candidate antibodies having affinity with the target antigen according to the predicted affinity information.

(C17) An aspect of the present invention is a screening method including: a learning dataset acquisition step of acquiring, for characterization of a plurality of antigen-binding molecules against a target antigen, sequence information representing sequences including some or all of the sequences of the antigen-binding molecules and learning datasets according to evaluation result information on the characterization of the antigen-binding molecules; a learning step of learning on the basis of the learning datasets; a control step of predicting, for an antigen-binding molecule represented by input sequence information inputted, characterization information representing characterization of the antigen-binding molecule against the target antigen on the basis of learning results in the learning step; and an output step of outputting candidate antigen-binding molecule information representing candidate antigen-binding molecules having a characteristic against the target antigen according to the predicted characterization information, wherein a set of the learning datasets on a second cycle of the characterization of antigen-binding molecules evaluated to have a characteristic against the target antigen in a first cycle of the characterization is acquired in the learning dataset acquisition step.

(C18) An aspect of the present invention is an antibody-binding molecule outputted by an output in an information processing device, the information processing device including: a learning dataset acquirer configured to acquire, for characterization of a plurality of antigen-binding molecules against a target antigen, sequence information representing sequences including some or all of the sequences of the antigen-binding molecules and learning datasets according to evaluation result information on the characterization of the antigen-binding molecules; a learner configured to learn on the basis of the learning datasets; a controller configured to predict, for an antigen-binding molecule represented by input sequence information inputted, characterization information representing characterization of the antigen-binding molecule against the target antigen on the basis of learning results from the learner; and an output configured to output candidate antigen-binding molecule information representing candidate antigen-binding molecules having a characteristic against the target antigen according to the predicted characterization information, wherein the learning dataset acquirer acquires a set of the learning datasets on a second cycle of the characterization of antigen-binding molecules evaluated to have a characteristic against the target antigen in a first cycle of the characterization.

(D1) An aspect of the present invention is an information processing system including: an information acquirer configured to acquire sequence information on amino acid sequences of antigen-binding molecules that bind to a target antigen, and acquire, for characteristics of the antigen-binding molecules, first characteristic information representing a first-type characteristic of the characteristics and second characteristic information representing a second-type characteristic of the characteristics; a learner configured to learn on the basis of the sequence information, the first characteristic information, and the second characteristic information; a predictor configured to predict, for an antigen-binding molecule represented by input sequence information inputted, first characterization information representing the first-type characteristic and second characterization information representing the second-type characteristic on the basis of learning results from learning by the learner; and an output configured to output candidate antigen-binding molecule information representing candidate antigen-binding molecules having the characteristics according to the first characterization information and the second characterization information predicted by the predictor.

(D2) In the information processing system as an aspect of the present invention, the learner generates a first trained model as learning results based on the sequence information and the first characteristic information and a second trained model as learning results based on the sequence information and the second characteristic information, and the predictor predicts first characterization information on the basis of the first trained model and predicts second characterization information on the basis of the second trained model.

(D3) In the information processing system as an aspect of the present invention, the first characteristic information is activity information representing activity between the target antigen and each of the antigen-binding molecules, and the second characteristic information is physical property information representing physical properties of each of the antigen-binding molecules.

(D4) In the information processing system as an aspect of the present invention, the first characteristic information and the second characteristic information are at least two sets of information of binding activity information representing binding activity between the target antigen and each of the antigen-binding molecules, pharmacological activity information representing pharmacological activity between the target antigen and each of the antigen-binding molecules, and stability information representing stability of the antigen-binding molecules.

(D5) In the information processing system as an aspect of the present invention, the first characteristic information and the second characteristic information are a plurality of types of the binding activity information, a plurality of types of the pharmacological activity information, or a plurality of types of the stability information.

(D6) The information processing system as an aspect of the present invention further includes a sequence information generator configured to select, on the basis of first sequence information and second sequence information differing in at least some of the amino acid sequences, a position in a sequence in the first sequence information and mutate the amino acid at the selected position in the sequence to generate the input sequence information.

(D7) In the information processing system as an aspect of the present invention, the sequence information generator selects a predetermined number or less of positions in the sequence and mutates the amino acids at the selected positions in the sequence.

(D8) In the information processing system as an aspect of the present invention, the sequence information generator mutates an amino acid at a position different from the selected position in the sequence in the input sequence information to generate an additional set of the input sequence information.

(D9) In the information processing system as an aspect of the present invention, the first sequence information is a plurality of sets of sequence information, and the sequence information generator selects a position in a sequence in the first sequence information on the base of the input sequence information generated and the first sequence information, and mutates the amino acid at the selected position in the sequence to generate an additional set of the input sequence information.

(D10) In the information processing system as an aspect of the present invention, the sequence information includes at least one of character string information representing amino acid sequences of the antigen-binding molecules, physical property value information representing physical property values of amino acids contained in amino acid sequences of the antigen-binding molecules, and three-dimensional structure information representing characteristics of three-dimensional structure based on the amino acid sequence of each of the antigen-binding molecules.

(D11) An aspect of the present invention is an information processing device including: an information acquirer configured to acquire sequence information on amino acid sequences of antigen-binding molecules that bind to a target antigen and characteristic information representing characteristics of the antigen-binding molecules; a learner configured to learn for each type of the characteristics on the basis of the sequence information and the characteristic information; a predictor configured to predict, for an antigen-binding molecule represented by input sequence information inputted, characterization information representing characterization of the antigen-binding molecule for each type of the characteristics on the basis of learning results from learning by the learner; and an output configured to output candidate antigen-binding molecule information representing candidate antigen-binding molecules having the characteristics according to a plurality of sets of the characterization information predicted by the predictor for respective types of the characteristics.

(D12) An aspect of the present invention is an information processing device including: an information acquirer configured to acquire sequence information on amino acid sequences of antigen-binding molecules that bind to a target antigen and characteristic information representing characteristics of the antigen-binding molecules; and a learner configured to learn on the basis of the sequence information and the characteristic information for each type of the characteristics.

(D13) An aspect of the present invention is an information processing device including: a predictor configured to read out, on the basis of sequence information on amino acid sequences of antigen-binding molecules that bind to a target antigen and characteristic information representing characteristics of the antigen-binding molecules, learning results from learning for each type of the characteristics, and predict, for an antigen-binding molecule represented by input sequence information inputted, characterization information representing characterization of the antigen-binding molecule for each type of the characteristics on the basis of learning results from learning by the learner, and an output configured to output candidate antigen-binding molecule information representing candidate antigen-binding molecules having the characteristics according to a plurality of sets of the characterization information predicted by the predictor for respective types of the characteristics.

(D14) An aspect of the present invention is an information processing method including: an information acquisition step in which an information processor acquires sequence information on amino acid sequences of antigen-binding molecules that bind to a target antigen and characteristic information representing characteristics of the antigen-binding molecules; a learning step in which a learner learns for each type of the characteristics on the basis of the sequence information and the characteristic information; a prediction step in which a predictor predicts, for an antigen-binding molecule represented by input sequence information inputted, characterization information representing characterization of the antigen-binding molecule for each type of the characteristics on the basis of learning results from learning in the learning step; and an output step in which an output outputs candidate antigen-binding molecule information representing candidate antigen-binding molecules having the characteristics according to a plurality of sets of the characterization information predicted in the prediction step for respective types of the characteristics.

(D15) An aspect of the present invention is a program configured to allow a computer to execute: an information acquisition step of acquiring sequence information on amino acid sequences of antigen-binding molecules that bind to a target antigen and characteristic information representing characteristics of the antigen-binding molecules; a learning step of learning for each type of the characteristics on the basis of the sequence information and the characteristic information; a prediction step of predicting, for an antigen-binding molecule represented by input sequence information inputted, characterization information representing characterization of the antigen-binding molecule for each type of the characteristics on the basis of learning results from learning in the learning step; and an output step of outputting candidate antigen-binding molecule information representing candidate antigen-binding molecules having the characteristics according to a plurality of sets of the characterization information predicted in the prediction step for respective types of the characteristics.

(D16) An aspect of the present invention is a trained model trained, according to sequence information on amino acid sequences of antigen-binding molecules that bind to a target antigen and characteristic information representing characteristics of the antigen-binding molecules, for each type of the characteristics, in order to allow a computer to function to output, for an antigen-binding molecule represented by input sequence information inputted, characterization information representing characterization of the antigen-binding molecule for each type of the characteristics.

(E1) In the information processing system as an aspect of the present invention, the antigen-binding molecule is a protein.

(E2) In the information processing system as an aspect of the present invention, the antigen-binding molecule is an antibody.

(E3) In the information processing system as an aspect of the present invention, the antigen-binding molecule is a peptide.

A part of the information processing system 1 (1a to Id) in the above-described embodiments, such as the processor 33 (33a to 33d), may be implemented with a computer. In this case, implementation may be carried out by storing a program to achieve the functions in a computer-readable storage medium and allowing a computer system to read and execute the program stored in the storage medium. The "computer system" mentioned here is a computer system built in the information processing system 1 (1a to Id), and intended to include an OS (Operating System) and hardware such as peripherals.

The "computer-readable storage medium" refers to a storage device such as a portable medium, such as a flexible disk, a magneto-optical disk, a ROM, and a CD-ROM, and a hard disk built in computer systems. Further, the "computer-readable storage medium" may include a medium to dynamically retain a program in a short time like a communication wire in sending a program via a network such as the Internet or a communication line such as a telephone line, or a medium regaining a program for a certain time like a volatile memory in the inside of a computer system to serve as a server or a client in that case. The program may be a program to achieve some of the above-described functions, or a program that can achieve the above-described functions with a combination of a program already stored in a computer system.

A part or the entire of the information processing system 1 (1a to Id) in the above-described embodiments may be implemented as an integrated circuit, for example, by LSI (Large Scale Integration). The functional units of the processor 33 (33a to 33d) may be individually implemented as a processor, and a part or the entire of the processor 33 (33a to 33d) may be integrated to implement as a processor. The technique for integrating into an integrated circuit is not limited to LSI, and implementation may be carried out with a special circuit or a versatile processor. If any technology for integrating into an integrating circuit as an alternative to LSI appears because of the progress of the semiconductor technology, an integrated circuit made with the technology may be used.

Thus, certain embodiments of the present invention have been described in detail with reference to the drawings; however, specific configurations are not limited to those described above, and various design modifications are acceptable as long as the modifications do not depart from the spirit of the present invention.

[Reference Signs List]

1, 1a, 1b, 1c, Id...Information processing system
10, 10c...User terminal; 11...communicator, 12...input; 13...storage; 14, 14c...processor; 15...display
20...Next-generation sequencer; 21...communicator; 22...input; 23...storage; 24...nucleotide sequence measurer; 25...controller, 26...display
30, 30a, 30b, 30c, 30d...Server, 31, 31c...communicator; 32, 32a, 32c, 32d...storage; 321...experiment information storage; 322, 322a...dataset storage; 323, 323a...classification criteria storage; 324, 324c, 324d...learning dataset storage; 325...focused position information storage; 326, 326b, 326c, 326d...learning result storage; 327, 327c...mutation information storage; 328, 328c...sequence storage; 329, 329c...characterization information storage; 33, 33b, 33c...processor; 331, 331c...information acquirer; 332...estimator; 333...classifier; 334, 334b, 334c, 334d...learner; PA, PAc...prediction target sequence generator; PA1, PA1b, PAc, PAd... sequence selector; PA2, PA2b, PA2c, PA2d...sequence learner; PA3, PA3b, PA3c, PA3d...virtual sequence generator; 335, 335c, 335d...controller; 336, 336c...output processor 901...CPU; 902...storage medium interface; 903...storage medium; 904...input; 905...output; 906...ROM; 907...RAM; 908...auxiliary storage; 909...interface

[Industrial Applicability]

The present invention can be used, for example, in drug discovery, prediction of substances, prediction of antibodies, prediction of antigens, prediction of sequences, or prediction of characteristics.

## Claims

1. An information processing system comprising:

   a sequence learner configured to perform machine learning on the basis of sequence information representing sequences including some or all of the sequences of a plurality of antigen-binding molecules and thereby generate a trained model that has learned a character of the sequences; and
   a sequence generator configured to generate virtual sequence information obtained by mutating at least one of constituent elements constituting a sequence represented by the sequence information on the basis of the trained model.

2. An information processing system comprising:

   a sequence learner configured to perform machine learning on the basis of sequence information representing sequences including some or all of the sequences of a plurality of proteins and thereby generate a trained model that has learned a character of the sequences; and
   a sequence generator configured to generate virtual sequence information obtained by mutating at least one of constituent elements constituting a sequence represented by the sequence information on the basis of the trained model.

3. The information processing system according to claim 1 or 2, wherein
   the character of the sequences is a character including positions of the constituent elements in the sequences and anteroposterior relationship of the constituent elements.

4. The information processing system according to any one of claims 1 to 3, wherein the virtual sequence information is generated by changing at least one of the constituent elements in preset sites including one or more of the constituent elements in a sequence.

5. The information processing system according to claim 4, wherein the plurality of sites is included in a sequence of a heavy chain variable region, a light chain variable region, or a constant region of an antibody.

6. The information processing system according to any one of claims 1 to 5, wherein the sequence information is sequence information selected according to results of characterization of antigen-binding molecules or proteins with the sequences represented by the sequence information.

7. The information processing system according to claim 6, wherein the sequence learner performs the machine learning with use of a deep learning model or a probability model.

8. The information processing system according to claim 7, wherein the sequence learner performs the machine learning with use of a deep learning model, and
   performs the machine learning with use of, as the deep learning model, a Long short-term memory (LSTM), a recursive neural network (RNN), a Gated Recurrent Unit (GRU), a Generative Adversarial Network (GAN), or a Variational Autoencoder (VAE), or a Flow deep generative model.

9. An information processing system comprising:

   a learner configured to perform machine learning on the basis of sequence information representing sequences including some or all of the sequences of a plurality of antigen-binding molecules or proteins and results of characterization of antigen-binding molecules or proteins represented by the sequences and thereby generate a second trained model; and
   an estimator configured to input virtual sequence information generated on the basis of a first trained model being the trained model according to any one of claims 1 to 8 into the second trained model, execute arithmetic processing of the second trained model, and thereby estimate predicted values for characterization of antigen-binding molecules or proteins with sequences represented by the inputted virtual sequence information.

10. The information processing system according to claim 9, comprising:
an output configured to, according to the predicted values estimated by the estimator, output on the basis of virtual sequence information and the predicted values.

11. The information processing system according to claim 10, wherein

the sequence learner to generate the first trained model performs machine learning on the basis of the virtual sequence information and thereby generates a new version of the first trained model, and/or
the learner to generate the second trained model performs machine learning on the basis of the virtual sequence information and results of characterization of antigen-binding molecules or proteins with sequences represented by the virtual sequence information and thereby generates a new version of the second trained model.

12. The information processing system according to any one of claims 1 to 11, wherein the sequence learner performs the machine learning on the basis of the sequence information represented by character strings, numeric vectors, or physical property values of constituent elements constituting sequences.

13. An information processing method in an information processing system, the method comprising:

a sequence learning step of performing machine learning on the basis of sequence information representing sequences including some or all of the sequences of a plurality of antigen-binding molecules or proteins and thereby generating a trained model that has learned a character of the sequence information; and
a sequence generation step of generating virtual sequence information obtained by mutating at least one of constituent elements constituting a sequence represented by the sequence information on the basis of the trained model.

14. A program configured to allow a computer in an information processing system to execute:

a sequence learning procedure of performing machine learning on the basis of sequence information representing sequences including some or all of the sequences of a plurality of antigen-binding molecules or proteins and thereby generating a trained model; and
a sequence generation procedure of generating virtual sequence information obtained by mutating at least one of constituent elements constituting a sequence represented by the sequence information on the basis of the trained model.

15. A method for producing an antigen-binding molecule or protein with use of the information processing system according to any one of claims 9 to 12, wherein the antigen-binding molecule or protein is represented by a virtual sequence, and a predicted value for characterization has been estimated for the virtual sequence.

Fig. 1

Fig. 2

PANNING ID: P1

EXPERIMENT CONDITIONS: CONDITIONS 1

BINDING EXPERIMENT (ROUND 1)

NON-BINDING ANTIBODIES

BINDING ANTIBODIES

SEQUENCE ANALYSIS

SEQUENCE INFORMATION 1 (EVALUATION RESULT INFORMATION 1)
HEAVY CHAIN SEQUENCE A (NUMBER OF APPEARANCES: A1) LIGHT
CHAIN SEQUENCE C (NUMBER OF APPEARANCES: C1)
HEAVY CHAIN SEQUENCE B (NUMBER OF APPEARANCES: B1) LIGHT
CHAIN SEQUENCE D (NUMBER OF APPEARANCES: D1)
...

EXPERIMENT CONDITIONS: CONDITIONS 2

BINDING EXPERIMENT (ROUND 2)

NON-BINDING ANTIBODIES

BINDING ANTIBODIES

SEQUENCE ANALYSIS

SEQUENCE INFORMATION 2 (EVALUATION RESULT INFORMATION 2)
HEAVY CHAIN SEQUENCE A (NUMBER OF APPEARANCES: A2) LIGHT
CHAIN SEQUENCE C (NUMBER OF APPEARANCES: C2)
HEAVY CHAIN SEQUENCE B (NUMBER OF APPEARANCES: B2) LIGHT
CHAIN SEQUENCE D (NUMBER OF APPEARANCES: D2)
...

EXPERIMENT CONDITIONS: CONDITIONS N

BINDING EXPERIMENT (ROUND N)

NON-BINDING ANTIBODIES

BINDING ANTIBODIES

SEQUENCE ANALYSIS

SEQUENCE INFORMATION N (EVALUATION RESULT INFORMATION N)
HEAVY CHAIN SEQUENCE A (NUMBER OF APPEARANCES: AN) LIGHT
CHAIN SEQUENCE C (NUMBER OF APPEARANCES: CN)
HEAVY CHAIN SEQUENCE B (NUMBER OF APPEARANCES: BN) LIGHT
CHAIN SEQUENCE D (NUMBER OF APPEARANCES: DN)
...

EXPERIMENT CONDITIONS: CONDITIONS N+1

BINDING EXPERIMENT (ROUND N+1)

EP 3 982 369 A1

Fig. 3

```
                              ⌒10
        ┌──────────────────────────────┐
        │         USER TERMINAL        │
        │  ┌────────────────────┐       │
        │  │   COMMUNICATOR     │⌒11    │
        │  └────────────────────┘       │
        │                               │
        │  ┌────────────────────┐       │
        │  │       INPUT        │⌒12    │
        │  └────────────────────┘       │
        │                               │
        │  ┌────────────────────┐       │
        │  │      STORAGE       │⌒13    │
        │  └────────────────────┘       │
        │                               │
        │  ┌────────────────────┐       │
        │  │     PROCESSOR      │⌒14    │
        │  └────────────────────┘       │
        │                               │
        │  ┌────────────────────┐       │
        │  │      DISPLAY       │⌒15    │
        │  └────────────────────┘       │
        └──────────────────────────────┘
```

Fig. 4

EP 3 982 369 A1

D11

<Screen of search>

[Basic settings]　(Specification of either Target antigen or Experiment is required)

Target antigen: _____

Experiment:　Not specify　[ Specify panning ]

[Search conditions]

Antibody: _____
[ Select library ]　[ Select antibody ]

Experiment conditions:
- Antibody concentration _____ [ Select range ]
- Antigen display technique _____ [ Select technique ]
- Antibody DP technique _____ [ Select technique ]
- Antibody origin _____ [ Select range ]
- Solution composition _____ [ Select composition ]
- Reaction time _____ [ Select range ]
- Reaction temperature _____ [ Select range ]

[Setting of trained model]
- Classification criteria:　　　　　　　　　[ Set criteria ]
- Criterion 1　Number of appearances in R1　X1 or more　Change rate for R12　Y1 or more　Change rate for R13　Z1 or more
- Criterion 2　Number of appearances in R1　X2 or more　Change rate for R12　Y2 or more　Change rate for R13　Z2 or more
- Criterion 3　Number of appearances in R1　X3 or more　Change rate for R12　Y3 or more　Change rate for R13　Z3 or more
- Focused position:　[ Specify focused position ]

[Setting of conditions for prediction target]

Mutation information:　[ Specify mutation position ]

[ Search ]

BT111

D12

<Screen of search result>
Target antigen: XXXXXXXXX

Avidity (strong → weak)　[ Sort ]　[ Filter ]

Candidate antibody　　Prediction score

ME ···DSR··· MF　　45.3
ME ···DSK··· MF　　39.7
ME ···WSR··· MF　　25.2
ME ···WSK··· MF　　20.1
⋮　　　　⋮

[ Next ]

D111

<Screen of setting of classification criteria information>

Criterion: [ 1　▽ ]

Round 1　　　Threshold for number of appearances: [ X1 ]
Round 1 → 2　Threshold for change rage: [ Y1 ]
Round 2 → 3　Threshold for change rage: [ Z1 ]

[ Specify thresholds ]

73

Fig. 5

NEXT-GENERATION SEQUENCER — 20

21 — COMMUNICATOR

22 — INPUT

23 — STORAGE

24 — NUCLEOTIDE SEQUENCE MEASURER

25 — CONTROLLER

26 — DISPLAY

Fig. 6

SERVER /30

COMMUNICATOR /31

/33
PROCESSOR

/32
STORAGE

/321
EXPERIMENT INFORMATION STORAGE

/322
DATASET STORAGE

/323
CLASSIFICATION CRITERIA STORAGE

/324
LEARNING DATASET STORAGE

/325
FOCUSED POSITION INFORMATION STORAGE

/326
LEARNING RESULT STORAGE

/327
MUTATION INFORMATION STORAGE

/328
SEQUENCE STORAGE

/329
CHARACTERIZATION INFORMATION STORAGE

/331
INFORMATION ACQUIRER

/332
ESTIMATOR

/333
CLASSIFIER

/PA
PREDICTION TARGET SEQUENCE GENERATOR

/PA1
SEQUENCE SELECTOR

/PA2
SEQUENCE LEARNER

/PA3
VIRTUAL SEQUENCE GENERATOR

/334
LEARNER

/335
CONTROLLER

/336
OUTPUT PROCESSOR

Fig. 7

| Panning group ID | Target antigen | Antibody library | Datasets | Experiment conditions ID | Round 2 experiment conditions ID | Round 3 experiment conditions ID |
|---|---|---|---|---|---|---|
| P1 | antibody 1 | library 1 | H12345.csv L54321.csv | conditions 1 | conditions 2 | conditions 3 |
| ... | ... | ... | ... | ... | ... | ... |

Fig. 8

| Experiment conditions ID | Antibody display technique | Origin of antibodies | Concentration of target antigen | Composition of buffer | Reaction time | Reaction temperature | ... |
|---|---|---|---|---|---|---|---|
| conditions 1 | phage | mouse | 1 | composition A | T0 | t1 | ... |
| ... | ... | ... | ... | ... | ... | ... | ... |

Fig. 9

EP 3 982 369 A1

File: H12345.csv
Panning group ID: P1

| Sequence ID | H1 | H2 | ... | H35a | H35b | H36 | ... | Appearance frequency in round 1 | Appearance frequency in round 2 | Appearance frequency in round 3 | ... | Change rate (1→2) | Change rate (2→3) | ... |
|---|---|---|---|---|---|---|---|---|---|---|---|---|---|---|
| VH001 | M | E | ... | P | S | Q | ... | 0.10 | 0.25 | 0.50 | ... | 2.50 | 2.00 | ... |
| VH002 | M | E | ... | D | S | Q | ... | 0.02 | 0.02 | 0.04 | ... | 1.00 | 2.00 | ... |
| VH003 | M | E | ... | W | S | Q | ... | 0.05 | 0.04 | 0.10 | ... | 0.80 | 2.50 | ... |
| VH004 | M | E | ... | P | S | R | ... | 0.04 | 0.04 | 0.12 | ... | 1.00 | 3.00 | ... |
| VH005 | M | E | ... | P | S | K | ... | 0.00 | 0.01 | 0.00 | ... | 0.00 | 0.00 | ... |
| ... | ... | ... | ... | ... | ... | ... | ... | ... | ... | ... | ... | ... | ... | ... |

# Fig. 10

File: L54321.csv
Panning group ID: P1

| Sequence ID | L1 | L2 | ... | L27 | ... | Appearance frequency in round 1 | Appearance frequency in round 2 | Appearance frequency in round 3 | ... | Change rate (1→2) | Change rate (2→3) | ... |
|---|---|---|---|---|---|---|---|---|---|---|---|---|
| VL001 | M | F | ... | A | ... | 0.10 | 0.25 | 0.50 | ... | 2.50 | 2.00 | ... |
| VL002 | M | F | ... | R | ... | 0.02 | 0.02 | 0.04 | ... | 0.80 | 2.50 | ... |
| VL003 | M | F | ... | G | ... | 0.05 | 0.04 | 0.10 | ... | 1.00 | 2.00 | ... |
| VL004 | M | F | ... | H | ... | 0.04 | 0.04 | 0.12 | ... | 1.00 | 3.00 | ... |
| VL005 | M | F | ... | L | ... | 0.00 | 0.01 | 0.00 | ... | 0.00 | 0.00 | ... |
| ... | ... | ... | ... | ... | ... | ... | ... | ... | ... | ... | ... | ... |

Fig. 11

EP 3 982 369 A1

| Panning group ID | Learning dataset (criterion 1) | Learning dataset (criterion 2) | Learning dataset (criterion 3) | ... | Trained model | ... |
|---|---|---|---|---|---|---|
| P1 | P1_HL5678_1.csv | P1_HL5678_2.csv | P1_HL5678_2.csv | ... | ... | ... |
| ... | ... | ... | ... | ... | ... | ... |

File: P1_HL5678_1.csv
Panning group ID: P1

| Sequence ID | H1 | H2 | ... | H35a | H35b | H36 | ... | L1 | L2 | ... | L27 | ... | Appearance frequency in round 1 | Appearance frequency in round 2 | Appearance frequency in round 3 | ... | Change rate (1→2) | Change rate (2→3) | ... | Binding determination |
|---|---|---|---|---|---|---|---|---|---|---|---|---|---|---|---|---|---|---|---|---|
| VHL0001 | M | E | ... | P | S | Q | ... | M | F | ... | A | ... | ... | ... | ... | ... | ... | ... | ... | binding |
| VHL0002 | M | E | ... | D | S | Q | ... | M | F | ... | R | ... | ... | ... | ... | ... | ... | ... | ... | binding |
| VHL0003 | M | E | ... | W | S | Q | ... | M | F | ... | G | ... | ... | ... | ... | ... | ... | ... | ... | non-binding |
| VHL0004 | M | E | ... | P | S | R | ... | M | F | ... | H | ... | ... | ... | ... | ... | ... | ... | ... | binding |
| ... | ... | ... | ... | ... | ... | ... | ... | ... | ... | ... | ... | ... | ... | ... | ... | ... | ... | ... | ... | ... |

Fig. 12

Panning group ID: P1

| Sequence ID | H1 | H2 | ... | H35a | H35b | H36 | ... | L1 | L2 | ... | L27 | ... |
|---|---|---|---|---|---|---|---|---|---|---|---|---|
| V000001 | M | E | ... | D | S | R | ... | M | F | ... | A | ... |
| V000002 | M | E | ... | D | S | K | ... | M | F | ... | A | ... |
| V000003 | M | E | ... | W | S | R | ... | M | F | ... | A | ... |
| V000004 | M | E | ... | W | S | K | ... | M | F | ... | A | ... |
| ... | ... | ... | ... | ... | ... | ... | ... | ... | ... | ... | ... | ... |

## Fig. 13

Panning group ID: P1

| Sequence ID | Prediction score |
|---|---|
| V000001 | |
| V000002 | |
| V000003 | |
| V000004 | |
| ... | ... |

## Fig. 14

| Sequence ID | H1 | H2 | H3 |
|---|---|---|---|
| VHL0001 | M | E | E |
| ... | ... | ... | ... |

"END"

$h_0$  $h_1$  $h_2$  $h_M$

$A_0$ → $A_1$ → $A_2$ → $A_M$

$X_0$  $X_1$  $X_2$  ....... $X_M$

"START"

| Sequence ID | H1 | H2 | ... | L107a |
|---|---|---|---|---|
| VHL0001 | M | E | ... | – |
| ... | ... | ... | ... | ... |

$\begin{pmatrix} 0 \\ 0 \\ 0 \\ 1 \\ 0 \\ \vdots \\ 0 \end{pmatrix}$

## Fig. 15

Fig. 16

START

INSTRUCT GENERATION OF SEQUENCES — S1

GENERATE SEQUENCES — S2

STORE AS GROUP OF SEQUENCES — S3

S4
IS END CONDITION SATISFIED?
NO
YES

END

Fig. 17

START

ACQUIRE VARIOUS TYPES
OF INFORMATION FROM
USER TERMINAL 10 — S101

ACQUIRE ANALYSIS
RESULT INFORMATION — S102

ESTIMATE COMBINATION OF
HEAVY CHAIN/LIGHT CHAIN — S103

GENERATE LEARNING
DATASET — S104

EVALUATE
LEARNING/PRECISION — S105

DETERMINE CLASSIFICATION
CRITERIA AND TRAINED MODEL — S106

END

Fig. 18

START

GENERATE PREDICTION
TARGET SEQUENCES — S201

GIVE PREDICTION
SCORES — S202

OUTPUT CANDIDATE
ANTIBODY INFORMATION — S203

END

Fig. 19

## Fig. 20

File: H23456.csv
Panning group ID: P1, P2, P3

| H1 | H2 | ... | H35a | H35b | H36 | ... | P1, appearance frequency in round 1 (antigen+ small molecule+) | P2, appearance frequency in round 1 (antigen- small molecule+) | P3, appearance frequency in round 1 (antigen+ small molecule-) |
|---|---|---|---|---|---|---|---|---|---|
| M | E | ... | P | S | Q | ... | 0.516 | 0.000 | 0.001 |
| M | E | ... | D | S | Q | ... | 0.302 | 0.001 | 0.000 |
| M | E | ... | W | S | Q | ... | 0.125 | 0.002 | 0.156 |
| M | E | ... | P | S | R | ... | 0.050 | 0.003 | 0.250 |
| M | E | ... | P | S | K | ... | 0.037 | 0.045 | 0.005 |
| ... | ... | ... | ... | ... | ... | ... | ... | ... | ... |

Fig. 21

EP 3 982 369 A1

File: L65432.csv
Panning group ID: P1, P2, P3

| L1 | L2 | ... | L27 | ... | P1, appearance frequency in round 1 (antigen+ small molecule+) | P2, appearance frequency in round 1 (antigen- small molecule+) | P3, appearance frequency in round 1 (antigen+ small molecule-) | ... |
|---|---|---|---|---|---|---|---|---|
| M | F | ... | A | ... | 0.050 | 0.000 | 0.001 | ... |
| M | F | ... | R | ... | 0.052 | 0.003 | 0.245 | ... |
| M | F | ... | G | ... | 0.120 | 0.003 | 0.150 | ... |
| M | F | ... | H | ... | 0.322 | 0.001 | 0.000 | ... |
| M | F | ... | L | ... | 0.035 | 0.042 | 0.004 | ... |
| ... | ... | ... | ... | ... | ... | ... | ... | ... |

## Fig. 22

Fig. 23

Fig. 24

10c

USER TERMINAL

| COMMUNICATOR | 11 |

| INPUT | 12 |

| STORAGE | 13 |

| PROCESSOR | 14c |

| DISPLAY | 15 |

Fig. 25

/30c

SERVER

/31c

COMMUNICATOR

/33c

PROCESSOR

/331c

INFORMATION
ACQUIRER

/32c

STORAGE

/324c

LEARNING DATASET
STORAGE

/326c

LEARNING RESULT
STORAGE

/327c

MUTATION INFORMATION
STORAGE

/328c

SEQUENCE STORAGE

/329c

CHARACTERIZATION
INFORMATION STORAGE

/PAc

PREDICTION TARGET
SEQUENCE GENERATOR
/PA1c

SEQUENCE
SELECTOR

/PA2c

SEQUENCE
LEARNER

/PA3c

VIRTUAL SEQUENCE
GENERATOR

/334c

LEARNER

/335c

CONTROLLER

/336c

OUTPUT PROCESSOR

Fig. 26

EP 3 982 369 A1

| Sequence ID | H1 | H2 | ⋯ | H35a | H35b | H36 | ⋯ | H113 | L1 | L2 | ⋯ | L107 | L107a |
|---|---|---|---|---|---|---|---|---|---|---|---|---|---|
| S000001 | M | E | ⋯ | P | S | Q | ⋯ | K | M | F | ⋯ | I | – |
| S000002 | M | E | ⋯ | D | S | Q | ⋯ | K | M | F | ⋯ | I | – |
| ⋯ | ⋯ | ⋯ | ⋯ | ⋯ | ⋯ | ⋯ | ⋯ | ⋯ | ⋯ | ⋯ | ⋯ | ⋯ | ⋯ |

Fig. 27

| Sequence ID | KD | Expression level | Self-polymerization | ... | Sensorgram | ... |
|---|---|---|---|---|---|---|
| S000001 | 1.00E-08 | 3.20E-01 | 9.92E-01 | ... | SG000001.jpg | ... |
| S000002 | 2.30E-08 | 3.50E-01 | 9.97E-01 | ... | (none) | ... |
| ... | ... | ... |  | ... | ... | ... |

EP 3 982 369 A1

93

## Fig. 28

Fig. 29

| Prediction target sequence ID | H1 | H2 | ... | H35a | H35b | H36 | ... | H113 | L1 | L2 | ... | L107 | L107a |
|---|---|---|---|---|---|---|---|---|---|---|---|---|---|
| V000001 | M | E | ... | D | S | R | ... | K | M | F | ... | I | − |
| V000002 | M | E | ... | D | S | K | ... | K | M | F | ... | I | − |
| ... | ... | ... | ... | ... | ... | ... | ... | ... | ... | ... | ... | ... | ... |

Fig. 30

| Prediction target sequence ID | KD | Expression level | Self-polymerization | ... | Sensorgram | ... |
|---|---|---|---|---|---|---|
| V000001 | 1.12E−08 | 8.70E−01 | 9.87E−01 | ... | | ... |
| V000002 | 7.75E−09 | 4.20E−01 | 6.47E−01 | ... | | ... |
| ... | ... | ... | ... | ... | ... | ... |

Fig. 31

Fig. 32

Tree1

Tree2

TreeK

AFFINITY ESTIMATION
INFORMATION T1

AFFINITY ESTIMATION
INFORMATION T2

AFFINITY ESTIMATION
INFORMATION TK

PREDICT AFFINITY ESTIMATION INFORMATION

EP 3 982 369 A1

## Fig. 33

```
           START

  STORE SEQUENCE INFORMATION/     S301
  CHARACTERISTIC INFORMATION

     FOR EACH SELECTION
        CONDITION

      SELECT SEQUENCE             S311
       INFORMATION

     LEARN FOR SEQUENCE           S312        S31
        GENERATION

  GENERATE MODEL TRAINED WITH     S313
     SEQUENCE LEARNING


        FOR EACH
      CHARACTERISTIC

  SELECT SEQUENCE INFORMATION/    S321
  CHARACTERISTIC INFORMATION

   LEARN FOR CHARACTERISTIC       S322        S32
         PREDICTION

  GENERATE MODEL TRAINED WITH     S323
  CHARACTERISTIC PREDICTION LEARNING


            END
```

Fig. 34

```
        ( START )
            |
            v
+---------------------------+
|   GENERATE PREDICTION     |___ S401
|    TARGET SEQUENCES       |
+---------------------------+
            |
            v
+---------------------------+
| PREDICT CHARACTERISTIC SCORES FOR |___ S402
|  PREDICTION TARGET SEQUENCES      |
+---------------------------+
            |
            v
+---------------------------+
|    OUTPUT CANDIDATE       |___ S403
|   ANTIBODY INFORMATION    |
+---------------------------+
            |
            v
        (  END  )
```

Fig. 35

/30d

SERVER

/31c

COMMUNICATOR

/33d

PROCESSOR

/331c

INFORMATION
ACQUIRER

/32d

STORAGE

/324d

LEARNING DATASET
STORAGE

/326c

LEARNING RESULT
STORAGE

/327c

MUTATION INFORMATION
STORAGE

/328c

SEQUENCE STORAGE

/329c

CHARACTERIZATION
INFORMATION STORAGE

/PAd

PREDICTION TARGET
SEQUENCE GENERATOR

/PA1d

SEQUENCE
SELECTOR

/PA2d

SEQUENCE
LEARNER

/PA3d

VIRTUAL SEQUENCE
GENERATOR

/334d

LEARNER

/335d

CONTROLLER

/336c

OUTPUT PROCESSOR

Fig. 36

```
                    ┌─────────┐
                    │  START  │
                    └────┬────┘
                         │
         ┌───────────────▼──────────────────┐
         │ STORE SEQUENCE INFORMATION/       │ ── S301
         │ CHARACTERISTIC INFORMATION        │
         └───────────────┬──────────────────┘
                         │
    ┌───────────────────▼──────────────────┐
    │ │ LEARNING PROCESS TO GENERATE        │ ── S31
    │ │ PREDICTION TARGET SEQUENCES         │
    │ └───────────────┬──────────────────┘
    │                 │
    │ ┌───────────────▼──────────────────┐
    │ │ LEARNING PROCESS TO GIVE          │ ── S32
    │ │ PREDICTION SCORES                 │
    │ └───────────────┬──────────────────┘
    │                 │
    │ ┌───────────────▼──────────────────┐
    │ │ GENERATE PREDICTION TARGET        │ ── S401
    │ │ SEQUENCES                         │
    │ └───────────────┬──────────────────┘
    │                 │
    │ ┌───────────────▼──────────────────┐
    │ │ PREDICT CHARACTERISTIC SCORES FOR │ ── S402
    │ │ PREDICTION TARGET SEQUENCES       │
    │ └───────────────┬──────────────────┘
    │                 │
    │ ┌───────────────▼──────────────────┐
    │ │ OUTPUT CANDIDATE ANTIBODY         │ ── S403
    │ │ INFORMATION                       │
    │ └───────────────┬──────────────────┘
```

S501

ADDITIONAL CHARACTERISTIC EVALUATION? — NO

YES

ADDITIONAL CHARACTERISTIC EVALUATION — S502

ADD SEQUENCE INFORMATION/CHARACTERISTIC INFORMATION AND STORE THEM — S503

S504

ADDITIONAL LEARNING PROCESS? — NO

YES

END

Fig. 37

Fig. 38

Fig. 39

Fig. 40

spearman:-0.576

Fig. 41

Fig. 42

Fig. 43A

CC= 0.89  SCC= 0.914

Fig. 43B

CC= 0.746  SCC= 0.781

Fig. 43C

CC= 0.768  SCC= 0.852

## Fig. 43D

CC= 0.787  SCC= 0.802

Fig. 43E

CC= 0.701  SCC= 0.817

Fig. 43F

CC= 0.935  SCC= 0.917

Fig. 43G

## Fig. 43H

CC= 0.82  SCC= 0.79

Fig. 43I

CC= 0.83   SCC= 0.889

Fig. 44A

CC= 0.864  SCC= 0.895

Fig. 44B

CC= 0.774  SCC= 0.797

Fig. 44C

CC= 0.739  SCC= 0.804

Fig. 44D

CC= 0.795  SCC= 0.802

Fig. 44E

CC= 0.626  SCC= 0.764

Fig. 44F

CC= 0.916 SCC= 0.892

Fig. 44G

CC= 0.59  SCC= 0.657

Fig. 44H

CC= 0.789  SCC= 0.753

Fig. 44I

CC= 0.811  SCC= 0.851

# Fig. 45

## INTERNATIONAL SEARCH REPORT

| International application No. |
|---|
| PCT/JP2020/022576 |

**A. CLASSIFICATION OF SUBJECT MATTER**
Int. Cl. G16B40/20(2019.01)i, G06N3/04(2006.01)i, G06N20/00(2019.01)i
FI: G16B40/20, G06N3/04 145, G06N20/00, G06N3/04 154

According to International Patent Classification (IPC) or to both national classification and IPC

**B. FIELDS SEARCHED**

Minimum documentation searched (classification system followed by classification symbols)
Int. Cl. G16B40/20, G06N3/04, G06N20/00

Documentation searched other than minimum documentation to the extent that such documents are included in the fields searched
Published examined utility model applications of Japan       1922-1996
Published unexamined utility model applications of Japan     1971-2020
Registered utility model specifications of Japan             1996-2020
Published registered utility model applications of Japan     1994-2020

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | US 2019/0065677 A1 (MASSACHUSETTS INSTITUTE OF TECHNOLOGY) 28 February 2019, paragraphs [0056]-[0108], fig. 1-4 | 1-15 |
| Y | JP 2005-526518 A (ABMAXIS INC.) 08 September 2005, claim 126 | 1-15 |

☐ Further documents are listed in the continuation of Box C.   ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 16.07.2020 | 28.07.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**
Information on patent family members

| International application No. |
| --- |
| PCT/JP2020/022576 |

| Patent Documents referred to in the Report | Publication Date | Patent Family | Publication Date |
| --- | --- | --- | --- |
| US 2019/0065677 A1 | 28.02.2019 | WO 2018/132752 A1 page 9, line 28 to page 31, line 18, fig. 1-4 EP 3568782 A1 | |
| JP 2005-526518 A | 08.09.2005 | WO 2003/099999 A2 claim 126 US 2003/0022240 A1 EP 1699484 A2 CA 2485732 A1 CN 1672160 A SG 146696 A1 AU 2003248548 A KR 10-2007-0036018 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2019106814 A
- WO 2018132752 A
- WO 2004044011 A
- WO 2005040229 A
- WO 2002032925 A
- WO 1995001937 A
- WO 2002020565 A
- WO 2003029462 A
- WO 2008016854 A
- EP 404097 A
- WO 1993011161 A
- US 6248516 B1
- WO 2015143414 A
- US 20110123527 A1

### Non-patent literature cited in the description

- **KUNKEL et al.** *Proc. Natl. Acad. Sci. USA,* 1985, vol. 82, 488-492
- *Annu. Rev. Biophys. Biomol. Struct.,* 2006, vol. 35, 225-249
- *Proc. Natl. Acad. Sci. U.S.A.,* 2003, vol. 100 (11), 6353-6357
- **KABAT.** Sequences of Proteins of Immunological Interest. National Institute of Health, 1987
- **KINDT et al.** Kuby Immunology. W.H. Freeman and Co, 2007, 91
- **PORTOLANO et al.** *J. Immunol.,* 1993, vol. 150, 880-887
- **CLARKSON et al.** *Nature,* 1991, vol. 352, 624-628
- **CHOTHIA ; LESK.** *J. Mol. Biol.,* 1987, vol. 196, 901-917
- **KABAT et al.** Sequences of Proteins of Immunological Interest. Public Health Service, National Institutes of Health, 1991
- **MACCALLUM et al.** *J. Mol. Biol.,* 1996, vol. 262, 732-745
- **HOLLIGER et al.** *Proc. Natl. Acad. Sci. U.S.A.,* 1993, vol. 90, 6444-6448
- **HUSTON et al.** *Proc. Natl. Acad. Sci. U.S.A.,* 1988, vol. 85, 5879-5883
- **HUDSON et al.** *J. Immunol. Methods,* 1999, vol. 231, 177-189
- **CO, M. S. et al.** *J. Immunol.,* 1994, vol. 152, 2968-2976
- **BETTER, M. ; HORWITZ, A. H.** *Methods Enzymol,* 1989, vol. 178, 476-496
- **PLUCKTHUN, A. ; SKERRA, A.** *Methods Enzymol,* 1989, vol. 178, 497-515
- **LAMOYI, E.** *Methods Enzymol,* 1986, vol. 121, 652-663
- **ROUSSEAUX, J. et al.** *Methods Enzymol,* 1986, vol. 121, 663-669
- **BIRD, R. E. ; WALKER, B. W.** *Trends Biotechnol,* 1991, vol. 9, 132-137
- **C VINCKE et al.** *The Journal of Biological Chemistry,* vol. 284, 3273-3284
- *Methods in Molecular Biology,* 2012, vol. 911, 65-78
- *Biochimica et Biophysica Acta - Proteins and Proteomics,* 2006, vol. 1764 (8), 1307-1319
- *Journal of Applied Microbiology,* 2014, vol. 117 (2), 528-536
- *Journal of Biomolecular Screening,* 2016, vol. 21 (1), 35-43
- *Journal of Biological Chemistry,* 2016, vol. 291 (24), 12641-12657
- *AIDS,* 2016, vol. 30, 11
- **MULLER AT et al.** *J Chem Inf Model,* 26 February 2018, vol. 58 (2), 472-479
- **SAITO et al.** *ACS Synth Biol,* 21 September 2018, vol. 7 (9), 2014-2022